# EUROPÄISCHE PATENTSCHRIFT

(11) **EP 1 861 493 B1**
(45) Veröffentlichungstag und Bekanntmachung des Hinweises auf die Patenterteilung: **05.09.2012**
(21) Anmeldenummer: 06725146.2
(22) Anmeldetag: 17.03.2006
(51) Int. Cl.: C12N 9/04, C07K 14/34, C12P 13/08

(54) **MUTIERTE ALLELE DES ZWG-GENS (G6PDH) AUS CORYNEFORMEN BAKTERIEN ZUR GESTEIGERTEN LYSIN PRODUKTION**
MUTANT ALLELES OF THE ZWF GENE (G6PDH) FROM CORYNEFORM BACTERIA FOR INCREASING LYSINE PRODUCTION
ALLELES MUTES DU GENE ZWF (G6PDH) TIRE DE CORYNEBACTERIES POUR LA PRODUCTION ACCRUE DE LYSINE

(30) Priorität: 24.03.2005 DE 102005013676; 07.03.2006 WO PCT/EP2006/060519
(43) Veröffentlichungstag der Anmeldung: 05.12.2007
(73) Patentinhaber: Evonik Degussa GmbH, 45128 Essen (DE)
(72) Erfinder: BATHE, Brigitte, 33154 Salzkotten (DE); SCHISCHKA, Natalie, 33649 Bielefeld (DE); THIERBACH, Georg, 33613 Bielefeld (DE)
(86) Internationale Anmeldenummer: PCT/EP2006/060851
(87) Internationale Veröffentlichungsnummer: WO 2006/100211

(56) Entgegenhaltungen:
- EP-A- 1 302 537
- WO-A-03/042389
- US-A1- 2003 175 911
- OHNISHI J ET AL: "A novel gnd mutation leading to increased l-lysine production in Corynebacterium glutamicum" FEMS MICROBIOLOGY LETTERS, AMSTERDAM, NL, Bd. 242, Nr. 2, 15. Januar 2005 (2005-01-15), Seiten 265-274, XP004696833 ISSN: 0378-1097
- MORITZ B ET AL: "KINETIC PROPERTIES OF THE GLUCOSE-6-PHOSPHATE AND 6-PHOSPHOGLUCONATE DEHYDROGENASES FROM CORYNEBACTERIUM GLUTAMICUM AND THEIR APPLICATION FOR PREDICTING PENTOSE PHOSPHATE PATHWAY FLUXIN VIVO" EUROPEAN JOURNAL OF BIOCHEMISTRY, BERLIN, DE, Bd. 267, Nr. 12, Juni 2000 (2000-06), Seiten 3442-3452, XP000957993 ISSN: 0014-2956 in der Anmeldung erwähnt

## Beschreibung

Gegenstand der Erfindung sind Mutanten und Allele des zwf-Gens coryneformer Bakterien kodierend für Varianten der Zwf-Untereinheit der Glucose-6-Phosphat-Dehydrogenase (EC: 1.1.1.49) und Verfahren zur Herstellung von Aminosäuren, insbesondere L-Lysin und L-Tryptophan unter Verwendung von Bakterien, die diese Allele enthalten.

### Stand der Technik

Aminosäuren finden in der Humanmedizin, in der pharmazeutischen Industrie, in der Lebensmittelindustrie und ganz besonders in der Tierernährung Anwendung.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien, wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die Aminosäuren produzieren. Ein bekannter Antimetabolit ist das Lysinanalogon S-(2-Aminoethyl)-L-Cystein (AEC).

Seit einigen Jahren werden ebenfalls Methoden der rekombinanten DNA-Technik zur Stammverbesserung von L-Aminosäure produzierenden Stämmen von Corynebacterium eingesetzt, indem man einzelne Aminosäure-Biosynthesegene amplifiziert und die Auswirkung auf die Aminosäure-Produktion untersucht. Eine zusammenfassende Darstellung über verschiedenste Aspekte der Genetik, des Stoffwechsels und der Biotechnologie von Corynebacterium glutamicum findet sich bei Pühler (chief ed.) im Journal of Biotechnology 104 (1-3), 1-338, 2003.

Die Nukleotidsequenz des für die Glucose-6-Phosphat-Dehydrogenase von Corynebacterium glutamicum kodierenden Gens ist unter anderem in der Datenbank des National Center for Biotechnology Information (NCBI) der National Library of Medicin (Bethesda, MD, USA) allgemein verfügbar. Sie kann weiterhin der Patentanmeldung WO 01/00844 als Sequenz Nr. 243 (= AX065117) entnommen werden.

In der WO 01/70995 ist eine Verbesserung der fermentativen Produktion von L-Aminosäuren durch coryneforme Bakterien durch die Verstärkung des Gens zwf beschrieben.

In der WO 01/98472, WO 03/042389 und US 2003/0175911 A1 wird von neuen Mutationen im zwf-Gen berichtet.

Moritz et al. (European Journal of Biochemistry 267, 3442-3452 (2000)) berichten über physiologische und biochemische Untersuchungen an der Glucose-6-Phosphat-Dehydrogenase von Corynebacterium glutamicum. Nach Untersuchungen von Moritz et al. besteht die Glucose-6-phosphat-Dehydrogenase aus einer Zwf-Untereinheit und einer OpcA-Untereinheit.

Die mikrobielle Biosynthese von L-Aminosäuren in coryneformen Bakterien ist ein komplexes System und vielschichtig mit diversen anderen Stoffwechselwegen in der Zelle vernetzt. Daher kann keine Vorhersage gemacht werden, welche Mutation die katalytischen Aktivität der Glucose-6-Phosphat-Dehydrogenase dergestalt verändert, dass die Produktion von L-Aminosäuren verbessert wird. Es ist daher wünschenswert weitere Varianten der Glucose-6-Phosphat-Dehydrogenase zur Verfügung zu haben.

Der besseren Übersichtlichkeit halber ist die Nukleotidsequenz des für die Glucose-6-Phosphat-Dehydrogenase beziehungsweise des für die Zwf-Untereinheit der Glucose-6-Phosphat-Dehydrogenase aus Corynebacterium glutamicum kodierenden zwf-Gens ("Wildtyp-Gen") gemäß den Angaben der NCBI-Datenbank in SEQ ID NO:1 und die sich daraus ergebende Aminosäuresequenz der kodierten Glucose-6-Phosphat-Dehydrogenase in SEQ ID NO:2 und 4 dargestellt. In SEQ ID NO:3 sind stromaufwärts (upstream) und stromabwärts (downstream) gelegene Nukleotidsequenzen zusätzlich angegeben.

### Aufgabe der Erfindung

Die Erfinder haben sich zur Aufgabe gestellt neue Maßnahmen zur verbesserten Herstellung von Aminosäuren, insbesondere L-Lysin und L-Tryptophan, bereitzustellen.

### Beschreibung der Erfindung

Gegenstand der Erfindung sind erzeugte beziehungsweise isolierte Mutanten coryneformer Bakterien, die bevorzugt Aminosäuren ausscheiden, und welche ein Gen bzw. Allel enthalten, das für ein Polypeptid mit Glucose-6-Phosphat-Dehydrogenase Aktivität kodiert, dadurch gekennzeichnet, dass das Polypeptid eine Aminosäuresequenz gemäβ SEQ ID NO:2 umfasst, bei der an der Position 321L - serin enthalten ist.

Das Polypeptid, das in den erfindungsgemäßen Mutanten enthalten ist, kann ebenfalls als Zwf-Polypeptid oder Zwf-Untereinheit der Glucose-6-phosphat-Dehydrogenase bezeichnet werden.

Bei den coryneformen Bakterien wird die Gattung Corynebacterium bevorzugt. Besonders bevorzugt sind Aminosäure-ausscheidende Stämme, die auf folgenden Arten beruhen:
Corynebacterium efficiens, wie zum Beispiel der Stamm DSM44549,
Corynebacterium glutamicum, wie zum Beispiel der Stamm ATCC13032,
Corynebacterium thermoaminogenes wie zum Beispiel der Stamm FERM BP-1539, und
Corynebacterium ammoniagenes, wie zum Beispiel der Stamm ATCC6871,
wobei die Art Corynbacterium glutamicum ganz besonders bevorzugt wird.

Einige Vertreter der Art Corynebacterium glutamicum sind im Stand der Technik auch unter anderen Artbezeicnungen bekannt. Hierzu gehören beispielsweise:
Corynebacterium acetoacidophilum ATCC13870,
Corynebacterium lilium DSM20137,
Corynebacterium melassecola ATCC17965,
Brevibacterium flavum ATCC14067,
Brevibacterium lactofermentum ATCC13869, und
Brevibacterium divaricatum ATCC14020.

Bekannte Vertreter Aminosäure ausscheidender Stämme coryneformer Bakterien sind beispielsweise
die L-Lysin produzierenden Stämme
Corynebacterium glutamicum DM58-1/pDM6 (= DSM4697) beschrieben in EP 0 358 940,
Corynebacterium glutamicum MH20-22B (= DSM16835)
beschrieben in Menkel et al. (Applied and Environmental Microbiology 55(3), 684-688 (1989)),
Corynebacterium glutamicum AHP-3 (= Ferm BP-7382)
beschrieben in EP 1 108 790,
Corynebacterium glutamicum NRRL B-11474
beschrieben in US 4,275,157
Corynebacterium thermoaminogenes AJ12521 (= FERM BP-3304) beschrieben in US 5,250,423,
oder die L-Tryptophan produzierenden Stämme
Corynebacterium glutamicum K76 (=FermBP-1847)
beschrieben in US 5,563,052,
Corynebacterium glutamicum BPS13 (=FermBP-1777) beschrieben in US 5,605,818, und
Corynebacterium glutamicum FermBP-3055
beschrieben in US 5,235,940.

Angaben zur taxonomischen Einordnung von Stämmen dieser Gruppe von Bakterien findet man unter anderem bei Seiler (Journal of General Microbiology 129, 1433-1477 (1983)), Kämpfer und Kroppenstedt (Canadian Journal of Microbiology 42, 989-1005 (1996)), Liebl et al (International Journal of Systematic Bacteriology 41, 255-260 (1991)) und in der USA-5,250,434.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit der Bezeichnung "NRRL" können von der Agricultural Research Service Patent Culture Collection (ARS, Peoria, Illinois, US) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden. Die genannten Stämme von Corynebacterium thermoaminogenes (FERM BP-1539, FERM BP-1540, FERM BP-1541 und FERM BP-1542) sind in der US-A 5,250,434 beschrieben.

Unter proteinogenen Aminosäuren versteht man die Aminosäuren, die in natürlichen Proteinen, das heißt in Proteinen von Mikroorganismen, Pflanzen, Tieren und Menschen vorkommen. Hierzu gehören insbesondere L-Aminosäuren, ausgewählt aus der Gruppe L-Asparaginsäure, L-Asparagin, L-Threonin, L-Serin, L-Glutaminsäure, L-Glutamin, Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Prolin und L-Arginin. Zu den L-Aminosäuren gehört ebenfalls das L-Homoserin.

Die erfindungsgemäßen Mutanten scheiden bevorzugt die genannten proteinogen Aminosäuren, insbesondere L-Lysin und L-Tryptophan aus. Der Begriff Aminosäuren umfasst auch deren Salze wie beispielsweise das Lysin-Monohydrochlorid oder Lysin-Sulfat im Falle der Aminosäure L-Lysin.

Gegenstand der Erfindung sind weiterhin Mutanten coryneformer Bakterien, die ein zwf-Allel enthalten, das für ein Polypeptid mit Glucose-6-Phosphat-Dehydrogenase Enzymaktivität kodiert, das die Aminsäuresequenz von SEQ ID NO: 2 umfasst, wobei an Position 321 L-serin enthalten ist. Gegebenenfalls enthält die Aminosäuresequenz des Polypeptids darüberhinaus an der Position 8 einen Aminosäureaustausch L-Serin gegen eine andere proteinogene Aminosäure, vorzugsweise L-Threonin. Die Beschreibung offenbart weiterhin Mutanten coryneformer Bakterien, die ein zwf-Allel enthalten, das für ein Polypeptid mit Glucose-6-Phosphat-Dehydrogenase Enzymaktivität kodiert, das an der Position 321 der Aminosäuresequenz von SEQ ID NO:2 entsprechenden Position jede proteinogene Aminosäure ausgenommen Glycin, bevorzugt L-Serin enthält, wobei das Gen eine Nukleotidsequenz umfasst, die mit der Nukleotidsequenz eines Polynukleotids identisch ist, das durch eine Polymerasekettenreaktion (PCR) unter Verwendung eines Primerpaars erhältlich ist, deren Nukleotidsequenzen jeweils mindestens 15 aufeinanderfolgende Nukleotide umfassen, die aus aus der Nukleotidsequenz zwischen Position 1 und 307 von SEQ ID NO:3 oder SEQ ID NO:11 und aus der komplementären Nukleotidsequenz zwischen Position 2100 und 1850 von SEQ ID NO:3 oder SEQ ID NO:11 ausgewählt werden. Beispiele für derartige geeignete Primerpaare sind in SEQ ID NO:17 und SEQ ID NO:18 und in SEQ ID NO:19 und SEQ ID NO:20 dargestellt. Als Ausgangsmaterial ("template"-DNA) wird chromosomale DNA coryneformer Bakterien bevorzugt, die insbesondere mit einem Mutagen behandelt worden sind. Besonders bevorzugt wird die chromosomale DNA der Gattung Corynebacterium und ganz besonders bevorzugt die der Art Corynebacterium glutamicum.

Die Beschreibung offenbart weiterhin Mutanten coryneformer Bakterien, die ein zwf-Allel enthalten, das für ein Polypeptid mit Glucose-6-Phosphat-Dehydrogenase Enzymaktivität kodiert, das eine Aminsäuresequenz mit einer Länge entsprechend 514 L-Aminosäuren umfasst, wobei an Position 321 jede proteinogene Aminosäure ausgenommen Glycin, bevorzugt L-Serin, enthalten ist. Gegebenenfalls enthält die Aminosäuresequenz des Polypeptids darüberhinaus einen Aminosäureaustausch L-Serin gegen eine andere proteinogene Aminosäure, vorzugsweise L-Threonin, an der Position 8.

Die Beschreibung offenbart weiterhin Mutanten coryneformer Bakterien, die ein zwf-Allel enthalten, das für ein Polypeptid mit Glucose-6-Phosphat-Dehydrogenase Enzymaktivität kodiert, das an Position 312 bis 330 der Aminosäuresequenz die Aminosäuresequenz entsprechend Position 312 bis 330 von SEQ ID NO:6 oder 8 enthält. Bevorzugt enthält die Aminosäuresequenz des kodierten Polypeptids eine Aminosäuresequenz entsprechend Position 307 bis 335 von SEQ ID NO:6 oder 8 oder Position 292 bis 350 von SEQ ID NO:6 oder 8 oder Position 277 bis 365 von SEQ ID NO:6 oder 8 oder Position 262 bis 380 von SEQ ID NO:6 oder 8 oder Position 247 bis 395 von SEQ ID NO:6 oder 8 oder Position 232 bis 410 von SEQ ID NO:6 oder 8 oder Position 202 bis 440 von SEQ ID NO:6 oder 8 oder Position 172 bis 470 von SEQ ID NO:6 oder 8 oder Position 82 bis 500 von SEQ ID NO:6 oder 8 oder Position 2 bis 512 von SEQ ID NO:6 oder 8 oder Position 2 bis 513 von SEQ ID NO:6 oder 8 oder Position 2 bis 514 von SEQ ID NO:6 oder 8. Ganz besonders bevorzugt umfasst die Länge des kodierten Polypeptids 514 Aminosäuren.

Die Beschreibung offenbart weiterhin Mutanten coryneformer Bakterien, die ein zwf-Allel enthalten, das für ein Polypeptid mit Glucose-6-Phosphat-Dehydrogenase Enzymaktivität kodiert, welches an Position 321 beziehungsweise an der entsprechenden Position der Aminosäuresequenz jede Aminosäure ausgenommen Glycin enthält, wobei dem Austausch gegen L-Serin der Vorzug gegeben wird und dessen Aminosäuresequenz außerdem zu mindestens 90%, bevorzugt mindestens 92% oder mindestens 94% oder mindestens 96%, und ganz besonders bevorzugt mindestens 97% oder mindestens 98% oder mindestens 99% identisch ist mit der Aminosäuresequenz von SEQ ID NO:6. Ein Beispiel für eine Aminosäuresequenz, die mindestens 99% Identität mit der Aminosäuresequenz von SEQ ID NO:6 besitzt, ist in SEQ ID NO:8 und 10 gezeigt. Das Polypeptid dieser Glucose-6-Phosphat-Dehydrogenase besitzt zusätzlich zu dem Aminosäureaustausch an Position 321 den Aminosäureaustausch L-Serin gegen L-Threonin an der Position 8.

Die Beschreibung offenbart weiterhin Mutanten coryneformer Bakterien, die ein zwf-Allel enthalten, das für ein Polypeptid mit Glucose-6-Phosphat-Dehydrogenase Enzymaktivität kodiert, welches an Position 321 beziehungsweise an der entsprechenden Position der Aminosäuresequenz jede Aminosäure ausgenommen Glycin enthält, wobei dem Austausch gegen L-Serin der Vorzug gegeben wird und dessen Nukleotidsequenz außerdem zu mindestens 90%, bevorzugt mindestens 92% oder mindestens 94% oder mindestens 96%, und ganz besonders bevorzugt mindestens 97% oder mindestens 98% oder mindestens 99% identisch ist mit der Nukleotidsequenz von SEQ ID NO:5. Ein Beispiel für eine Nukleotidsequenz eines zwf-Allels, die mindestens 99% Identität mit der Nukleotidsequenz von SEQ ID NO:5 besitzt, ist in SEQ ID NO:7 gezeigt. Die Nukleotidsequenz dieses zwf-Allels besitzt zusätzlich zu dem Nukleotidaustausch Guanin gegen Adenin an Position 961 (Siehe SEQ ID NO:5) den Nukleotidaustausch Thymin gegen Adenin an der Position 22 (Siehe SEQ ID NO:7). Ein weiteres Beispiel für eine Nukleotidsequenz eines zwf-Allels, die mindestens 99% Identität mit der Nukleotidsequenz von SEQ ID NO:5 besitzt, ist in SEQ ID NO:9 gezeigt. Die Nukleotidsequenz dieses zwf-Allels besitzt zusätzlich zu dem Nukleotidaustausch Guanin gegen Adenin an Position 961 (Siehe SEQ ID NO:5) und dem Nukleotidaustausch Thymin gegen Adenin an der Position 22 (Siehe SEQ ID NO:7) die Nukleotidaustausche Cytosin gegen Thymin an Position 138, Cytosin gegen Thymin an Position 279, Thymin gegen Cytosin an Position 738, Cytosin gegen Thymin an Position 777 und Guanin gegen Adenin an Position 906 (Siehe SEQ ID NO:9).

Es ist bekannt, dass konservative Aminosäureaustausche die Enzymaktivität nur unwesentlich verändern. Dementsprechend kann das in Mutanten enthaltene zwf-Allel, das für ein Polypeptid mit Glucose-6-Phosphat-Dehydrogenase Enzymaktivität kodiert, zusätzlich zu der in SEQ ID NO:6 und SEQ ID NO:8 beziehungsweise SEQ ID NO:10 gezeigten Aminosäuresequenz einen (1) oder mehrere konservative Aminosäureaustausch(e) enthalten. Bevorzugt enthält das Polypeptid höchstens zwei (2), höchstens drei (3), höchstens vier (4) oder höchstens fünf (5) konservative Aminosäureaustausche.

Bei den aromatischen Aminosäuren spricht man von konservativen Austauschen, wenn Phenylalanin, Tryptophan und Tyrosin gegeneinander ausgetauscht werden. Bei den hydrophoben Aminosäuren spricht man von konservativen Austauschen, wenn Leucin, Isoleucin und Valin gegeneinander ausgetauscht werden. Bei den polaren Aminosäuren spricht man von konservativen Austauschen, wenn Glutamin und Asparagin gegeneinander ausgetauscht werden. Bei den basischen Aminosäuren spricht man von konservativen Austauschen, wenn Arginin, Lysin und Histidin gegeneinander ausgetauscht werden. Bei den sauren Aminosäuren spricht man von konservativen Austauschen, wenn Asparaginsäure und Glutaminsäure gegeneinander ausgetauscht werden. Bei den Hydroxyl-Gruppen enthaltenden Aminosäuren spricht man von konservativen Austauschen, wenn Serin und Threonin gegeneinander ausgetauscht werden.

Ein Beispiel für einen konservativen Aminosäureaustausch ist der Austausch Serin gegen Threonin an Position 8 von SEQ ID NO:6, der zu der Aminosäuresequenz gemäß SEQ ID NO:8 beziehungsweise SEQ ID NO:10 führt.

Bei der Arbeit an der vorliegenden Erfindung wurde durch Vergleich der Aminosäuresequenz mit dem Clustal-Programm (Thompson et al., Nucleic Acids Research 22, 4637-4680 (1994)) festgestellt, dass die Aminosäuresequenzen der Glucose-6-Phosphat-Dehydrogenase verschiedener Bakterien wie bespielsweise Escherichia coli, Bacillus subtilis, Mycobacterium tuberculosis, Mycobacterium bovis, Streptomyces coeliclor, Streptomyces avermitilis, Corynebacterium efficiens und Corynebacterium glutamicum ein Sequenzmotiv bestehend aus der Abfolge Val-Ile-Phe-Gly-Ali-Afa-Gly-Asp-Leu, ein Sequenzmotiv bestehend aus der Abfolge Arg-Ile-Asp-His-Tyr-Leu-Gly-Lys und auch ein Sequenzmotiv bestehend aus der Abfolge Arg-Trp-Ala-Gly-Val-Pro-Phe-Tyr-Bra-Arg-Thr-Gly-Lys-Arg enthalten. Die Bezeichnung "Ali" steht für die Aminosäuren Ala oder Val, die Bezeichnung "Afa" für die Aminosäuren Lys oder Thr und die Bezeichnung "Bra" für die Aminosäuren Ile oder Leu.

Dementsprechend sind solche Mutanten coryneformer Bakterien bevorzugt, die ein zwf-Allel enthalten, das für ein Polypeptid mit Glucose-6-Phosphat-Dehydrogenase Enzymaktivität kodiert, welches mindestens eine Aminosäuresequenz ausgewählt aus der Gruppe Val-Ile-Phe-Gly-Ali-Afa-Gly-Asp-Leu, Arg-Ile-Asp-His-Tyr-Leu-Gly-Lys und Arg-Trp-Ala-Gly-Val-Pro-Phe-Tyr-Bra-Arg-Thr-Gly-Lys-Arg umfasst und an Position 321 beziehungsweise der entsprechenden oder vergleichbaren Position der Aminosäuresequenz jede Aminosäure ausgenommen Glycin, bevorzugt L-Serin, enthält. Gegebenenfalls enthält die Aminosäuresequenz darüberhinaus einen Aminosäureaustausch L-Serin gegen eine andere proteinogene Aminosäure, vorzugsweise L-Threonin, an der Position 8 gemäß SEQ ID NO:2.

Das Aminosäuresequenzmotiv Val-Ile-Phe-Gly-Val-Thr-Gly-Asp-Leu ist beispielsweise in der SEQ ID NO:6, 8 oder 10 von Position 32 bis 40 enthalten. Das Aminosäuresequenzmotiv Arg-Ile-Asp-His-Tyr-Leu-Gly-Lys ist beispielsweise in der SEQ ID NO:6, 8 beziehungsweise 10 von Position 203 bis 210 enthalten. Das Aminosäuresequenzmotiv Arg-Trp-Ala-Gly-Val-Pro-Phe-Tyr-Leu-Arg-Thr-Gly-Lys-Arg ist beispielsweise in der SEQ ID NO:6, 8 oder 10 von Position 354 bis 367 enthalten.

Gegenstand der Erfindung sind schließlich Mutanten coryneformer Bakterien, die ein zwf-Allel enthalten, das für ein Polypeptid mit Glucose-6-Phosphat-Dehydrogenase Enzymaktivität kodiert, welches die Aminosäuresequenz von SEQ ID NO:6 oder SEQ ID NO:8 beziehungsweise SEQ ID NO:10 umfasst.

Es ist bekannt, dass durch wirtseigene Enzyme, sogenannte Aminopeptidasen, das endständige Methionin bei der Proteinsynthese entfernt wird.

Unter dem Begriff "einer zu Position 321 der Aminosäuresequenz entsprechenden Position" oder "einer zu Position 321 der Aminosäuresequenz vergleichbaren Position" versteht man die Tatsache, dass durch Insertion oder Deletion eines für eine Aminosäure kodierenden Kodons im N-terminalen Bereich (bezogen auf die Position 321 von SEQ ID NO:6, 8 oder 10) des kodierten Polypeptids die Positionsangabe und Längenangabe im Falle einer Insertion formal um eine Einheit erhöht oder im Falle einer Deletion um eine Einheit verringert wird. Beispielsweise rückt durch Deletion des für die Aminosäure L-Asparagin kodierenden AAC-Kodons an Position 4 von SEQ ID NO:6, 8 oder 10 das L-Serin von Position 321 an Position 320. Die Längenangabe wäre dann: 513 Aminosäuren. In gleicher Weise wird durch Insertion oder Deletion eines für eine Aminosäure kodierenden Kodons im C-terminalen Bereich (bezogen auf die Position 321) des kodierten Polypeptids die Längenangabe im Falle einer Insertion formal um eine Einheit erhöht oder im Falle einer Deletion um eine Einheit verringert. Derartige vergleichbare Positionen lassen sich durch Vergleich der Aminosäuresequenzen in Form eines "alignments" beispielsweise mit Hilfe des Clustal-Programmes leicht identifizieren.

Durch derartige Insertionen und Deletionen wird die enzymatische Aktivität im wesentlichen nicht berührt. "Im wesentlichen nicht berührt" bedeutet, dass sich die enzymatische Aktivität der genannten Varianten um maximal 5%, maximal 2,5% oder maximal 1% von der Aktivität des Polypeptids mit der Aminosäuresequenz von SEQ ID NO:6 oder 8 beziehungsweise 10 unterscheidet.

Gegenstand der Erfindung sind dementsprechend auch zwf-Allele, die für Polypeptid Varianten von SEQ ID NO:6 oder 8 beziehungsweise 10 kodieren, die eine oder maximal 5, maximal 4, maximal 3 oder maximal 2 Insertionen oder Deletionen von Aminosäuren enthalten.

Die Sequenzmotive Val-Ile-Phe-Gly-Ali-Afa-Gly-Asp-Leu, und Arg-Ile-Asp-His-Tyr-Leu-Gly-Lys und Arg-Trp-Ala-Gly-Val-Pro-Phe-Tyr-Bra-Arg-Thr-Gly-Lys-Arg werden durch derartige Insertionen/Deletionen vorzugsweise nicht zerrissen.

Zur Herstellung der erfindungsgemäßen Mutanten können klassische in-vivo Mutageneseverfahren mit Zellpopulationen coryneformer Bakterien unter Verwendung mutagener Stoffe wie beispielsweise N-Methyl-N'-Nitro-N-Nitrosoguanidin (MNNG), Ethylmethansulfonat (EMS), 5-Bromuracil, oder ultraviolettes Licht verwendet werden. Mutagenesemethoden sind beispielsweise im Manual of Methods for General Bacteriology (Gerhard et al. (Eds.), American Society for Microbiology, Washington, DC, USA, 1981) oder bei Tosaka et al. (Agricultural and Biological Chemistry 42(4), 745-752 (1978)) oder bei Konicek et al (Folia Microbiologica 33, 337-343 (1988)) beschrieben. Typische Mutagenesen unter Verwendung von MNNG umfassen Konzentrationen von 50 bis 500 mg/l oder auch höhere Konzentrationen bis zu maximal 1 g/l, eine Inkubationszeit von 1 bis 30 Minuten bei einem pH von 5,5 bis 7,5. Unter diesen Bedingungen wird die Zahl der lebensfähigen Zellen um einen Anteil von ca. 50% bis 90%
oder ca. 50% bis 99% oder ca. 50% bis 99,9% oder mehr reduziert.

Aus der mutagenisierten Zellpopulation werden Mutanten beziehungsweise Zellen entnommen und vermehrt. Vorzugsweise wird in einem weiteren Schritt deren Fähigkeit untersucht, in einer Satzkultur (batch) unter Verwendung eines geeigneten Nährmediums Aminosäuren, bevorzugt L-Lysin oder L-Tryptophan, auszuscheiden. Geeignete Nährmedien und Testbedingungen sind unter anderem in der US 6,221,636, in der US 5,840,551, in der US 5,770,409, in der US 5,605,818, in der US 5,275,940 und in der US 4,224,409 beschrieben. Bei Verwendungen von geeigneten Roboteranlagen, wie beispielsweise bei Zimmermann et al. (VDI Berichte Nr. 1841, VDI-Verlag, Düsseldorf, Deutschland 2004, 439-443) oder Zimmermann (Chemie Ingenenieur Technik 77 (4), 426-428 (2005))beschrieben, können zahlreiche Mutanten in kurzer Zeit untersucht werden. Im Allgemeinen werden maximal 3.000, maximal 10.000, maximal 30.000 oder auch maximal 60.000 Mutanten gegebenenfalls auch mehr untersucht. Auf diese Weise werden Mutanten identifiziert, die im Vergleich zum Elternstamm beziehungsweise nicht mutagenisierten Ausgangsstamm vermehrt Aminosäuren in das Nährmedium oder in das Zellinnere ausscheiden. Dazu gehören beispielsweise solche Mutanten, deren Aminosäuresekretion um mindestens 0,5% erhöht ist.

Anschließend wird aus den Mutanten DNA bereitgestellt, beziehungsweise isoliert und mit Hilfe der Polymerase-Kettenreaktion unter Verwendung von Primerpaaren, die die Amplifizierung des zwf-Gens beziehungsweise des erfindungsgemäßen zwf-Allels oder der erfindungsgemäßen Mutation an Position 321 erlauben, das entsprechende Polynukleotid synthetisiert. Vorzugsweise wird die DNA aus solchen Mutanten isoliert, die in erhöhter Weise Aminosäuren ausscheiden.

Hierzu können beliebige Primerpaare aus der stromaufwärts und stromabwärts der erfindungsgemäßen Mutation gelegenen Nukleotidsequenz und der dazu komplementären Nukleotidsequenz ausgewählt werden. Ein Primer eines Primerpaares umfasst hierbei bevorzugt mindestens 15, mindestens 18, mindestens 20, mindestens 21 oder mindestens 24 aufeinanderfolgende Nukleotide ausgewählt aus der Nukleotidsequenz zwischen Position 1 und 1267 von SEQ ID NO:3 oder SEQ ID NO:11. Der dazugehörige zweite Primer eines Primerpaares umfasst mindestens 15, mindestens 18, mindestens 20, mindestens 21 oder mindestens 24 aufeinanderfolgende Nukleotide ausgewählt aus der komplementären Nukleotidsequenz von Position 2100 und 1271 von SEQ ID NO:3 oder SEQ ID NO:11. Ist die Amplifikation der Kodierregion gewünscht, so wird das Primerpaar vorzugsweise aus der Nukleotidsequenz zwischen Position 1 und 307 von SEQ ID NO:3 oder SEQ ID NO:11 und aus der komplementären Nukleotidsequenz zwischen Position 2100 und 1850 von SEQ ID NO:3 oder SEQ ID NO:11 ausgewählt. Ist die Amplifikation eines Teils der Kodierregion, wie beispielsweise in SEQ ID NO:14 und 15 dargestellt, erwünscht, so wird das Primerpaar vorzugsweise aus der Nukleotidsequenz zwischen Position 309 und 1267 von SEQ ID NO:3 oder SEQ ID NO:11 und aus der komplementären Nukleotidsequenz zwischen Position 1848 und 1271 von SEQ ID NO:3 oder SEQ ID NO:11 ausgewählt. Geeignete Primerpaare sind beispielsweise das unter SEQ ID NO:17 und SEQ ID NO:18 wiedergegebene Primerpaar zwf-K1 und zwf-K2 oder das unter SEQ ID NO:19 und SEQ ID NO:20 wiedergegebene Primerpaar zwf-L1 und zwf-L2. Der Primer kann überdies mit Erkennungsstellen für Restriktionsenzyme, mit einer Biotin-Gruppe oder weiteren Accessoirs, wie sie im Stand der Technik beschrieben sind, ausgerüstet sein. Die Gesamtlänge des Primers beträgt im Allgemeinen maximal 30, 40, 50 oder 60 Nukleotide.

Für die Herstellung von Polynukleotiden durch Amplifikation ausgewählter Sequenzen, wie dem erfindungsgemäßen zwf-Allel, aus vorgelegter, beispielsweise chromosomaler DNA ("template DNA")durch Amplifikation mittels PCR, werden im Allgemeinen thermostabile DNA-Polymerasen eingesetzt. Beispiele derartiger DNA-Polymerasen sind die Taq-Polymerase aus Thermus aquaticus, die unter anderem von der Firma Qiagen (Hilden, Deutschland) vertrieben wird, die Vent-Polymerase aus Thermococcus litoralis, die unter anderem von der Firma New England Biolabs (Frankfurt, Deutschland) vertrieben wird oder die Pfu-Polymerase aus Pyrococcus furiosus, die unter anderem von der Firma Stratagene (La Jolla, USA) vertrieben wird. Bevorzugt werden Polymerasen mit "proof-reading"-Aktivität. "proof-reading"-Aktivität bedeutet, dass diese Polymerasen in der Lage sind, fehlerhaft eingebaute Nukleotide zu erkennen und den Fehler durch erneute Polymerisation zu beheben (Lottspeich und Zorbas, Bioanalytik, Spektrum Akademischer Verlag, Heidelberg, Deutschland (1998)). Beispiele für Polymerasen mit "proof-reading"-Aktivität sind die Vent-Polymerase und die Pfu-Polymerase.

Die Bedingungen im Reaktionsansatz werden nach Angaben des Herstellers eingestellt. Die Polymerasen werden vom Hersteller im Allgemeinen zusammen mit dem gebräuchlichen Puffer geliefert, welcher üblicherweise Konzentrationen von 10 - 100 mM Tris/HCl und 6 - 55 mM KCl bei pH 7,5 - 9,3 aufweist. Magnesiumchlorid wird in einer Konzentration von 0,5 - 10 mM zugesetzt, falls es nicht in dem vom Hersteller gelieferten Puffer enthalten ist. Dem Reaktionsansatz werden weiterhin Desoxynucleosidtriphosphate in einer Konzentration von 0,1 - 16,6 mM zugesetzt. Die Primer werden im Reaktionsansatz mit einer Endkonzentration von 0,1 - 3 µM vorgelegt und die Template-DNA im optimalen Fall mit 10² bis 10⁵ Kopien. Es können auch 10⁶ bis 10⁷ Kopien eingesetzt werden. Die entsprechende Polymerase wird dem Reaktionsansatz in einer Menge von 2-5 Units zugegeben. Ein typischer Reaktionsansatz hat ein Volumen von 20 - 100 µl.

Als weitere Zusätze können der Reaktion Rinderserumalbumin, Tween-20, Gelatin, Glycerin, Formamid oder DMSO beigesetzt werden (Dieffenbach und Dveksler, PCR Primer - A Laboratory Manual, Cold Spring Harbor Laboratory Press, USA 1995).

Ein typischer PCR-Verlauf besteht aus drei unterschiedlichen, sich aufeinanderfolgend wiederholenden Temperaturstufen. Vorab wird die Reaktion mit einer Temperaturerhöhung auf 92°C - 98°C für 4 bis 10 Minuten gestartet, um die vorgelegte DNA zu denaturieren. Dann folgen sich wiederholend zuerst ein Schritt zur Denaturierung der vorgelegten DNA von 10 - 60 Sekunden bei ungefähr 92-98°C, dann ein Schritt zum Binden der Primer an die vorgelegte DNA von 10 - 60 Sekunden bei einer bestimmten, von den Primern abhängigen Temperatur ("Annealing-Temperatur"), die erfahrungsgemäß bei 50°C bis 60°C liegt und sich für jedes Primerpaar einzeln berechnen läßt. Genaue Informationen dazu findet der Fachmann bei Rychlik et al. (Nucleic Acids Research 18 (21): 6409-6412). Anschließend folgt ein Synthese-Schritt zur Verlängerung der vorgelegten Primer ("Extension") bei dem jeweils für die Polymerase angegebenen Aktivitätsoptimum, üblicherweise je nach Polymerase im Bereich von 73°C bis 67°C bevorzugt 72°C bis 68°C. Die Dauer dieses Extensionschrittes hängt von der Leistungsfähigkeit der Polymerase und Länge des zu amplifizierenden PCR-Produktes ab. In einer typischen PCR dauert dieser Schritt 0,5 - 8 Minuten, bevorzugt 2 - 4 Minuten. Diese drei Schritte werden 30 bis 35 mal, gegebenenfalls bis zu 50 mal wiederholt. Ein abschließender "Extension"-Schritt von 4 - 10 Minuten beendet die Reaktion. Die auf diese Weise hergestellten Polynukleotide werden auch als Amplifikate bezeichnet; der Begriff Nukleinsäurefragment ist ebenfalls gebräuchlich.

Weitere Anleitungen und Informationen zur PCR findet der Fachmann beispielsweise im Handbuch "PCR-Strategies" (Innis, Felfand und Sninsky, Academic Press , Inc., 1995), im Handbuch von Diefenbach und Dveksler "PCR Primer - a laboratory manual" (Cold Spring Harbor Laboratory Press,1995), im Handbuch von Gait "Oligonukleotide synthesis: A Practical Approach" (IRL Press, Oxford, UK, 1984) und bei Newton und Graham "PCR" (Spektrum Akademischer Verlag, Heidelberg, Deutschland, 1994).

Die Nukleotidsequenz wird anschließend beispielsweise nach dem Kettenabbruchverfahren von Sanger et al. (Proceedings of the National Academies of Sciences, U.S.A., 74, 5463-5467 (1977)) mit den von Zimmermann et al. (Nucleic Acids Research 18, 1067pp (1990)) angegebenen Modifikationen bestimmt und das von dieser Nukleotidsequenz kodierte Polypeptid insbesondere bezüglich der Aminosäuresequenz analysiert. Dazu wird die Nukleotidsequenz in ein Programm zur Übersetzung von DNA-Sequenz in eine Aminosäuresequenz eingegeben. Geeignete Programme sind beispielsweise das Programm "Patentin", das bei Patentämtern, beispielsweise dem US-Patentamt (USPTO) erhältlich ist, oder das "Translate Tool", das auf dem ExPASy Proteomics Server im World Wide Web (Gasteiger et al., Nucleic Acids Research 31, 3784-3788 (2003)) verfügbar ist.

Auf diese Weise werden Mutanten identifiziert, deren zwf-Allele für Polypeptide mit Glucose-6-Phosphat-Dehydrogenase Enzymaktivität kodieren, welche an Position 321 der Aminosäuresequenz, beziehungsweise der entsprechenden oder vergleichbaren Position, jede proteinogene Aminosäure ausgenommen Glycin enthalten. Bevorzugt wird der Austausch gegen L-Serin. Gegebenenfalls enthält die Aminosäuresequenz darüberhinaus einen Aminosäureaustausch L-Serin gegen eine andere proteinogene Aminosäure, vorzugsweise L-Threonin, an der Position 8 beziehungsweise an der entsprechenden oder vergleichbaren Position.

Gegenstand der Erfindung ist dementsprechend eine Mutante eines coryneformen Bakteriums, dadurch gekennzeichnet, dass diese durch folgende Schritte erhältlich ist:
a) Behandlung eines coryneformen Bakteriums, das die Fähigkeit besitzt Aminosäuren auszuscheiden, mit einem mutagenen Agenz,
b) Isolierung und Vermehrung der in a) erzeugten Mutante,
c) Bereitstellung von Nukleinsäure aus der in b) erhaltenen Mutante,
d) Herstellung eines Nukleinsäuremoleküls/ Amplifikates/Nukleinsäurefragments unter Verwendung der Polymerasekettenreaktion, der Nukleinsäure aus c), und eines Primerpaares bestehend aus einem ersten Primer umfassend mindestens 15 aufeinanderfolgenden Nukleotide ausgewählt aus der Nukleotidsequenz zwischen Position 1 und 1267 von SEQ ID NO:3 oder SEQ ID NO:11 und einem zweitem Primer umfassend mindestens 15 aufeinanderfolgende Nukleotide ausgewählt aus der komplementären Nukleotidsequenz zwischen Position 2100 und 1271 von SEQ ID NO:3 oder 11.
e) Bestimmung der Nukleotidsequenz des in e) erhaltenen Nukleinsäuremoleküls, und Bestimmung der kodierten Aminosäuresequenz,
f) gegebenfalls Vergleich der in f) bestimmten Aminosäuresesequenz mit SEQ ID NO:6, 8 oder 10, und
g) Identifizierung einer Mutante, die ein Polynukleotid enthält, das für ein Polypeptid mit glucose-6-Phosphat Dehydrogenase Aktivität kodiert, welches an der Position entsprechend Position 321 von SEQ NO:2 L-Serin enthält.

Die auf diese Weise erzeugten Mutanten enthalten typischerweise eine (1) Kopie des beschriebenen zwf Allels. In der SEQ ID NO:5, 7 und 9 sind beispielhaft die Kodierregionen von zwf-Allelen erfindungsgemäßer Mutanten wiedergegeben. Die Kodierregion des Wildtypgens ist als SEQ ID NO:1 wiedergegeben. SEQ ID NO:1 enthält an Position 961 die Nukleobase Guanin, an Position 962 die Nukleobase Guanin und an Position 963 die Nukleobase Cytosin. SEQ ID NO:1 enthält an Position 961 bis 963 das für die Aminosäure Glycin kodierende GGC Kodon. SEQ ID NO:5 enthält an Position 961 die Nukleobase Adenin. Durch diese Guanin Adenin Transition entsteht an Position 961 bis 963 das für die Aminosäure L-Serin kodierende AGC Kodon.

SEQ ID NO:1 enthält an Position 22 die Nukleobase Thymin, an Position 23 die Nukleobase Cytosin und an Position 24 die Nukleobase Cytosin. SEQ ID NO:1 enthält dementsprechend an Position 22 bis 24 das für die Aminosäure Serin kodierende TCC Kodon. SEQ ID NO:7 enthält an Position 22 die Nukleobase Adenin. Durch diese Thymin-Adenin Transversion entsteht an Position 22 bis 24 das für die Aminosäure L-Serin kodierende AGC Kodon.

Darüber hinaus können die in SEQ ID NO: 5 und 7 dargestellten Nukleotidsequenzen weitere Basenaustausche enthalten, die sich durch die Mutagenesebehandlung ergeben haben, sich jedoch nicht in einer veränderten Aminosäuresequenz äußern. Derartige Mutationen werden in der Fachwelt auch als stille oder neutrale Mutationen bezeichnet. Diese stillen Mutationen können ebenfalls in dem für Mutagenesebehandlung eingesetzten coryneformen Bakterium bereits enthalten sein. Beispiele für derartige stille Mutationen sind die Cytosin-Thymin Transition an Position 138, die Cytosin-Thymin Transition an Position 279, die Thymin-Cytosin Transition an Position 738, die Cytosin-Thymin Transition an Position 777 und die Guanin-Adenin Transition an Position 906 wie in SEQ ID NO:9 dargestellt.

Die für die Mutagenese verwendeten coryneformen Bakterien besitzen bevorzugt bereits die Fähigkeit, die gewünschte Aminosäure in das sie umgebende Nährmedium beziehungsweise Fermentationsbrühe auszuscheiden oder im Zellinneren anzureichern.

L-Lysin produzierende coryneforme Bakterien besitzen typischerweise eine "feed back" resistente beziehungsweise desensibilisierte Aspartatkinase. Unter "feed back" resistenten Aspartatkinasen versteht man Aspartatkinasen, die im Vergleich zur Wildform eine geringere Empfindlichkeit gegenüber der Hemmung durch Mischungen von Lysin und Threonin oder Mischungen von AEC (Aminoethylcystein) und Threonin oder Lysin allein oder AEC allein aufweisen. Die für diese desensibilisierten Aspartatkinasen kodierenden Gene beziehungsweise Allele werden auch als lysC^{FBR}-Allele bezeichnet. Im Stand der Technik (Tabelle 1) sind zahlreiche lysC^{FBR}-Allele beschrieben, die für Aspartatkinase-Varianten kodieren, welche Aminosäureaustausche im Vergleich zum Wildtypprotein besitzen. Die Kodierregion des Wildtyp lysC-Gens von Corynebacterium glutamicum entsprechend der Zugangsnummer AX756575 der NCBI Datenbank ist in SEQ ID NO:21 und das von diesem Gen kodierte Protein in SEQ ID NO:22 dargestellt.

**Tabelle 1**

| lysC^{FBR}-Allele kodierend für feed back resistente Aspartatkinasen | | | |
|---|---|---|---|
| Bezeichnung des Allels | Weitere Angaben | Referenz | Zugangsnummer |
| lysC^{FBR}-E05108 | | JP 1993184366-A (Sequenz 1) | E05108 |
| lysC^{FBR}-E06825 | lysC A279T | JP 1994062866-A (Sequenz 1) | E06825 |
| lysC^{FBR}-E06826 | lysC A279T | JP 1994062866-A (Sequenz 2) | E06826 |
| lysC^{FBR}-E06827 | | JP 1994062866-A (Sequenz 3) | E06827 |
| lysC^{FBR}-E08177 | | JP 1994261766-A (Sequenz 1) | E08177 |
| lySC^{FBR}-E08178 | lysC A279T | JP 1994261766-A (Sequenz 2) | E08178 |
| lysC^{FBR}-E08179 | lysC A279V | JP 1994261766-A (Sequenz 3) | E08179 |
| lysC^{FBR}-E08180 | lysC S301F | JP 1994261766-A (Sequenz 4) | E08180 |
| lysC^{FBR}-E08181 | lysC T308I | JP 1994261766-A (Sequenz 5) | E08181 |
| lysC^{FBR}-E08182 | | JP 1994261766-A (Sequenz 6) | E08182 |
| lysC^{FBR}-E12770 | | JP 1997070291-A (Sequenz 13) | E12770 |
| lysC^{FBR}-E14514 | | JP 1997322774-A (Sequenz 9) | E14514 |
| lysC^{FBR}-E16352 | | JP 1998165180-A (Sequenz 3) | E16352 |
| lySC^{FBR}-E16745 | | JP 1998215883-A (Sequenz 3) | E16745 |
| lySC^{FBR}-E16746 | | JP 1998215883-A (Sequenz 4) | E16746 |
| lysC^{FBR}-174588 | | US 5688671-A (Sequenz 1) | I74588 |
| lysC^{FBR}-I74589 | lysC A279T | US 5688671-A (Sequenz 2) | I74589 |
| lysC^{FBR}-I74S90 | | US 5688671-A (Sequenz 7) | I74590 |
| lysC^{FBR}-I74591 | lysC A279T | US 5688671-A (Sequenz 8) | I74591 |
| lysC^{FBR}-I74592 | | US 5688671-A (Sequenz 9) | I74592 |
| lysC^{FBR}-I74593 | lysC A279T | US 5688671-A (Sequenz 10) | 174593 |
| lysC^{FBR}-I74594 | | US 5688671-A (Sequenz 11) | I74594 |
| lysC^{FBR}-I74595 | lysC A279T | US 5688671-A (Sequenz 12) | I74595 |
| lysC^{FBR}-I74596 | | US 5688671-A (Sequenz 13) | I74596 |
| lysC^{FBR}-I74597 | lysC A279T | US 5688671-A (Sequenz 14) | I74597 |
| lysC^{FBR}-X57226 | lysC S301Y | EP0387527 Kalinowski et al., Molecular and General Genetics 224:317-324 (1990) | X57226 |
| lysC^{FBR}-L16848 | lysC G345D | Follettie and Sinskey NCBI Nucleotide Database (1990) | L16848 |
| lysC^{FBR}-L27125 | lysC R320G lysC G345D | Jetten et al., Applied Microbiology Biotechnology 43:76-82 (1995) | L27125 |
| lysC^{FBR} | lysC T311I | WO0063388 (Sequenz 17) | |
| lysC^{FBR} | lysC S301F | US3732144 | |
| lysC^{FBR} | lysC S381F | EP0435132 | |
| lysC^{FBR} | lysC S317A | US5688671 (Sequenz 1) | |
| lysC^{FBR} | lysC T380I | WO 01/49854 | |

L-Lysin ausscheidende coryneforme Bakterien besitzen typischerweise einen oder mehrere der in Tabelle 1 aufgelisteten Aminosäureaustausche.

Bevorzugt werden folgende lysC^{FBR}-Allele: lysC A279T (Austausch von Alanin an Position 279 des kodierten Aspartatkinaseproteins gemäß SEQ ID NO: 22 gegen Threonin), lysC A279V (Austausch von Alanin an Position 279 des kodierten Aspartatkinaseproteins gemäß SEQ ID NO: 22 gegen Valin), lysC S301F (Austausch von Serin an Position 301 des kodierten Aspartatkinaseproteins gemäß SEQ ID NO: 22 gegen Phenylalanin), lysC T308I (Austausch von Threonin an Position 308 des kodierten Aspartatkinaseproteins gemäß SEQ ID NO: 22 gegen Isoleucin), lysC S301Y (Austausch von Serin an Position 308 des kodierten Aspartatkinaseproteins gemäß SEQ ID NO: 22 gegen Tyrosin), lysC G345D (Austausch von Glycin an Position 345 des kodierten Aspartatkinaseproteins gemäß SEQ ID NO: 22 gegen Asparaginsäure), lysC R320G (Austausch von Arginin an Position 320 des kodierten Aspartatkinaseproteins gemäß SEQ ID NO: 22 gegen Glycin), lysC T311I (Austausch von Threonin an Position 311 des kodierten Aspartatkinaseproteins gemäß SEQ ID NO: 22 gegen Isoleucin), lysC S381F (Austausch von Serin an Position 381 des kodierten Aspartatkinaseproteins gemäß SEQ ID NO: 22 gegen Phenylalanin) und lysC S317A (Austausch von Serin an Position 317 des kodierten Aspartatkinaseproteins gemäß SEQ ID NO: 22 gegen Alanin).

Besonders bevorzugt werden das lysC^{FBR}-Allel lysC T311I (Austausch von Threonin an Position 311 des kodierten Aspartatkinaseproteins gemäß SEQ ID NO: 22 gegen Isoleucin) und ein lysC^{FBR}-Allel enthaltend mindestens einen Austausch ausgewählt aus der Gruppe A279T (Austausch von Alanin an Position 279 des kodierten Aspartatkinaseproteins gemäß SEQ ID NO: 22 gegen Threonin), S381F (Austausch von Serin an Position 381 des kodierten Aspartatkinaseproteins gemäß SEQ ID NO: 22 gegen Phenylalanin) und S317A (Austausch von Serin an Position 317 des kodierten Aspartatkinaseproteins gemäß SEQ ID NO: 22 gegen Alanin).

Das lysC^{FBR}-Allel lysC T311I ist in dem bei der DSMZ hinterlegten Stamm DM1797 enthalten. DM1797 ist eine Mutante von Corynebacterium glutamicum ATCC13032.

Der Stamm NRRL B-11474 besitzt ein lysC^{FBR}-Allel, das für ein Aspartatkinaseprotein kodiert, welches die Aminosäureaustausche A279T und S381F enthält.

Nach der oben beschriebenen Art und Weise wurde, ausgehend von Stamm DM1797, eine als DM1816 bezeichnete Mutante isoliert, die ein zwf-Allel enthält, das für ein Polypeptid kodiert, bei dem an Position 321 der Aminosäuresequenz L-Serin enthalten ist. Die Nukleotidsequenz der Kodierregion des zwf-Allels der Mutante DM1816 ist als SEQ ID NO:9 und die Aminosäuresequenz des kodierten Polypeptids als SEQ ID NO:10 beziehungsweise 12 dargestellt. Die Mutante DM1816 enthält darüber hinaus Nukleotidaustausche in der Nukleotidsequenz zwischen Position 1 bis 307 von SEQ ID NO:3. Diese Nukleotidaustausche sind in SEQ ID NO:11 dargestellt. SEQ ID NO:11 enthält an Position 208 Guanin anstelle Adenin, an Position 235 Adenin anstelle Guanin, an Position 245 Cytosin anstelle Thymin, an Position 257 Guanin anstelle Adenin und an Position 299 Guanin anstelle Adenin. SEQ ID NO:11 enthält weiterhin an Position 329 Adenin anstelle Thymin, an Position 445 Thymin anstelle Cytosin, an Position 586 Thymin anstelle Cytosin, an Position 1045 Cytosin anstelle Thymin, an Position 1084 Thymin anstelle Cytosin, an Position 1213 Adenin anstelle Guanin und an Position 1268 Adenin anstelle Guanin.

Nach der oben beschriebenen Art und Weise wurde weiterhin, ausgehend von Stamm NRRL B-11474, eine als DM1889 bezeichnete Mutante isoliert, die ein zwf-Allel enthält, das für ein Polypeptid kodiert, bei dem an Position 321 der Aminosäuresequenz L-Serin enthalten ist.

Darüberhinaus können L-Lysin ausscheidende coryneforme Bakterien verwendet werden, die eine abgeschwächte Homoserin-Dehydrogenase oder Homoserinkinase aufweisen oder andere Eigenschaften besitzen, wie sie aus dem Stand der Technik bekannt sind.

L-Tryptophan produzierende coryneforme Bakterien besitzen typischerweise eine "feed back" resistente beziehungsweise desensibilisierte Anthranilatsynthase. Unter "feed back" resistenter Anthranilatsynthase versteht man Anthranilatsynthasen, die im Vergleich zur Wildform eine geringere Empfindlichkeit gegenüber der Hemmung (5 bis 10%, 10% bis 15% oder 10% bis 20%)durch Tryptophan oder 5-Fluorotryptophan (Matsui et al., Journal of Bacteriology 169 (11): 5330 - 5332(1987)) oder ähnliche Analoga aufweisen. Die für diese desensibilisierten Anthranilatsynthasen kodierenden Gene beziehungsweise Allele werden auch als trpE^{FBR} -Allele bezeichnet. Beispiele für derartige Mutanten beziehungsweise Allele sind beispielsweise in der US 6180373 und EP0338474 beschrieben.

Die erhaltenen Mutanten zeigen eine im Vergleich zum eingesetzten Ausgangsstamm beziehungsweise Elternstamm erhöhte Ausscheidung beziehungsweise Produktion der gewünschten Aminosäure in einem Fermentationsprozess.

Gegenstand der Erfindung ist ebenfalls ein isoliertes Polynukleotid, das für ein Polypeptid gemäβ SEQ ID NO: 2 mit Glucose-6-Phosphat-Dehydrogenase Enzymaktivität kodiert, welches an Position 321 der Aminosäuresequenz SEQ ID NO:2 L-Serin enthält.

Das erfindungsgemäße Polynukleotid kann aus einer erfindungsgemäßen Mutante isoliert werden.

Weiterhin können für die Mutagenese des zwf-Gens in-vitro Methoden eingesetzt werden. Bei der Verwendung von in-vitro Methoden werden isolierte Polynukleotide, welche ein zwf-Gen eines coryneformen Bakteriums, vorzugsweise das im Stand der Technik beschriebene Wildtyp-Gen von Corynebacterium glutamicum, enthalten, einer mutagenen Behandlung unterzogen.

Bei den isolierten Polynukleotiden kann es sich beispielsweise um isolierte Gesamt-DNA beziehungsweise chromosomale DNA oder auch um Amplifikate des zwf-Gens handeln, die mit Hilfe der Polymerasekettenreaktion (PCR) hergestellt wurden. Derartige Amplifikate werden auch als PCR-Produkte bezeichnet. Anleitungen zur Amplifikation von DNA-Sequenzen mit Hilfe der Polymerase-Kettenreaktion findet der Fachmann unter anderem im Handbuch von Gait: Oligonucleotide Synthesis: A Practical Approach (IRL Press, Oxford, UK, 1984) und bei Newton und Graham: PCR (Spektrum Akademischer Verlag, Heidelberg, Deutschland, 1994). Es ist ebenfalls möglich, das zu mutagenisierende zwf-Gen zunächst in einen Vektor, beispielsweise in einen Bakteriophagen oder in ein Plasmid einzubauen.

Geeignete Methoden für die in-vitro Mutagenese sind unter anderem die Behandlung mit Hydroxylamin nach Miller (Miller, J. H.: A Short Course in Bacterial Genetics. A Laboratory Manual and Handbook for Escherichia coli and Related Bacteria, Cold Spring Harbor Laboratory Press, Cold Spring Harbor, 1992), die Verwendung mutagener Oligonukleotide (T. A. Brown: Gentechnologie für Einsteiger, Spektrum Akademischer Verlag, Heidelberg, 1993 und R. M. Horton: PCR-Mediated Recombination and Mutagenesis, Molecular Biotechnology 3, 93-99 (1995)) und der Einsatz einer Polymerasekettenreaktion unter Verwendung einer DNA-Polymerase, die eine hohe Fehlerquote aufweist. Eine derartige DNA-Polymerase ist beispielsweise die Mutazyme DNA Polymerase (GeneMorph PCR Mutagenesis Kit, Nr.600550) der Firma Stratagene (LaJolla, CA, USA).

Weitere Anleitungen und Übersichten zur Erzeugung von Mutationen in-vivo oder in-vitro können dem Stand der Technik und bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden. Gegenstand der Erfindung ist weiterhin ein isoliertes Polynukleotid, dass für ein Polypeptid mit Glucose-6-Phosphat-Dehydrogenase Enzymaktivität kodiert, welches die Aminosäuresequenz von SEQ ID NO:2 umfasst, wobei an Position 321 der Aminosäuresequenz L-Serin enthalten ist. Gegebenenfalls enthält die Aminosäuresequenz des Polypeptids darüberhinaus einen Aminosäureaustausch L-Serin gegen eine andere Aminosäure, vorzugsweise L-Threonin, an Position 8.

Die Beschreibung offenbart weiterhin ein isoliertes Polynukleotid, dass für ein Polypeptid mit Glucose-6-Phosphat-Dehydrogenase Enzymaktivität kodiert, welches eine Aminosäuresequenz mit einer Länge von 514 Aminoäuren umfasst und wobei an Position 321 jede proteinogene L-Aminosäure ausgenommen Glycin, vorzugsweise L-Serin, enthalten ist.

Die Beschreibung offenbart weiterhin ein isoliertes Polynukleotid, dass für ein Polypeptid mit Glucose-6-Phosphat-Dehydrogenase Enzymaktivität kodiert, welches von Position 312 bis 330 der Aminosäuresquenz die Aminosäuresequenz entsprechend Position 312 bis 330 von SEQ ID NO:6 oder 8 enthält. Bevorzugt enthält die Aminosäuresequenz des kodierten Polypeptids eine Aminosäuresequenz entsprechend Position 307 bis 335 von SEQ ID NO:6 oder 8 oder Position 292 bis 350 von SEQ ID NO:6 oder 8 oder Position 277 bis 365 von SEQ ID NO:6 oder 8 oder Position 262 bis 380 von SEQ ID NO:6 oder 8 oder Position 247 bis 395 von SEQ ID NO:6 oder 8 oder Position 232 bis 410 von SEQ ID NO:6 oder 8 oder Position 202 bis 440 von SEQ ID NO:6 oder 8 oder Position 172 bis 470 von SEQ ID NO:6 oder 8 oder Position 82 bis 500 von SEQ ID NO:6 oder 8 oder Position 2 bis 512 von SEQ ID NO:6 oder 8 oder Position 2 bis 513 von SEQ ID NO:6 oder 8 oder Position 2 bis 514 von SEQ ID NO:6 oder 8. Ganz besonders bevorzugt umfasst die Länge des kodierten Polypeptids 514 Aminosäuren.

Die Beschreibung offenbart weiterhin ein isoliertes Polynukleotid, dass für ein Polypeptid mit Glucose-6-Phosphat-Dehydrogenase Enzymaktivität kodiert, welches an Position 321 der Aminosäuresequenz beziehungsweise einer entsprechenden oder vergleichbaren Position jede proteinogene Aminosäure ausgenommen Glycin, bevorzugt L-Serin, enthält und das eine Nukleotidsequenz umfasst, welche mit der Nukleotidsequenz eines Polynukleotids identisch ist, das durch eine Polymerasekettenreaktion (PCR) unter Verwendung des Primerpaars erhältlich ist, deren Nukleotidsequenzen jeweils mindestens 15 aufeinanderfolgende Nukleotide umfassen, die aus der Nukleotidsequenz zwischen Position 1 und 307 von SEQ ID NO:3 oder SEQ ID NO:11 und aus der komplementären Nukleotidsequenz zwischen Position 2100 und 1850 von SEQ ID NO:3 oder SEQ ID NO:11 ausgewählt werden. Beispiele für derartige geeignete Primerpaare sind in SEQ ID NO:17 und SEQ ID NO:18 und in SEQ ID NO:19 und SEQ ID NO:20 dargestellt. Als Ausgangsmaterial ("template"-DNA) wird chromosomale DNA coryneformer Bakterien bevorzugt, die insbesondere mit einem Mutagen behandelt worden sind. Besonders bevorzugt wird die chromosomale DNA der Gattung Corynebacterium und ganz besonders bevorzugt die der Art Corynebacterium glutamicum.

Die Beschreibung offenbart weiterhin ein isoliertes Polynukleotid, das mit der zu SEQ ID NO:5, 7 oder 9 komplementären Nukleotidsequenz unter stringenten Bedingungen hybridisiert und für ein Polypeptid mit Glucose-6-Phosphat-Dehydrogenase Enzymaktivität kodiert, welches an Position 321 der Aminosäuresequenz beziehungsweise einer entsprechenden oder vergleichbaren Position jede proteinogene Aminosäure ausgenommen Glycin, bevorzugt L-Serin, und gegebenenfalls an einer der Position 8 entsprechenden Position jede proteinogene Aminosäure ausgenommen L-Serin, bevorzugt L-Threonin, enthält.

Anleitungen zur Hybridisierung von Nukleinsäuren beziehungsweise Polynukleotiden findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen die Sonde d.h. ein Polynukleotid umfassend die zu SEQ ID NO:5, 7 oder 9 komplementäre Nukleotidsequenz und die Zielsequenz, d. h. die mit der Sonde behandelten beziehungsweise identifizierten Polynukleotide, mindestens 90% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 90% Identität zur Nukleotidequenz der eingesetzten Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich, die Salzkonzentration bis auf eine Konzentration entsprechend 0,2x SSC oder 0,1x SSC zu senken. Gegebenenfalls enthält der SSC-Puffer Natriumdodecylsulfat (SDS) in einer Konzentration von 0,1%. Durch schrittweise Erhöhung der Hybridisierungstemperatur in Schritten von ca. 1 - 2°C von 50°C auf 68°C können Polynukleotidfragmente isoliert werden, die mindestens 90% oder mindestens 91%, bevorzugt mindestens 92% oder mindestens 93% oder mindestens 94% oder mindestens 95% oder mindestens 96% und ganz besonders bevorzugt mindestens 97% oder mindestens 98% oder mindestens 99% Identität zur Sequenz beziehungsweise komplementären Sequenz der eingesetzten Sonde besitzen und für ein Polypeptid mit Glucose-6-Phosphat-Dehydrogenase Enzymaktivität kodieren, welches den erfindungsgemäßen Aminosäureaustausch enthält. Die Nukleotidsequenz des auf diese Weise erhaltenen Polynukleotids wird mit bekannten Methoden bestimmt. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). Die so erhaltenen Nukleotidsequenzen kodieren für Polypeptide mit Glucose-6-Phosphat-Dehydrogenase Enzymaktivität, die mindestens 90% bevorzugt mindestens 92% oder mindestens 94% oder mindestens 96%, und ganz besonders bevorzugt mindestens 97% oder mindestens 98% oder mindestens 99% identisch sind mit der Aminosäuresequenz von SEQ ID NO:6 oder SEQ ID NO:8 und den erfindungsgemäßen Aminosäureaustausch enthalten.

Die Beschreibung offenbart weiterhin ein isoliertes Polynukleotid, das für ein Polypeptid mit Glucose-6-Phosphat-Dehydrogenase Enzymaktivität kodiert, welches an Position 321 beziehungsweise einer entsprechenden oder vergleichbaren Position der Aminosäuresequenz jede Aminosäure ausgenommen Glycin enthält, wobei dem Austausch gegen L-Serin der Vorzug gegeben wird und das eine Aminosäuresequenz umfasst, die außerdem zu mindestens 90%, bevorzugt mindestens 92% oder mindestens 94% oder mindestens 96%, und ganz besonders bevorzugt mindestens 97% oder mindestens 98% oder mindestens 99% identisch ist mit der Aminosäuresequenz von SEQ ID NO:6. Ein Beispiel für ein Polypeptid mit Glucose-6-Phosphat-Dehydrogenase Enzymaktivität, das eine Aminosäuresequenz umfasst, die zu mindestens 99% identisch ist mit der von SEQ ID NO:6, ist in SEQ ID NO:8 und SEQ ID NO:10 gezeigt.

Die Beschreibung offenbart weiterhin ein isoliertes Polynukleotid, das für ein Polypeptid mit Glucose-6-Phosphat-Dehydrogenase Enzymaktivität kodiert, welches an Position 321 beziehungsweise einer entsprechenden oder vergleichbaren Position der Aminosäuresequenz jede Aminosäure ausgenommen Glycin enthält, wobei dem Austausch gegen L-Serin der Vorzug gegeben wird und das eine Nukleotidsequenz umfasst, die außerdem zu mindestens 90%, bevorzugt mindestens 92% oder mindestens 94% oder mindestens 96%, und ganz besonders bevorzugt mindestens 97% oder mindestens 98% oder mindestens 99% identisch ist mit der Nukleotidsequenz von SEQ ID NO:5. Ein Beispiel für ein Polynukleotid, das für ein erfindungsgemäßes Polypeptid mit Glucose-6-Phosphat-Dehydrogenase Enzymaktivität kodiert, und eine Nukleotidsequenz besitzt, die mindestens 99% identisch ist mit der von SEQ ID NO:5, ist in SEQ ID NO:7 gezeigt. Die Nukleotidsequenz dieses zwf-Allels besitzt zusätzlich zu dem Nukleotidaustausch Guanin gegen Adenin an Position 961 (siehe SEQ ID NO:5) den Nukleotidaustausch Thymin gegen Adenin an der Position 22 (siehe SEQ ID NO:7). Ein weiteres Beispiel für eine Nukleotidsequenz eines zwf-Allels, die mindestens 99% Identität mit der Nukleotidsequenz von SEQ ID NO:5 besitzt, ist in SEQ ID NO:9 gezeigt. Die Nukleotidsequenz dieses zwf-Allels besitzt zusätzlich zu dem Nukleotidaustausch Guanin gegen Adenin an Position 961 (siehe SEQ ID NO:5) und dem Nukleotidaustausch Thymin gegen Adenin an der Position 22 (siehe SEQ ID NO:7) die Nukleotidaustausche Cytosin gegen Thymin an Position 138, Cytosin gegen Thymin an Position 279, Thymin gegen Cytosin an Position 738, Cytosin gegen Thymin an Position 777 und Guanin gegen Adenin an Position 906 (siehe SEQ ID NO:9).

Darüberhinaus sind solche isolierte Polynukleotide bevorzugt, die für ein Polypeptid mit Glucose-6-Phosphat-Dehydrogenase Enzymaktivität kodieren, welches an Position 321 der Aminosäuresequenz beziehungsweise einer entsprechenden oder vergleichbaren Position jede Aminosäure ausgenommen Glycin, bevorzugt L-Serin, enthält und die mindestens ein Sequenzmotiv beziehungsweise eine Aminosäuresequenz ausgewählt aus der Gruppe Val-Ile-Phe-Gly-Ali-Afa- Gly-Asp-Leu, Arg-Ile-Asp-His-Tyr-Leu-Gly-Lys, und Arg-Trp-Ala-Gly-Val-Pro-Phe-Tyr-Bra-Arg-Thr-Gly-Lys-Arg enthalten.

Die Bezeichnung "Ali" steht für die Aminosäuren Ala oder Val, die Bezeichnung "Afa" für die Aminosäuren Lys oder Thr und die Bezeichnung "Bra" für die Aminosäuren Ile oder Leu.

Gegenstand der Erfindung ist weiterhin ein isoliertes Polynukleotid, dass für ein Polypeptid mit Glucose-6-Phosphat-Dehydrogenase Enzymaktivität kodiert, welches die Aminosäuresequenz von SEQ ID NO:6 oder 8 beziehungsweise 10 umfasst.

Die Beschreibung offenbart weiterhin ein isoliertes Polynukleotid, dass für ein Polypeptid mit Glucose-6-Phosphat-Dehydrogenase Enzymaktivität kodiert, welches die Aminosäuresequenz von SEQ ID NO:6 oder 8 beziehungsweise 10 einschließlich einer Verlängerung am N- oder C-Terminus um mindestens eine (1) Aminosäure umfasst. Diese Verlängerung beträgt nicht mehr als 50, 40, 30, 20, 10, 5, 3 oder 2 Aminosäuren beziehungsweise Aminosäurereste.

Gegenstand der Erfindung sind schließlich auch zwf-Allele, die für Polypeptid Varianten von SEQ ID NO:6, 8 oder 10 kodieren, die eine oder maximal 5, maximal 4, maximal 3 oder maximal 2 Insertionen von Aminosäuren und an der der Position 321 entsprechenden Position L-Serin enthalten. Das Sequenzmotive Val-Ile-Phe-Gly-Ali-Afa-Gly-Asp-Leu und/oder Arg-Ile-Asp-His-Tyr-Leu-Gly-Lys und/oder Arg-Trp-Ala-Gly-Val-Pro-Phe-Tyr-Bra-Arg-Thr-Gly-Lys-Arg wird durch derartige Insertionen/Deletionen vorzugsweise nicht zerrissen.

Gegenstand der Erfindung ist weiterhin ein isoliertes Polynukleotid, das die Nukleotidsequenz gemäß SEQ ID NO:5, 7, 9 oder 11 umfasst.

Die Beschreibung offenbart weiterhin ein isoliertes Polynukleotid, das die Nukleotidsequenz zwischen Position 1 und 307 von SEQ ID NO:11, bevorzugt die Nukleotidsequenz zwischen Position 198 und 304 von SEQ ID NO:11 und ganz besonders bevorzugt die Nukleotidsequenz zwischen Position 208 und 299 von SEQ ID NO:11 umfasst.

Gegenstand der Erfindung ist schließlich ein isoliertes Polynukleotid enthaltend das zwf-Allel der Mutante DM1816.

Gegenstand der Erfindung ist außerdem ein isoliertes Polynukleotid, das einen Teil der Kodierregion eines erfindungsgemäßen zwf-Allels umfasst, wobei das isolierte Polynukleotid in jedem Fall den Teil der Kodierregion umfasst, der den Aminosäureaustausch an der Position 321 der Aminosäuresequenz des kodierten Polypeptids enthält. Insbesondere ist ein Nukleinsäure-Molekül beziehungsweise DNA-Fragment umfasst, das für mindestens eine Aminosäuresequenz entsprechend Position 307 bis 335 von SEQ ID NO:2, oder das für mindestens eine Aminosäuresequenz entsprechend Position 292 bis 350 von SEQ ID NO:2, oder das für mindestens eine Aminosäuresequenz entsprechend Position 277 bis 365 von SEQ ID NO:2, oder das für mindestens eine Aminosäuresequenz entsprechend Position 262 bis 380 von SEQ ID NO:2, oder das für mindestens eine Aminosäuresequenz entsprechend Position 247 bis 395 von SEQ ID NO:2, oder das für mindestens eine Aminosäuresequenz entsprechend Position 232 bis 410 von SEQ ID NO:2, oder das für mindestens eine Aminosäuresequenz entsprechend Position 202 bis 440 von SEQ ID NO:2 kodiert, oder das für mindestens eine Aminosäuresequenz entsprechend Position 172 bis 470 von SEQ ID NO:2 kodiert, oder das für mindestens eine Aminosäuresequenz entsprechend Position 82 bis 500 von SEQ ID NO:2 kodiert, oder das für mindestens eine Aminosäuresequenz entsprechend Position 2 bis 512 von SEQ ID NO:2 kodiert, oder das für mindestens eine Aminosäuresequenz entsprechend Position 2 bis 513 von SEQ ID NO:2 kodiert, oder das für mindestens eine Aminosäuresequenz entsprechend Position 2 bis 514 von SEQ ID NO:2 kodiert beziehungsweise ein entsprechendes Leseraster beinhaltet, wobei an der Position entsprechend 321 von SEQ ID NO:2 jede proteinogene Aminsäure ausgenommen Glycin, bevorzugt L-Serin, enthalten ist und wobei gegebenenfalls an der Position entsprechend 8 jede proteinogene Aminosäure außer L-Serin, bevorzugt L-Threonin, enthalten ist.

in Beispiel eines erfindungsgemäßen Leserasters umfassend ein Polynukleotid, das für mindestens die Aminosäuresequenz von Position 307 bis 335 entsprechend SEQ ID NO:2 kodiert, wobei an der Position entsprechend 321 der Aminosäuresequenz L-Serin enthalten ist, ist nachfolgend aufgeführt:

Es ist ebenfalls als SEQ ID NO:13 dargestellt. Die von diesem Leseraster kodierte Aminosäuresequenz ist als SEQ ID NO:14 dargestellt. Die Position 15 in SEQ ID NO:14 entspricht der Position 321 von SEQ ID NO:6, 8, 10 oder 12.

Bevorzugt werden Nukleinsäuremoleküle, die für mindestens eine Aminosäuresequenz entsprechend Position 307 bis 335 von SEQ ID NO:6 oder 8 beziehungsweise 10 kodieren.

Ein Beispiel eines erfindungsgemäßen Leserasters umfassend ein Polynukleotid, das für mindestens die Aminosäuresequenz entprechend Position 307 bis 335 von SEQ ID NO:6 kodiert, ist nachfolgend aufgeführt:

Das Leseraster ist ebenfalls als SEQ ID NO:15 dargestellt. SEQ ID NO:16 zeigt die von diesem Leseraster kodierte Aminosäuresequenz. Die Position 15 in SEQ ID NO:16 entspricht der Position 321 von SEQ ID NO:6, 8, 10 oder 12.

Ganz besonders bevorzugt werden Nukleinsäuremoleküle, die mindestens eine Nukleotidsequenz entsprechend Position 919 bis 1005 von SEQ ID NO:5, 7 oder 9 umfassend.

Die erfindungsgemäßen Leseraster, wie sie beispielhaft in SEQ ID NO:13 und 15 als Nukleotidsequenz und in SEQ ID NO:14 und SEQ ID NO:16 in Form der kodierten Aminosäuresequenz gezeigt sind, können darüberhinaus eine oder mehrere Mutationen enthalten, die zu einem oder mehreren konservativen Aminosäureaustauschen führen. Bevorzugt führen die Mutationen zu maximal 4%, zu maximal 2% oder zu maximal 1% konservativen Aminosäureaustauschen. Weiterhin können die erfindungsgemäßen Leseraster eine oder mehrere stille Mutationen enthalten. Bevorzugt enthalten die erfindungsgemäßen Leseraster höchstens 4% und besonders bevorzugt höchstens 2% bis höchstens 1% stille Mutationen.

Die erfindungsgemäßen, isolierten Polynukleotide können dazu verwendet werden, um rekombinante Stämme von Mikroorganismen herzustellen, die im Vergleich zum Ausgangs- beziehungsweise Elternstamm in verbesserter Weise Aminosäuren in das sie umgebende Medium abgeben oder im Zellinneren anhäufen.

Eine verbreitete Methode zum Einbau von Mutationen in Gene coryneformer Bakterien ist die des Allelaustausches, die auch unter der Bezeichnung "gene replacement" bekannt ist. Bei diesem Verfahren wird ein DNA-Fragment, welches die interessierende Mutation enthält, in den gewünschten Stamm eines coryneformen Bakteriums überführt und die Mutation durch wenigstens zwei Rekombinationsereignisse beziehungsweise "cross over"-Ereignisse in das Chromosom des gewünschten Stammes inkorporiert beziehungsweise die im betreffenden Stamm vorhandene Sequenz eines Gens gegen die mutierte Sequenz ausgetauscht.

Von Schwarzer und Pühler (Bio/Technology 9, 84-87 (1991) wurde diese Methode verwendet um ein lysA-Allel, welches eine Deletion trug, und um ein lysA-Allel, welches eine Insertion trug in das Chromosom von C. glutamicum anstelle des Wildtypgens einzubauen. Von Schäfer et al. (Gene 145, 69-73 (1994)) wurde diese Methode eingesetzt um eine Deletion in das hom-thrB Operon von C. glutamicum einzubauen. Von Nakagawa et al. (EP 1108790) und Ohnishi et al. (Applied Microbiology and Biotechnology 58(2), 217-223 (2002)) wurde diese Methode eingesetzt um verschiedene Mutationen ausgehend von den isolierten Allelen in das Chromosom von C. glutamicum einzubauen. Auf diese Weise gelang es Nakagawa et al. eine als Val59Ala bezeichnete Mutation in das Homoserin-Dehydrogenase Gen (hom), eine als Thr311IIle bezeichnete Mutation in das Aspartatkinase-Gen (lysC bzw, ask), eine als Pro458Ser bezeichnete Mutation in das Pyruvat-Carboxylase-Gen (pyc) und eine als Ala213Thr bezeichnete Mutation in das Glucose-6-phoshat-Dehydrogenase Gen (zwf) von C. glutamicum Stämmen einzubauen.

Für ein erfindungsgemäßes Verfahren kann ein erfindungsgemäßes Polynukleotid verwendet werden, welches die gesamte Kodierregion umfasst, wie beispielsweise in SEQ ID NO:5, 7 oder 9 gezeigt, oder welches einen Teil der Kodierregion umfasst, wie beispielsweise die Nukleotidsequenz, die für mindestens die Aminosäuresequenz entsprechend Position 307 bis 335 von SEQ ID NO:6, 8 oder 10 kodiert und die als SEQ ID NO:13 und 15 dargestellt sind. Der Teil der Kodierregion entsprechend SEQ ID NO:13 und 15 hat eine Länge von ≥ 87 Nukleobasen. Bevorzugt werden solche Teile der Kodierregion, deren Länge ≥ 267 Nukleobasen beträgt, wie beispielsweise Nukleinsäuremoleküle, die für mindestens eine Aminosäuresequenz entsprechend Position von 277 bis 365 von SEQ ID NO:6, 8 oder 10 kodieren. Ganz besonders bevorzugt werden solche Teile der Kodierregion, deren Länge ≥ 357 Nukleobasen beträgt, wie beispielsweise Nukleinsäuremoleküle, die für mindestens eine Aminosäuresequenz entsprechend Position von 262 bis 380 von SEQ ID NO:6, 8 oder 10 kodieren.

Das DNA-Fragment enthaltend die interessierende Mutation liegt bei dieser Methode typischerweise in einem Vektor, insbesondere einem Plasmid, vor, das vorzugsweise von dem mit der Mutation zu versehenden Stamm nicht oder nur begrenzt repliziert wird. Als Hilfs- oder Zwischenwirt, in dem der Vektor replizierbar ist, wird im Allgemeinen ein Bakterium der Gattung Escherichia bevorzugt der Spezies Escherichia coli verwendet.

Beispiele für derartige Plasmidvektoren sind die von Schäfer et al. (Gene 145, 69-73 (1994)) beschriebenen pK*mob und pK*mobsacB Vektoren, wie beispielsweise pK18mobsacB, und die in der WO 02/070685 und WO 03/014362 beschriebenen Vektoren. Diese sind in Escherichia coli aber nicht in coryneformen Bakterien replikativ. Besonders geeignet sind Vektoren, die ein konditional negativ dominant wirkendes Gen wie beispielsweise das sacB-Gen (Levansucrase-Gen) von beispielsweise Bacillus oder das galK-Gen (Galaktosekinase-Gen) von beispielsweise Escherichia coli enthalten. (Unter einem konditional negativ dominant wirkenden Gen versteht man ein Gen, das unter bestimmten Bedingungen nachteilig beispielsweise toxisch für den Wirt ist, unter anderen Bedingungen aber keine negativen Auswirkungen auf den das Gen tragenden Wirt hat.) Diese ermöglichen die Selektion auf Rekombinationsereignisse, bei denen der Vektor aus dem Chromosom eliminiert wird. Weiterhin wurde von Nakamura et al. (US-A-6,303,383) ein Temperatur-sensitives Plasmid für coryneforme Bakterien beschrieben, das lediglich bei Temperaturen unterhalb von 31°C replizieren kann.

Der Vektor wird anschließend durch Konjugation beispielsweise nach der Methode von Schäfer (Journal of Bacteriology 172, 1663-1666 (1990)) oder Transformation beispielsweise nach der Methode von Dunican und Shivnan (Bio/Technology 7, 1067-1070 (1989)) oder der Methode von Thierbach et al. (Applied Microbiology and Biotechnology 29, 356-362 (1988)) in das coryneforme Bakterium überführt. Gegebenenfalls kann die Überführung der DNA auch durch Partikelbeschuss erzielt werden.

Nach homologer Rekombination mittels eines ersten, Integration bewirkenden "cross-over"-Ereignisses und eines geeigneten zweiten, eine Exzision bewirkenden "cross-over"-Ereignisses im Zielgen bzw. in der Zielsequenz erreicht man den Einbau der Mutation und erhält ein rekombinantes Bakterium.

Zur Identifizierung und Charakterisierung der erhaltenen Stämme können unter anderem die Methoden der Southern Blotting Hybridisierung, der Polymerase-Kettenreaktion, der Sequenzbestimmung, die Methode des "Fluorescence Resonance Energy Transfer" (FRET) (Lay et al. Clinical Chemistry 43, 2262-2267 (1997)) oder Methoden der Enzymologie eingesetzt werden.

Ein weiterer Gegenstand der Erfindung ist dementsprechend ein Verfahren zur Herstellung eines coryneformen Bakteriums, bei dem man
a) ein erfindungsgemäßes Polynukleotid in ein coryneformes Bakterium überführt,
b) das im Chromosom des coryneformen Bakteriums vorhandene Glucose-6-Phosphat-Dehydrogenase Gen, das für eine Aminosäuresequenz mit Glycin an Position 321 und gegebenenfalls L-Serin an Position 8 oder einer vergleichbaren Position der Aminosäuresequenz SEQ ID NO:2 kodiert, gegen das Polynukleotid aus a) austauscht, und
c) das gemäß Schritt a) und b) erhaltene coryneforme Bakterium vermehrt.

Auf diese Weise erhält man ein rekombinantes coryneformes Bakterium, welches anstelle des Wildtyp zwf-Gens ein (1) erfindungsgemäßes zwf-Allel enthält.

Ein weiteres erfindungsgemässes Verfahren zur Herstellung eines Mikroorganismus besteht darin, dass man
a) ein erfindungsgemäßes Polynukleotid, das für ein Polypeptid mit Glucose-6-Phosphat-Dehydrogenase Enzymaktivität kodiert, in einen Mikrorganismus überführt,
b) das Polynukleotid in dem Mikroorganismus repliziert, und
c) den gemäß Schritt a) und b) erhaltenen Mikroorganismus vermehrt.

Auf diese Weise erhält man einen rekombinanten Mikroorganismus, welcher mindestens eine (1) Kopie oder mehrere Kopien eines erfindungsgemäßen Polynukleotids enthält, das für eine Glucose-6-Phosphat-Dehydrogenase kodiert, die an Position 321 oder einer vergleichbaren Position der Aminosäuresequenz des kodierten Polypeptids, jede proteinogene Aminosäure ausgenommen Glycin enthält, wobei der Austausch gegen L-Serin bevorzugt wird. Gegebenenfalls enthält das Polypeptid an Position 8 oder einer vergleichbaren Position jede proteinogene Aminosäure ausgenommen L-Serin, bevorzugt die Aminosäure L-Threonin.

Weitere Gegenstände der Erfindung sind dementsprechend Wirte beziehungsweise Wirtszellen, bevorzugt Mikroorganismen, besonders bevorzugt coryneforme Bakterien und Bakterien der Gattung Escherichia, die die erfindungsgemäßen Polynukleotide enthalten. Gegenstand der Erfindung sind gleichfalls Mikrorganismen, die unter Verwendung der isolierten Polynukleotide hergestellt wurden. Derartige Mikroorganismen oder Bakterien werden auch als rekombinante Mikroorganismen oder rekombinante Bakterien bezeichnet. In gleicher Weise sind Vektoren, die die erfindungsgemäßen Polynukleotide enthalten, Gegenstand der Erfindung. Schließlich sind Wirte, die diese Vektoren enthalten, ebenfalls Gegenstand der Erfindung.

Die erfindungsgemäßen, isolierten Polynukleotide können ebenfalls zur Erzielung einer Überexpression der von ihnen kodierten Polypeptide verwendet werden.

Unter Überexpression versteht man allgemein eine Erhöhung der intrazellulären Konzentration oder Aktivität einer Ribonukleinsäure, eines Proteins oder eines Enzyms. Im Falle der vorliegenden Erfindung werden zwf-Allele beziehungsweise Polynukleotide überexprimiert, die für Glucose-6-Phosphat-Dehydrogenasen kodieren, die an Position 321 der Aminosäuresequenz des kodierten Polypeptids jede proteinogene Aminosäure ausgenommen Glycin enthalten, wobei der Austausch gegen L-Serin bevorzugt wird. Gegebenenfalls enthält das kodierte Protein außerdem einen Austausch von L-Serin gegen eine andere proteinogene Aminosäure, bevorzugt L-Threonin an Position 8 der Aminosäuresequenz. Es ist bekannt, dass durch wirtseigene Enzyme - sogenannte Aminopeptidasen - N-terminale Aminosäuren, insbesondere das N-terminale Methionin, vom gebildeten Polypeptid abgespalten werden können. Die genannte Erhöhung der Konzentration oder Aktivität eines Genproduktes lässt sich beispielsweise dadurch erzielen, dass man die Kopienzahl der entsprechenden Polynukleotide um mindestens eine Kopie erhöht.

Eine weit verbreitete Methode zur Erhöhung der Kopienzahl besteht darin, dass man das entsprechende Gen oder Allel in einen Vektor, bevorzugt ein Plasmid, einbaut, der von einem coryneformen Bakterium repliziert wird. Geeignete Plasmidvektoren sind beispielsweise pZ1 (Menkel et al., Applied and Environmental Microbiology (1989) 64: 549-554) oder die von Tauch et al. (Journal of Biotechnology 99, 79-91 (2002)) beschriebenen pSELF-Vektoren. Ein

Übersichtsartikel zum Thema Plasmide in Corynebacterium glutamicum findet sich bei Tauch et al. (Journal of Biotechnology 104, 27-40 (2003)).

Eine andere verbreitete Methode zur Erzielung einer Überexpression ist das Verfahren der chromosomalen Genamplifikation. Bei dieser Methode wird mindestens eine zusätzliche Kopie des interessierenden Gens oder Allels in das Chromosom eines coryneformen Bakteriums eingefügt.

In einer Ausführungsform, wie sie beispielsweise bei Reinscheid et al. (Applied and Environmental Microbiology 60, 126-132 (1994)) für das hom-thrB-Operon beschrieben ist, wird ein in C. glutamicum nicht-replikatives Plasmid, welches das interessierende Gen enthält, in ein coryneformes Bakterium überführt. Nach homologer Rekombination mittels eines "cross over"-Ereignisses enthält der resultierende Stamm mindestens zwei Kopien des betreffenden Gens beziehungsweise Allels.

In einer anderen Ausführungsform, die in der WO 03/040373 und US-2003-0219881-A1 beschrieben ist, wird eine oder mehrere Kopie(n) des interessierenden Gens mittels mindestens zweier Rekombinationsereignisse in einen gewünschten Ort des Chromosoms von C. glutamicum eingefügt. Auf diese Weise wurde beispielsweise eine Kopie eines lysC-Allels, das für eine L-Lysin insensitive Aspartatkinase kodiert, in das gluB-Gen von C. glutamicum eingebaut.

In einer weiteren Ausführungsform, die in der WO 03/014330 und US-2004-0043458-A1 beschrieben ist, wird mittels mindestens zweier Rekombinationsereignisse am natürlichen Ort mindestens eine weitere Kopie, vorzugsweise in tandem-Anordnung zu dem bereits vorhandenen Gen oder Allel, des interessierenden Gens eingebaut. Auf diese Weise wurde beispielsweise eine tandem-Duplikation eines lysC^{FBR}-Allels am natürlichen lysC-Genort erzielt.

Eine weitere Methode zur Erzielung einer Überexpression besteht darin, das entsprechende Gen beziehungsweise Allel in funktioneller Weise (operably linked) mit einem Promotor beziehungsweise einer Expressionskassette zu verknüpfen. Geeignete Promotoren für Corynebacterium glutamicum sind beispielsweise in dem Übersichtsartikel von Patek et al. (Journal of Biotechnology 104(1-3), 311-323 (2003) beschrieben. Weiterhin können die hinlänglich bekannten von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden. Ein derartiger Promotor kann beispielsweise stromaufwärts des zwf-Allels, typischerweise im Abstand von ungefähr 1 - 500 oder 1 -307 Nukleotiden vom Startkodon, eines rekombinanten coryneformen Bakteriums eingefügt werden, welches anstelle der an Position 321 natürlicherweise vorhanden Aminosäure Glycin eine andere proteinogene Aminsäure enthält. Ein derartiger Promotor kann naturgemäß ebenfalls stromaufwärts des zwf-Allels einer erfindungsgemäßen Mutante eingefügt werden. Es ist weiterhin möglich ein erfindungsgemäßes isoliertes Polynukleotid, das für eine erfindungsgemäße Variante der Glucose-6-Phosphat-Dehydrogenase kodiert, mit einem Promotor zu verknüpfen und die erhaltene Expressionseinheit in ein extrachromosomal replizierendes Plasmid oder in das Chromosom eines coryneformen Bakteriums einzubauen.

Darüberhinaus kann die Promotor- und Regulationsregion oder die Ribosomenbindungsstelle, die sich stromaufwärts des Strukturgens befindet, mutiert werden. Ein Beispiel für eine mutierte Promotorregion des zwf-Gens beziehungsweise zwf-Allels ist die Nukleotidsequenz umfassend Position 208 bis 299 von SEQ ID NO:11. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Alternativ kann weiterhin eine Überexpression des betreffenden Gens oder Allels durch Veränderung der Medienzusammensetzung und Kulturführung erreicht werden.

Durch die Maßnahmen der Überexpression wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000%, bezogen auf die Aktivität oder Konzentration des Proteins im Ausgangs-Mikroorganismus beziehungsweise Elternstammes erhöht. Unter einem Ausgangs-Mikroorganismus oder Elternstamm versteht man einen Mikroorganismus, an dem die Massnahmen der Erfindung durchgeführt werden.

Eine Methode zur Bestimmung der enzymatischen Aktivität der Glucose-6-Phosphat-Dehydrogenase ist bei Moritz et al. (European Journal of Biochemistry 267, 3442-3452 (2000) beschrieben.

Die Konzentration des Proteins kann über 1- und 2-dimensionale Proteingelauftrennung und anschließende optische Identifizierung der Proteinkonzentration mit enstprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit ensprechender Software zur Konzentrationsbestimmung (Lohaus und Meyer (1998) Biospektrum 5:32-39; Lottspeich, Angewandte Chemie 321: 2630-2647 (1999)) bestimmt werden.

Gegenstand der Erfindung sind dementsprechend Verfahren zur Überexpression der erfindungsgemäßen Glucose-6-Phosphat-Dehydrogenasen. Ein erfindungsgemässes Verfahren zur Überexpression besteht unter anderem darin, dass man die Kopienzahl eines erfindungsgemässen Polynukleotids, das für eine Glucose-6-Phosphat-Dehydrogenase Variante kodiert, bei der an Position 321 oder der entsprechenden Position der kodierten Aminosäuresequenz jede proteinogene Aminosäure ausgenommen Glycin und bei der gegebenenfalls an Position 8 oder der entsprechenden Position jede proteinogene Aminosäure ausgenommen L-Serin enthalten ist, um mindestens eine (1) oder um mehrere Kopien erhöht. Ein weiteres erfindungsgemässes Verfahren besteht darin, dass man einen Promotor funktionell mit dem Polynukleotid verknüpft.

Gegenstand der Erfindung sind weiterhin Mikroorganismen, die eine erhöhte Konzentration oder Aktivität der erfindungsgemäßen Glucose-6-Phosphat-Dehydrogenase Varianten in ihrem Zellinneren aufweisen.

Zusätzlich kann es für die verbesserte Produktion von L-Aminosäuren vorteilhaft sein, in den erfindungsgemäßen Mutanten oder rekombinanten Stämme eines oder mehrere Enzyme des jeweiligen Biosyntheseweges, der Glykolyse, der Anaplerotik, des Zitronensäure-Zyklus, des Pentosephosphat-Zyklus, des Aminosäure-Exports und gegebenenfalls regulatorische Proteine überzuexprimieren. Die Verwendung endogener Gene wird im allgemeinen bevorzugt.

Unter "endogenen Genen" oder "endogenen Nukleotidsequenzen" versteht man die in der Population einer Art vorhandenen Gene beziehungsweise Nukleotidsequenzen oder Allele.

So kann für die Herstellung von L-Lysin eines oder mehrere der Gene, ausgewählt aus der Gruppe
- ein für ein Dihydrodipicolinat-Synthase kodierendes Gen dapA, wie beispielsweise das in der EP 0 197 335 beschriebene dapA-Gen des Wildtyps von Corynebacterium glutamicum,
- ein für eine Glucose-6-Phosphat Dehydrogenase kodierendes Gen zwf, wie beispielsweise das in der JP-A-09224661 und EP-A-1108790 beschriebene zwf-Gen des Wildtyps von Corynebacterium glutamicum,
- die in der US-2003-0175911-A1 beschriebenen zwf-Allele von Corynebacterium glutamicum, die für ein Protein kodieren, bei dem beispielsweise das L-Alanin an Position 243 der Aminosäuresequenz durch L-Threonin ersetzt ist oder bei dem die L-Asparaginsäure an Position 245 durch L-Serin ersetzt ist,
- ein für eine Pyruvat-Carboxylase kodierendes Gen pyc, wie beispielsweise das in der DE-A-198 31 609 und EP 1108790 beschriebene pyc-Gen des Wildtyps von Corynebacterium glutamicum,
- das für das in der EP 1 108 790 beschriebene pyc-Allel von Corynebacterium glutamicum, das für ein Protein kodiert, bei dem L-Prolin an Position 458 der Aminosäuresequenz durch L-Serin ersetzt ist,
- die in der WO 02/31158 beschriebene pyc-Allele von Corynebacterium glutamicum, die für Proteine kodieren, welche gemäß Aspruch 1 einen oder mehrere der Aminosäureaustausche ausgewählt aus der Gruppe L-Glutaminsäure an Position 153 ersetzt durch L-Asparaginsäure, L-Alanin an Position 182 ersetzt durch L-Serin, L-Alanin an Position 206 ersetzt durch L-Serin, L-Histidin an Position 227 ersetzt durch L-Arginin, L-Alanin an Position 452 ersetzt durch Glycin und L-Asparaginsäure an Position 1120 ersetzt durch L-Glutaminsäure tragen (Figur 2A von WO 02/31158 gibt zwei verschiedene Startpositionen für die Pyruvat-Carboxylase an, die sich um eine Länge entsprechend 17 Aminosäuren unterscheiden. Dementsprechend entspricht die Position 153 nach Anspruch 1 von WO 02/31158 der Position 170 von Fig.2A von WO 02/31158, die Position 182 nach Anspruch 1 der Position 199 von Fig.2A, die Position 206 nach Anspruch 1 der Position 223 von Fig. 2A, die Position 227 nach Anspruch 1 der Position 244 von Fig.2A, die Position 452 nach Anspruch 1 der Position 469 von Fig. 2A, die Position 1120 nach Anspruch 1 der Position 1137 von Fig.2B. In Figur 2A von WO 02/31158 ist weiterhin ein Aminosäureaustausch A (Alanin) gegen G (Glycin) an Position 472 angegeben. Die Position 472 des Proteins mit der N terminalen Sequenz MTA entspricht der Position 455 des Proteins mit der N-terminalen Sequenz MST gemäß Fig. 2A. In Fig. 2B von WO 02/31158 ist weiterhin ein Aminosäureaustausch D (Asparaginsäure) gegen E (Glutaminsäure) an Position 1133 des Proteins mit dem N-Terminus MTA angegeben.),
- ein für eine Aspartatkinase kodierendes lysC Gen wie beispielsweise das als SEQ ID NO:281 in der EP-A-1108790 (Siehe auch Zugangsnummer AX120085 und 120365) und das als SEQ ID NO:25 in der WO 01/00843 (Siehe Zugangsnummer AX063743) beschriebene von lysC-Gen des Wildtyps von Corynebacterium glutamicum,
- ein für eine feed-back resistente Aspartatkinase Variante kodierendes lysC^{FBR} Allel, insbesondere entsprechend Tabelle 1,
- ein für ein Lysin-Export-Protein kodierendes Gen lysE, wie beispielsweise das in der DE-A-195 48 222 beschriebene lysE-Gen von des Wildtyps Corynebacterium glutamicum,
- das für das Zwa1-Protein kodierende Gen zwa1 des Wildtyps von Corynebacterium glutamicum (US 6,632,644)
überexprimiert werden.

Weiterhin kann es für die Produktion von L-Lysin vorteilhaft sein, neben der Verwendung der erfindungsgemäßen Allele des zwf-Gens gleichzeitig eines oder mehrere der endogenen Gene, ausgewählt aus der Gruppe
- ein für die Glucose-6-Phosphat-Isomerase kodierendes Gen pgi, wie beispielsweise das in der US 6,586,214 und US 6,465,238 beschriebene pgi-Gen von Corynebacterium glutamicum,
- ein für die Homoserin-Dehydrogenase kodierendes Gen hom, wie beispielsweise das in der EP-A-0131171 beschriebene hom-Gen von Corynebacterium glutamicum,
- ein für die Homoserin-Kinase kodierendes Gen thrB, wie beispielsweise das von Peoples et al. (Molecular Microbiology 2 (1988): 63 - 72)) beschriebene thrB-Gen von Corynebacterium glutamicum und
- ein für die Phosphofruktokinase kodierendes Gen pfkB, wie beispielsweise das in der WO 01/00844 (Sequenz Nr. 57) beschriebene pfkB-Gen von Corynebacterium glutamicum,
abzuschwächen oder auszuschalten.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im Ausgangs-Mikroorganismus, herabgesenkt.

Als Mutationen zur Erzeugung einer Abschwächung kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nichtkonservativen Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stopp-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stopp-Kodon im Kodierbereich, so führt dies ebenfalls zu einem vorzeitigen Abbruch der Translation.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden. Weitere Maßnahmen sind im Stand der Technik beschrieben.

Die durch die Massnahmen der Erfindung erhaltenen isolierten coryneformen Bakterien zeigen eine im Vergleich zum eingesetzten Ausgangsstamm beziehungsweise Elternstamm erhöhte Ausscheidung beziehungsweise Produktion der gewünschten Aminosäure in einem Fermentationsprozess.

Unter isolierten Bakterien sind die erfindungsgemäßen, isolierten beziehungsweise erzeugten Mutanten und rekombinanten Bakterien, insbesonders coryneformer Bakterien, zu verstehen, welche ein zwf-Allel enthalten, das für eine Glucose-6-Phosphat-Dehydrogenase kodiert, die den beschriebenen Aminosäureaustausch an Position 321 der Aminosäuresequenz und gegebenenfalls einen Aminosäureaustausch L-Serin gegen eine andere proteinogene Aminosäure, bevorzugt L-Threonin, an Position 8 enthält.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den Ausgangstamm bzw. Elternstamm bzw. den Fermentationsprozess unter Verwendung derselben verbessert.

Die erfindungsgemäßen, isolierten coryneformen Bakterien können kontinuierlich - wie beispielsweise in der PCT/EP2004/008882 beschrieben- oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Laktose, Fructose, Maltose, Melasse, Saccharose-haltige Lösungen aus der Zuckerrüben- oder Zuckerrohrherstellung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden.

Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden. Dem Kulturmedium können überdies geeignete Vorstufen der jeweiligen Aminosäure zugesetzt werden.

Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20°C bis 45°C und vorzugsweise bei 25°C
a) ein isoliertes coryneformes Bakterium in einem geeignetem Medium fermentiert; wobei das Bakterium mindestens eine Kopie ein für ein Polypeptid mit Glucose-6-Phosphat-Dehydrogenase Enzymaktivität kodierendes Gen enthält, wobei die Aminosäuresequenzen des Polypeptids ausgewählt sind aus der Gruppe
   - SEQ ID NO:2 bei der an Position 321 L-Serin enthalten ist
   - SEQ ID NO:2 bei der an Position 321 L-Serin enthalten ist und bei der die Aminosäure L-Serin an Position 8 gegen eine andere proteinogene Aminosäure ausgetauscht ist
   - SEQ ID NO:6
   - SEQ ID NO:8
   - SEQ ID NO:10
   - Aminosäuresequenz gemäß SEQ ID NO:6, SEQ ID NO:8 oder SEQ ID NO:10 einschließlich einer oder maximal 5 Insertionen oder Deletionen von Aminosäuren, wobei die enzymatische Aktivität sich um maximal 5% von der Aktivität des Polypeptids mit der Aminosäuresequenz von SEQ ID NO:6, 8 oder 10 unterscheidet
b) die L-Aminosäure in der Fermentationsbrühe oder in den Zellen des isolierten coryneformen Bakteriums anreichert.

Die auf diese Weise hergestellte Fermentationsbrühe wird anschließend zu einem festen oder flüssigen Produkt weiterverarbeitet.

Unter einer Fermentationsbrühe versteht man ein Fermentationsmedium, in dem ein Mikroorganismus für eine gewisse Zeit und bei einer gewissen Temperatur kultiviert wurde. Das Fermentationsmedium beziehungsweise die während der Fermentation eingesetzten Medium enthält/enthalten sämtliche Substanzen beziehungsweise Komponenten, die eine Vermehrung des Mikroorganismus und eine Bildung der gewünschten Aminosäure sicherstellt.

Bei Abschluss der Fermentation enthält die entstandene Fermentationsbrühe dementsprechend a) die infolge der Vermehrung der Zellen des Mikroorganismus entstandene Biomasse des Mikroorganismus, b) die im Laufe der Fermentation gebildete gewünschte Aminosäure, c) die im Laufe der Fermentation gebildeten organischen Nebenprodukte und d) die durch die Fermentation nicht verbrauchten Bestandteile des/der eingesetzten Fermentationsmediums/Fermentationsmedien beziehungsweise der Einsatzstoffe wie beispielsweise Vitamine wie Biotin, Aminosäuren wie Homoserin oder Salze wie Magnesiumsulfat.

Zu den organischen Nebenprodukte gehören Stoffe, die von den bei der Fermentation eingesetzten Mikroorganismen gegebenenfalls neben der jeweiligen erwünschten L-Aminosäure erzeugt und gegebenenfalls ausgeschieden werden. Hierzu zählen L-Aminosäuren, die im Vergleich zur erwünschten Aminosäure weniger als 30%, 20% oder 10% ausmachen. Hierzu gehören weiterhin organische Säuren, die ein bis drei Carboxyl-Gruppen tragen wie zum Beispiel Essigsäure, Milchsäure, Zitronensäure, Apfelsäure oder Fumarsäure. Schließlich gehören dazu auch Zucker wie zum Beispiel Trehalose.

Typische für industrielle Zwecke geeignete Fermentationsbrühen haben einen Aminosäuregehalt von 40 g/kg bis 180 g/kg oder 50 g/kg bis 150 g/kg. Der Gehalt an Biomasse (als getrocknete Biomasse) beträgt im Allgemeinen 20 bis 50 g/kg.

Im Falle der Aminosäure L-Lysin sind im Stand der Technik im wesentlichen vier verschiedene Produktformen bekannt.

Eine Gruppe L-Lysin haltiger Produkte umfasst konzentrierte, wässrige, alkalische Lösungen von aufgereinigtem L-Lysin (EP-B-0534865). Eine weitere Gruppe, wie beispielsweise in der US 6,340,486 und US 6,465,025 beschrieben, umfasst wässrige, saure, Biomasse-haltige Konzentrate von L-Lysin-haltigen Fermentationsbrühen. Die bekannteste Gruppe fester Produkte umfasst pulverförmige oder kristalline Formen von aufgereinigtem beziehungsweise reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenfalls die Biomasse des eingesetzten Mikroorganismus mit einem Anteil von >0% - 100%.

Entsprechend der verschiedenen Produktformen kennt man verschiedenste Verfahren, bei denen die L-Aminosäure aus der Fermentationsbrühe gesammelt, isoliert oder gereinigt wird, um das L-Aminosäure haltige Produkt oder die gereinigte L-Aminosäure herzustellen.

Zur Herstellung von festen, reinen L-Aminosäuren werden im wesentlichen Methoden der Ionenaustauschchromatographie gegebenfalls unter Verwendung von Aktivkohle und Methoden der Kristallisierung angewendet. Im Falle des Lysin erhält man auf diese Weise die entsprechende Base oder ein entsprechendes Salz wie beispielsweise das Monohydrochlorid (Lys-HCl) oder das Lysinsulfat (Lys₂-H₂SO₄).

Im Falle des Lysin ist in der EP-B-0534865 ein Verfahren zur Herstellung wässriger, basischer L-Lysin haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellt. Die mineralischen Bestandteile (anorganischen Salze) werden nach Konzentrierung und Abkühlung durch Kristallisation aus der Brühe abgetrennt und entweder als Dünger verwendet oder verworfen.

Bei Verfahren zur Herstellung von Lysin unter Verwendung der erfindungsgemäßen Bakterien werden solche Verfahren bevorzugt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse beziehungsweise die Biomasse enthaltene Fermentationsbrühe während eines geeigneten Verfahrensschrittes inaktiviert beispielsweise durch thermische Behandlung (Erhitzen) oder durch Säurezugabe.

Die chemischen Bestandteile der Biomasse sind unter anderem die Zellhülle, beispielsweise das Peptidoglykan und das Arabinogalaktan, das Protein bzw. Polypeptid, beispielsweise das Glucose-6-Phosphat-Dehydrogenase Polypeptid, Lipide und Phospholipide und Nukleinsäuren (DNA und RNA), beispielsweise Polynukleotide enthaltend die erfindungsgemäße Mutation. Als Folge der Massnahmen der Inaktivierung und/oder der weitereren Verfahrensschritte (beispielsweise Ansäuerung,Sprühtrocknung, Granulation etc.) liegen Nukleinsäuren typischerweise als Fragmente mit eine Länge von unter anderem ≥40 - 60 bp, >60 - 80 bp, >80 - 100 bp, >100 - 200 bp, >200 - 300 bp, >300 - 400 bp, >400 - 500 bp, >500 - 750 bp, >750 - 1000 bp, >1000 -1250 bp, >1250 - 1500 bp, >1500 - 1750 bp, >1750 - 2000 bp, >2000 - 2500 bp, >2500 - 3000 bp, >3000 - 4000 bp, >4000 - 5000 bp vor.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure auf einen sauren pH Wert gestellt werden (GB 1,439,728 oder EP 1 331 220). Es ist gleichfalls möglich die Fermentationsbrühe mit der vollständig enthaltenen Biomasse anzusäuern (US 6,340,486 oder US 6,465,025). Schließlich kann die Brühe auch durch Zusatz von Natriumbisulfit (NaHSO₃, GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. Gegebenenfalls bleiben diese auch vollständig (100%) oder nahezu vollständig das heißt > 95% oder > 98% im Produkt. In diesem Sinne bedeutet der Begriff "Fermentationsbrühebasis", dass ein Produkt mindestens einen Teil der Bestandteile der Fermentationsbrühe enthält.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen beziehungsweise eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt d. h. ohne vorherige Aufkonzentrierung durch Sprühtrocknung oder Sprühgranulation zu trocknen.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Korngröße von 20 bis 200 µm Durchmesser gemeint.

Mit "grobkörnig" ist ein Produkt mit einem überwiegendem Anteil (> 50 %) einer Korngröße von 200 bis 2000 µm Durchmesser gemeint.

Die Korngrößenbestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmann, Wissenschaftliche Verlagsgesellschaft Stuttgart (1996) oder im Lehrbuch "Introduction to Particle Technology" von M. Rhodes, Verlag Wiley & Sons (1998) beschrieben.

Das rieselfähige, feinteilige Pulver kann wiederum durch geeignete Kompaktier- oder Granulier-Verfahren in ein grobkörniges, gut rieselfähiges, lagerbares und weitgehend staubfreies Produkt überführt werden.

Der Begriff "staubfrei" bedeutet, daß das Produkt lediglich geringe Anteile (< 5 %) an Körngrößen unter 100 µm Durchmesser enthält.

"Lagerbar", im Sinne dieser Erfindung, bedeutet ein Produkt, das mindestens ein (1) Jahr oder länger, bevorzugt mindestens 1,5 Jahre oder länger, besonders bevorzugt zwei (2) Jahre oder länger in trockener und kühler Umgebung gelagert werden kann ohne das ein wesentlicher Verlust (< 5%) der jeweiligen Aminosäure auftritt.

Ein weiterer Gegenstand der Erfindung ist dementsprechend ein Verfahren zur Herstellung eines L-Aminosäure, bevorzugt L-Lysin oder L-Tryptophan, haltigen Produktes, bevorzugt Tierfuttermittel-Additivs, aus Fermentationsbrühen, gekennzeichnet durch die Schritte
a) Kultivierung und Fermentation eines L-Aminosäure ausscheidenden coryneformen Bakteriums, welches mindestens ein zwf-Allel enthält, das für ein Polypeptid mit Glucose-6-Phosphat-Dehydrogenase Aktivität kodiert, welches eine Aminosäuresequenz umfasst, bei der an Position 321 oder der vergleichbaren Position jede proteinogene Aminosäure ausgenommen Glycin, bevorzugt L-Serin enthalten ist, und wobei gegebenenfalls an Position 8 oder der vergleichbaren Position jede proteinogene Aminosäure ausgenommen L-Serin, bevorzugt L-Threonin, enthalten ist, in einem Fermentationsmedium,
b) Entfernung der während der Fermentation gebildeten Biomasse in einer Menge von 0 bis 100 Gew.-%, und
c) Trocknung der gemäß a) und/oder b) erhaltenen Fermentationsbrühe, um das Produkt in der gewünschten Pulver- oder Granulatform zu erhalten
wobei gegebenenfalls vor Schritt b) oder c) eine Säure ausgewählt aus der Gruppe Schwefelsäure, Phosphorsäure oder Salzsäure hinzugefügt wird.

Vorzugsweise wird im Anschluss an Schritt a) oder b) Wasser aus der L-Aminosäure haltigen Fermentationsbrühe entfernt (Aufkonzentration).

Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) Stearaten.

Weiterhin ist es vorteilhaft die Oberfläche der erhaltenen Granulate mit Ölen zu versehen so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethycellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Korngröße von 100 bis 1800 µm oder einem Anteil von ≥ 95 Gew.-% einer Korngröße von 300 bis 1800 µm Durchmesser. Der Anteil an Staub d. h. Partikeln mit einer Korngrösse < 100 µm liegt bevorzugt bei > 0 bis 1 Gew.- besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonate, Kieselsäuren, Silikate, Alginate, Stearate, Stärken, Gummis und Celluloseether, wie in der DE-C-4100920 beschrieben, in einen Zustand gebracht werden, in dem es stabil gegenüber der Verdauung durch Tiermägen insbesondere dem Magen von Wiederkäuern ist.

Zur Einstellung einer gewünschten Aminosäurekonzentration im Produkt kann je nach Anforderung die entsprechende Aminosäure während des Verfahrens in Form eines Konzentrates oder gebenenfalls einer weitgehend reinen Substanz beziehungsweise dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses hinzugefügt werden.

Im Falle des Lysin wird bei der Herstellung Lysin-haltiger Produkte das Verhältnis der Ionen so eingestellt, dass das Ionenverhältnis entsprechend nachstehender Formel

2x[SO₄²⁻]+[Cl⁻]-[NH₄⁺]-[Na⁺]-[K⁺]-2x[Mg⁺]-2x[Ca²⁺]/[L-LYs]

0,68 bis 0,95, bevorzugt 0,68 bis 0,90 ergibt so wie von Kushiki et al. in der US 20030152633 beschrieben.

Im Falle des Lysin hat das auf diese Weise hergestellte feste Produkt auf Fermentationsbrühebasis einen Lysingehalt (als Lysinbase) von 10 Gew.-% bis 70 Gew.-% oder 20 Gew.-% bis 70 Gew.-%, bevorzugt 30 Gew.-% bis 70 Gew.-% und ganz besonders bevorzugt von 40 Gew.-% bis 70 Gew.-% bezogen auf die Trockenmasse des Produkts. Maximale Gehalte an Lysinbase von 71 Gew.-% , 72 Gew.-%, 73 Gew.-% sind ebenfalls möglich.

Im Falle einer elektrisch neutralen Aminosäure wie dem L-Tryptophan hat das auf diese Weise hergestellte feste Produkt auf Fermentationsbrühebasis einen Aminosäuregehalt von mindestens 5 Gew.-%, 10 Gew.-%, 20 Gew.-%, 30 Gew.-% und maximal 50 Gew.-%, 60 Gew.-%, 70 Gew.-%, 80 Gew.-%, 90 Gew.-% oder bis 95 Gew.-%.

Der Wassergehalt des festen Produktes beträgt bis zu 5 Gew.-%, bevorzugt bis zu 4 Gew.-%, und besonders bevorzugt weniger als 3 Gew.-%.

Die Beschreibung offenbart auch ein L-Lysin haltiges Futtermitteladditiv auf Fermentationsbrühebasis, welches folgende Merkmale aufweißt
a) einen Lysingehalt (als Base) von mindestens 10 Gew.-% bis maximal 73 Gew.-%,
b) einen Wassergehalt von höchstens 5 Gew.-%, und
c) einen Biomassegehalt ensprechend mindestens 0,1 % der in der Fermentationsbrühe enthaltenen Biomasse, wobei die gegebenenfalls inaktivierte Biomasse aus erfindungsgemäßen coryneformen Bakterien gebildet wird.

Die Beschreibung offenbart auch ein L-Tryptophan haltiges Futtermitteladditiv auf Fermentationsbrühebasis, welches folgende Merkmale aufweißt
a) einen Tryptophangehalt von mindestens 5 Gew.-% bis maximal 95 Gew.-%,
b) einen Wassergehalt von höchstens 5 Gew.-%, und
c) einen Biomassegehalt ensprechend mindestens 0,1 % der in der Fermentationsbrühe enthaltenen Biomasse, wobei die gegebenenfalls inaktivierte Biomasse aus erfindungsgemäßen coryneformen Bakterien gebildet wird.

Eine Mutante von Corynebacterium glutamicum mit der Bezeichnung DM1797, die den Aminosäureaustausch lysC T311I in der Aspartatkinase enthält, wurde am 28. Oktober 2004 bei der Deutschen Sammlung für Mikroorganismen und Zellkulturen (DSMZ, Braunschweig, Deutschland) als DSM 16833 hinterlegt.

Die erfindungsgemäße Mutante Corynebacterium glutamicum DM1816, die L-Serin an Position 321 der Aminosäuresequenz des Zwf-Polypeptids enthält, wurde am 09. Februar 2005 bei der Deutschen Sammlung für Mikroorganismen und Zellkulturen (DSMZ, Braunschweig, Deutschland) als DSM 17119 hinterlegt.

Die erfindungsgemäße Mutante Corynebacterium glutamicum DM1889, die L-Serin an Position 321 der Aminosäuresequenz des Zwf-Polypeptids enthält, wurde am 16. März 2006 bei der Deutschen Sammlung für Mikroorganismen und Zellkulturen (DSMZ, Braunschweig, Deutschland) als DSM 18062 hinterlegt.

### Beispiel 1

### Mutagenese des L-Lysin produzierenden Stammes DM1797

Der Corynebacterium glutamicum Stamm DM1797 wurde als Ausgangsstamm für die Mutagenese mit N-Methyl-N'-Nitro-N-Nitrosoguanidin (MNNG) eingesetzt. Der Stamm DM1797 ist eine Aminoethylcystein-resistente Mutante von Corynebacterium glutamicum ATCC13032 und unter der Bezeichnung DSM16833 bei der Deutschen Sammlung für Mikroorganismen und Zellkulturen (DSMZ, Braunschweig, Deutschland) hinterlegt.

Der Stamm DM1797 wurde in 10 ml LB-Bouillon (Merck, Darmstadt, Germany), die in einem 100 ml Erlenmeyerkolben enthalten waren, für 24 Stunden bei 33°C und 200 rpm auf einem Rotations-Schüttler vom Typ Certomat BS-1 (B. Braun Biotech International, Melsungen, Deutschland) kultiviert. Anschließend wurde die Kultur abzentrifugiert, das Sediment in 10 ml 0,9% NaCl-Lösung resuspendiert, die erhaltene Suspension erneut abzentrifugiert und das erhaltene Sediment in 10 ml 0,9% NaCl-Lösung aufgenommen. 5 ml dieser Zellsuspension wurden mit 400µg/ml MNNG für 15 Minuten bei 30°C und 200 rpm auf einem Schüttler (siehe oben) behandelt. Anschließend wurde der Mutageneseansatz abzentrifugiert und das Sediment in 10 ml 2% Na-Thiosulfat in 0,9% NaCl-Puffer (pH = 6,0) aufgenommen. Die Zellsuspension wurde anschließend im Verhältnis 1:1000, 1:10000 und 1:100000 mit 0,9% NaCl-Lösung verdünnt, und Aliquots auf Hirn-Herz-Agar (Merck, Darmstadt, Deutschland) ausplattiert. Auf diese Weise wurden ungefähr 2500 Mutanten isoliert.

### Beispiel 2

### Leistungstest der Mutanten des Stammes DM1797

Die in Beispiel 1 erhaltenen Mutanten wurden in einem zur Produktion von Lysin geeigneten Nährmedium kultiviert und der Lysingehalt im Kulturüberstand bestimmt.

Dazu wurden die Klone zunächst auf Hirn-Herz-Agarplatten (Merck, Darmstadt, Deutschland) für 24 Stunden bei 33°C vermehrt. Ausgehend von diesen Agarplattenkulturen wurde je eine Vorkultur angeimpft (10 ml Medium im 100 ml Erlenmeyerkolben). Als Medium für die Vorkultur wurde das Medium MM verwendet. Die Vorkultur wurde 24 Stunden bei 33°C und 240 rpm auf einem Schüttler inkubiert. Von dieser Vorkultur wurde eine Hauptkultur angeimpft, so dass die Anfangs-OD (660 nm) der Hauptkultur 0,1 OD betrug. Für die Hauptkultur wurde ebenfalls das Medium MM verwendet,

| | |
|---|---|
| Medium MM | |
| CSL | 5 g/l |
| MOPS | 20 g/l |
| Glucose (getrennt autoklaviert) | 50 g/l |
| Salze: | |
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| CaCO₃ | 25 g/l |

CSL (Corn Steep Liquor), MOPS (Morpholinopropansulfonsäure) und die Salzlösung wurden mit Ammoniakwasser auf pH 7 eingestellt und autoklaviert. Anschließend wurden die sterilen Substrat- und Vitaminlösungen sowie das trocken autoklavierte CaCO₃ zugesetzt.

Die Kultivierung erfolgte in Volumina von 10 ml, die in 100 ml Erlenmeyerkolben mit Schikanen enthalten waren. Die Temperatur betrug bei 33°C, die Umdrehungszahl war 250 rpm und die Luftfeuchte 80%.

Nach 24 Stunden wurde die optische Dichte (OD) bei einer Meßwellenlänge von 660 nm mit dem Biomek 1000 (Beckmann Instruments GmbH, München) bestimmt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt. Eine Mutante, die sich durch eine erhöhte Lysinbildung auszeichnete, wurde als DM1816 bezeichnet.

**Tabelle 1**

| Stamm | OD (660) | Lysin-HCl (g/l) |
|---|---|---|
| DM1797 | 11,7 | 3,6 |
| DM1816 | 11,8 | 3,9 |

### Beispiel 3

### Sequenzierung des zwf-Gens der Mutante DM1816

Aus dem Klon DM1816 wurde mit der Methode von Eikmanns et al. (Microbiology 140: 1817 - 1828 (1994)) chromosomale DNA isoliert. Mit Hilfe der Polymerase-Kettenreaktion wurde ein DNA-Abschnitt amplifiziert, welcher das zwf-Gen trägt. Hierfür wurden folgende Oligonukleotide als Primer verwendet:
zwf-L1 (SEQ ID NO: 19):
   5' agaagctgac gctgtgttct 3'
zwf-L2 (SEQ ID NO: 20):
   5' cattggtgga ctcggtaact 3'

Die dargestellten Primer wurden von der Firma MWG Biotech (Ebersberg, Deutschland) synthetisiert. Sie ermöglichen die Amplifizierung eines ca. 1,95 kb langen DNA-Abschnittes, welcher das zwf-Gen trägt. Der Primer zwf-L1 bindet an die Region entsprechend Position 59 bis 78 des zu SEQ ID NO: 3 komplementären Stranges. Der Primer zwf-L2 bindet an die Region ensprechend Position 2026 bis 2007 des Stranges gemäß SEQ ID NO: 3.

Die PCR-Reaktion wurde mit der Phusion High Fidelity DNA Polymerase (New England Biolabs, Frankfurt, Deutschland) durchgeführt. Der Reaktionsansatz wurde nach Herstellerangaben angesetzt und enthielt bei 50 µl GesamtVolumen 10 µl des mitgelieferten 5 x Phusion HF Buffer, Desoxynucleosidtriphosphate in einer Konzentration von jeweils 200 µM, Primer in einer Konzentration von 0,5 µM, ungefähr 50 ng Template-DNA und 2 Units Phusion Polymerase. Durch Zugabe von H₂O wurde das Volumen auf 50 µl eingestellt.

Der PCR-Ansatz wurde zuerst einer einleitenden Denaturierung bei 98°C für 30 Sekunden unterzogen. Darauf folgten sich 35x wiederholend ein Denaturierungsschritt bei 98°C für 20 Sekunden, ein Schritt zum Binden der Primer an die vorgelegte DNA bei 60°C für 20 Sekunden und der Extensionsschritt zur Verlängerung der Primer bei 72°C für 60 Sekunden. Nach dem abschliessenden Extensionsschritt für 5 Minuten bei 72°C wurde der PCR-Ansatz einer Agarosegel-Elektrophorese (0,8% Agarose) unterworfen. Ein DNA-Fragment von ca. 1,85 kb Länge wurde identifiziert, aus dem Gel isoliert und unter Verwendung des QIAquick Gel Extraction Kit der Firma Qiagen, (Hilden, Deutschland) aufgereinigt.

Die Nukleotidsequenz des amplifizierten DNA-Fragmentes bzw. PCR-Produktes wurde von der Firma Agowa (Berlin, Deutschland) bestimmt. Die erhaltene Sequenz der Kodierregion des zwf-Allels ist in der SEQ ID NO: 9 dargestellt. Die sich mit Hilfe des Programmes Patentin ergebende Aminosäuresequenz des Proteins ist in der SEQ ID NO: 10 dargestellt.

Die Nukleotidsequenz der Kodierregion des zwf-Allels der Mutante DM1816 enthält an Position 961 die Nukleobase Adenin (Siehe SEQ ID NO: 5 oder 9). Das Gen des Wildtyps (Siehe SEQ ID NO: 1) enthält an dieser Position die Nukleobase Guanin. Diese Guanin-Adenin Transition führt zu einem Aminosäureaustausch von Glycin zu Serin an Position 321 der resultierenden Aminosäuresequenz. Diese Mutation wird im Folgenden als zwfG321S bezeichnet. Weiterhin enthält das zwf-Allel von DM1816 noch den Nukleotidaustausch Thymin gegen Adenin an Position 22 der Nukleotidsequenz. Diese Thymin-Adenin Transversion führt zu einem Aminosäureaustausch von Serin zu Threonin an Position 8 der resultierenden Aminosäuresequenz.

Ausserdem enthält das zwf-Allel von DM1816 noch fünf weitere Nukleotidaustausche, die nicht zu einem Aminosäureaustausch führen ("stille Mutationen"): eine Cytosin-Thymin- Transition an Position 138, eine Cytosin-Thymin- Transition an Position 279, eine Thymin-Cytosin - Transition an Position 738, eine Cytosin-Thymin- Transition an Position 777 und eine Guanin-Adenin- Transition an Position 906.

### Beispiel 4

### Konstruktion des Austauschvektors pK18mobsacB_zwfG321S

Mit Hilfe der Polymerase-Kettenreaktion wurde ein Teil der Kodierregion, das heisst ein sogenanntes internes Fragment bzw. interner Bereich, des zwf-Allels amplifiziert, der die Mutation zwfG321S trägt. Als Template wurde die in Beispiel 3 gewonnene chromosomale DNA verwendet. Folgende Oligonukleotide wurden als Primer für die PCR ausgewählt:
zwf-int1-bam (SEQ ID NO: 23):
   5' ctag-ggatcc-acgtacgcgatgccgcaagt 3'
zwf-int2-bam (SEQ ID NO: 24):
   5' ctag-ggatcc-tcaggctgcacgcgaatcac 3'

Sie wurden von der Firma MWG Biotech (Ebersberg, Deutschland) synthetisiert und ermöglichen die Amplifizierung eines ca. 1 kb langen DNA-Abschnittes der Kodierregion. Die Nukleotide 11 bis 30 des Primers zwf-int1-bam binden an die Region entsprechend Position 546 bis 565 des zu SEQ ID NO:3 koplementären Stranges. Die Positionen 546 und 565 von SEQ ID NO:3 entsprechen den Positionen 239 und 258 in SEQ ID NO:1. Die Nukleotide 11 bis 30 des Primers zwf-int2-bam binden an die Region entsprechend Position 1527 bis 1508 des Stranges gemäß SEQ ID No. 3. Die Position 1527 und 1508 von SEQ ID NO:3 ensprechen den Positionen 1220 und 1201 von SEQ ID NO:1. Außerdem enthalten die Primer die Sequenzen für Schnittstellen der Restriktionsendonuklease BamHI, die in der oben dargestellten Nukleotidabfolge durch Unterstreichen markiert sind.

Die PCR-Reaktion wurde mit der Phusion High-Fidelity DNA Polymerase (New England Biolabs, Frankfurt, Deutschland) durchgeführt. Der Reaktionsansatz hatte die oben beschriebene Zusammensetzung. Die PCR wurde mit einer Ausnahme wie oben durchgeführt: der 72°C-Extensionsschritt in der 35-fachen Wiederholung wurde jeweils nur für 30 Sekunden durchgeführt.

Das ca. 1 kb lange Amplifikat wurde mit der Restriktionsendonuklease BamHI behandelt und durch Elektrophorese in einem 0,8%igen Agarosegel identifiziert. Es wurde anschließend aus dem Gel isoliert und mit dem QIAquick Gel Extraction Kit der Firma Qiagen aufgereinigt.

Das dergestalt gereinigte DNA-Fragment enthält die beschrieben zwfG321S-Mutation und besitzt BamHI kompatible Enden (zwfG321S-Fragment bzw. 'zwf' in Figur 1). Es wurde anschließend in den von Schäfer et al. (Gene, 145, 69-73 (1994) beschriebenen, mobilisierbaren Vektor pK18mobsacB eingebaut, um einen Allel- beziehungsweise Mutationsaustausch zu ermöglichen. Hierzu wurde pK18mobsacB mit dem Restriktionsenzym BamHI verdaut und die Enden mit alkalischer Phosphatase (Alkaline Phosphatase, Boehringer Mannheim, Deutschland) dephosphoryliert. Der so vorbereitete Vektor wurde mit dem zwfG321S-Fragment gemischt und der Ansatz mit dem Ready-To-Go T4 DNA Ligase Kit (Amersham-Pharmacia, Freiburg, Deutschland) behandelt. Anschließend wurde der E.coli Stamm S17-1 (Simon et al.,Bio/Technologie 1: 784-791, 1993) mit dem Ligationsansatz transformiert (Hanahan, In. DNA cloning. A practical approach. Vol.1. ILR-Press, Cold Spring Habor, New York, 1989). Die Selektion auf Plasmid-tragende Zellen erfolgte durch Ausplattieren des Tansformationsansatzes auf LB-Agar (Sambrock et al., Molecular Cloning: a laboratory manual. 2nd Ed. Cold Spring Habor, New York, 1989), der mit 25 mg/l Kanamycin supplementiert worden war.

Plasmid-DNA wurde aus einer Transformante mit Hilfe des QIAprep Spin Miniprep Kit der Firma Qiagen isoliert und durch Restriktionsspaltung jeweils einmal mit dem Enzym BamHI und einmal mit dem Enzym SacI und anschließender Agarosegel-Elektrophorese überprüft. Das Plasmid wurde pK18mobsacB_zwfG321S genannt und ist in Figur 1 dargestellt.

### Beispiel 5

### Einbau der Mutation zwfG321S in den Stamm DM1797

Der in Beispiel 4 beschriebene Vektor pK18mobsacB_zwfG321S wurde nach dem Protokoll von Schäfer et al. (Journal of Microbiology 172: 1663-1666 (1990)) in den C. glutamicum Stamm DM1797 durch Konjugation transferiert. Der Vektor kann in DM1797 nicht selbständig replizieren und bleibt nur dann in der Zelle erhalten, wenn er als Folge eines Rekombinationsereignisses im Chromosom integriert vorliegt. Die Selektion von Transkonjuganten, d. h. von Klonen mit integriertem pK18mobsacB_zwfG321S erfolgte durch Ausplattieren des Konjugationsansatzes auf LB-Agar, der mit 25 mg/l Kanamycin und 50 mg/l Nalidixinsäure supplementiert worden war. Kanamycin-resistente Transkonjuganten wurden anschließend auf mit Kanamycin (25 mg/l) supplementierten LB-Agarplatten ausgestrichen und für 24 Stunden bei 33°C inkubiert. Zur Selektion von Mutanten, bei denen als Folge eines zweiten Rekombinationsereignisses die Exzision des Plasmides stattgefunden hatte, wurden die Klone 30 Stunden unselektiv in LB-Flüssigmedium kultiviert, anschließend auf LB-Agar, der mit 10% Sucrose supplementiert worden war ausgestrichen und 24 Stunden bei 33°C bebrütet.

Das Plasmid pK18mobsacB_zwfG321S enthält ebenso wie das Ausgangsplasmid pK18mobsacB_neben dem Kanamycin-Resistenzgen eine Kopie des für die Levan-Sucrase aus Bacillus subtilis kodierenden sacB-Gens. Die durch Sucrose induzierbare Expression des sacB-Gens führt zur Bildung der Levan-Sucrase, die die Synthese des für C. glutamicum toxischen Produktes Levan katalysiert. Auf mit Sucrose supplementiertem LB-Agar wachsen daher nur solche Klone, bei denen das integrierte pK18mobsacB_zwfG321S als Folge eines zweiten Rekombinationsereignisses exzisiert hat. In Abhängigkeit von der Lage des zweiten Rekombinationsereignisses in bezug auf den Mutationsort findet bei der Exzision der Allelaustausch bzw. der Einbau der Mutation statt oder es verbleibt die ursprüngliche Kopie im Chromosom des Wirtes.

Anschließend wurde ein Klon gesucht, bei dem der gewünschte Austausch, d. h. der Einbau der Mutation zwfG321S, erfolgt war. Hierzu wurde von 10 Klonen mit dem Phänotyp "Wachstum in Gegenwart von Sucrose" und "Nicht-Wachstum in Gegenwart von Kanamycin" die Sequenz des zwf-Gens bestimmt. Auf diese Weise wurde ein Klon identifiziert, der die Mutation zwfG321S trägt. Dieser Stamm wurde als C. glutamicum DM1797_zwfG321S bezeichnet.

### Beispiel 6

### Vergleich der Leistung des Stammes DM1797_zwfG321S mit der des Ausgangsstammes DM1797

Der Leistungtest wurde wie in Beispiel 2 beschrieben durchgeführt. Der Stamm DM1797_zwfG321S zeigte im Vergleich zu DM1797 deutlich erhöhte Lysinausscheidung ähnlich wie DM1816 (Siehe Tabelle 1).

## Patentansprüche

1. Isolierte Mutanten coryneformer Bakterien, welche ein Gen enthalten, das für ein Polypeptid mit Glucose-6-Phosphat-Dehydrogenase Aktivität kodiert, **dadurch gekennzeichnet, dass** das Polypeptid eine Aminosäuresequenz gemäß SEQ ID NO : 2 umfasst, bei der an Position 321 L-Serin enthalten ist.

2. Mutanten coryneformer Bakterien gemäß Anspruch 1,
**dadurch gekennzeichnet, dass** es sich bei den coryneformen Bakterien um ein Bakterium ausgewählt aus der Gruppe Corynebacterium efficiens, Corynebacterium glutamicum, Corynebacterium thermoaminogenes und Corynebacterium aminogenes handelt.

3. Mutanten coryneformer Bakterien gemäß Anspruch 2,
**dadurch gekennzeichnet, dass** es sich um Corynebacterium glutamicum handelt.

4. Mutanten coryneformer Bakterien gemäß Anspruch 1, 2, oder 3, **dadurch gekennzeichnet, dass** es sich um L-Aminosäure ausscheidende Bakterien handelt.

5. Mutanten coryneformer Bakterien gemäß Anspruch 4,
**dadurch gekennzeichnet, dass** es sich um L-Lysin oder um L-Tryptophan ausscheidende Bakterien handelt.

6. Mutanten coryneformer Bakterien gemäß den Ansprüchen 1-5, **dadurch gekennzeichnet, dass** die Aminosäure L-Serin an Position 8 gegen eine andere proteinogene Aminosäure ausgetauscht ist.

7. Mutanten coryneformer Bakterien gemäß den Ansprüchen 1 bis 6, **dadurch gekennzeichnet, dass** das kodierte Protein eine Aminosäuresequenz ausgewählt aus der Gruppe
a) Aminosäuresequenz gemäß SEQ ID NO:6, SEQ ID NO:8 oder SEQ ID NO:10,
b) Aminosäuresequenz gemäß SEQ ID NO:6, SEQ ID NO:8 oder SEQ ID NO:10 einschließlich einer oder maximal 5 Insertionen oder Deletionen von Aminosäuren, wobei die enzymatische Aktivität sich um maximal 5% von der Aktivität des Polypeptids mit der Aminosäuresequenz von SEQ ID NO:6, 8 oder 10 unterscheidet und wobei an der der position 321 entsprechenden position L-Serin enthalten ist,
umfasst.

8. Mutanten coryneformer Bakterien gemäß Anspruch 7, **dadurch gekennzeichnet, dass** das Gen die Nukleotidsequenz von SEQ ID NO:5, SEQ ID NO:7 oder SEQ ID NO:9 umfasst.

9. Mutanten coryneformer Bakterien, **dadurch gekennzeichnet, dass** diese durch folgende Schritte erhältlich sind:
a) Behandlung eines coryneformen Bakteriums, das die Fähigkeit besitzt Aminosäuren auszuscheiden, mit einem mutagenen Agenz,
b) Isolierung und Vermehrung der in a) erzeugten Mutante,
c) Bereitstellung von Nukleinsäure aus der in b) erhaltenen Mutante,
d) Herstellung eines Nukleinsäuremoleküls unter Verwendung der Polymerasekettenreaktion, der Nukleinsäure aus c), und eines Primerpaares bestehend aus einem ersten Primer umfassend mindestens 15 aufeinanderfolgenden Nukleotide ausgewählt aus der Nukleotidsequenz zwischen Position 1 und 1267 von SEQ ID NO:3 oder SEQ ID NO:11 und einem zweitem Primer umfassend mindestens 15 aufeinanderfolgenden Nukleotide ausgewählt aus der komplementären
Nukleotidsequenz zwischen Position 2100 und 1271 von SEQ ID NO:3 oder 11,
e) Bestimmung der Nukleotidsequenz des in d) erhaltenen Nukleinsäuremoleküls, und Bestimmung der kodierten Aminosäuresequenz,
f) gegebenfalls Vergleich der in f) bestimmten Aminosäuresesequenz mit SEQ ID NO:6, 8 oder 10, und
g) Identifizierung einer Mutante, die ein Polynukleotid enthält, das für ein Polypeptid mit glucose - 6 - phosphat dehydrogenase activität kodiert, welches an der Position entsprechend position 321 von SEQ ID NO:2 L-Serin enthält.

10. Ein isoliertes Polynukleotid, das für ein Polypeptid gemäß SEQ ID NO:2 mit Glucose-6-Phosphat-Dehydrogenase Enzymaktivität kodiert, welches an Position 321 der Aminosäuresequenz SEQ ID NO:2 L-Serin enthält.

11. Isoliertes Polynukleotid gemäß Anspruch 10, **dadurch gekennzeichnet, dass** das kodierte Protein eine Aminosäuresequenz ausgewählt aus der Gruppe
a) Aminosäuresequenz gemäß SEQ ID NO:6, SEQ ID NO:8 oder SEQ ID NO:10,
b) Aminosäuresequenz gemäß SEQ ID NO:6, SEQ ID NO:8 oder SEQ ID NO:10 einschließlich einer oder maximal 5 Insertionen von Aminosäuren, wobei die enzymatische Aktivität sich um maximal 5% von der Aktivität des Polypedtids mit der Aminosäuresequenz von SEQ ID NO:6, 8 oder 10 unterscheidet und wobei an der der position 321 entsprechenden position L-Serin
enthalten ist.

12. Isoliertes Polynukleotid gemäß einem oder mehreren der Ansprüche 10 oder 11 **dadurch gekennzeichnet, dass** das kodierte Polypeptid die Aminosäuresequenz von SEQ ID NO:6, SEQ ID NO:8 oder SEQ ID NO:10 umfasst.

13. Isoliertes Polynukleotid gemäß Anspruch 12, **dadurch gekennzeichnet, dass** die Nukleotidsequenz SEQ ID NO:5, SEQ ID NO:7 oder SEQ ID NO:9 umfasst.

14. Isoliertes Polynukleotid gemäß Anspruch 13, **dadurch gekennzeichnet, dass** die Nukleotidsequenz SEQ ID NO:11 umfasst.

15. Ein isoliertes Polynukleotidfragment, welches ein Nukleinsäure-Molekül umfasst, das mindestens für ein Leseraster mit einer Aminosäuresequenz entsprechend Position 307 bis 335 von SEQ ID NO:2 kodiert, wobei an der Position 321 von SEQ ID NO:2 L-Serin enthalten ist.

16. Isoliertes Polynukleotid gemäß Anspruch 15, **dadurch gekennzeichnet, dass** es mindestens für ein Leseraster mit einer Aminosäuresequenz entsprechend Position 307 bis 335 von SEQ ID NO:6, SEQ ID NO:8 oder SEQ ID NO:10 kodiert.

17. Isoliertes Polynukleotid gemäß Anspruch 15, **dadurch gekennzeichnet, dass** die Aminosäuresequenz eine oder mehrere konservative Aminosäureaustausche enthält, wobei die konservativen Aminosäureaustausche nicht die Position 321 betreffen.

18. Isoliertes Polynukleotid gemäß Anspruch 15, **dadurch gekennzeichnet, dass** es eine oder mehrere stille Mutationen enthält.

19. Isoliertes Polynukleotid gemäß Anspruch 16, **dadurch gekennzeichnet, dass** es mindestens die Nukleotidsequenz entsprechend Position 919 bis 1005 von SEQ ID NO:5, SEQ ID NO:7 oder SEQ ID NO:9 umfasst.

20. Verfahren zur Herstellung eines rekombinanten coryneformen Bakteriums, **dadurch gekennzeichnet, dass** man
a) ein isoliertes Polynukleotid gemäß den Ansprüchen 10 bis 14 oder gemäß den Ansprüchen 14 bis 19 in ein coryneformes Bakterium überführt,
b) das im Chromosom des coryneformen Bakteriums vorhandene Glucose-6-Phosphat-Dehydrogenase Gen, das für eine Aminosäuresequenz mit Glycin an Position 321 und gegebenenfalls L-Serin an Position 8 oder einer vergleichbaren Position der Aminosäuresequenz SEQ ID NO:2 kodiert, gegen das Polynukleotid aus a) austauscht, und
c) das gemäß Schritt a) und b) erhaltene coryneforme Bakterium vermehrt.

21. Verfahren zur Herstellung eines rekombinanten Mikroorganismus, **dadurch gekennzeichnet, dass** man
a) ein isoliertes Polynukleotid gemäß Anspruch 10 bis 14, in einen Mikroorganismus überführt,
b) das isolierte Polynukleotid in dem Mikroorganismus repliziert, und
c) den gemäß Schritt a) und b) erhaltenen Mikroorganismus vermehrt.

22. Ein rekombinanter Mikroorganismus, der das isolierte Polynukleotid gemäß den Ansprüchen 10 bis 14 oder 15 bis 19 enthält.

23. Rekombinanter Mikroorganismus gemäß Anspruch 22,
**dadurch gekennzeichnet, dass** es sich um ein coryneformes Bakterium oder um ein Bakterium der Gattung Escherichia handelt.

24. Rekombinanter Mikroorganismus gemäß Anspruch 23, **dadurch gekennzeichnet, dass** es sich bei dem coryneformen Bakterium um die Gattung Corynebacterium handelt.

25. Rekombinanter Mikroorganismus gemäß Anspruch 24, **dadurch gekennzeichnet, dass** es sich bei dem Bakterium der Gattung Corynebacterium um die Art Corynebacterium glutamicum handelt.

26. Ein Vektor, der das isolierte Polynukleotid gemäß Ansprüchen 10 bis 14 oder 15 bis 19 enthält.

27. Ein rekombinanter Mikroorganismus, der den Vektor gemäß Anspruch 26 enthält.

28. Rekombinanter Mikroorganismus gemäß Anspruch 27, **dadurch gekennzeichnet, dass** es sich um ein coryneformes Bakterium oder um ein Bakterium der Gattung Escherichia handelt.

29. Rekombinanter Mikroorganismus gemäß Anspruch 28, **dadurch gekennzeichnet, dass** es sich bei dem coryneformen Bakterium um die Gattung Corynebacterium handelt.

30. Rekombinanter Mikroorganismus gemäß Anspruch 29, **dadurch gekennzeichnet, dass** es sich bei dem Bakterium der Gattung Corynebacterium um die Art Corynebacterium glutamicum handelt.

31. Verfahren zur Herstellung einer L-Aminosäure **dadurch gekennzeichnet, dass** man
a) ein isoliertes coryneformes Bakterium in einem geeignetem Medium fermentiert, wobei das Bakterium mindestens eine Kopie ein für ein Polypeptid mit Glucose-6-Phosphat-Dehydrogenase Enzymaktivität kodierendes Gen enthält, wobei die Aminosäuresequenzen des Polypeptids in den Ansprüchen 1, 6 und 7 definiert sind,
b) die L-Aminosäure in der Fermentationsbrühe oder in den Zellen des Bakteriums anreichert.

32. Verfahren gemäß Anspruch 31, **dadurch gekennzeichnet, dass** es sich bei dem isolierten coryneformen Bakterium um eine Mutante gemäß Anspruch 1 bis 9 handelt.

33. Verfahren gemäß Anspruch 31, **dadurch gekennzeichnet, dass** es sich bei dem isolierten coryneformen Bakterium um ein rekombinantes coryneformes Bakterium handelt, das ein isoliertes Polynukleotid gemäß Anspruch 10 bis 21 enthält oder unter Verwendung desselben hergestellt wurde.

34. Verfahren nach Anspruch 31, **dadurch gekennzeichnet, dass** man die L-Aminosäure isoliert oder sammelt

35. Verfahren nach Anspruch 34, **dadurch gekennzeichnet, dass** man die L-Aminosäure reinigt.

36. Verfahren nach Anspruch 31, **dadurch gekennzeichnet, dass** man die L-Aminosäure gemeinsam mit Bestandteilen aus der Fermentationsbrühe und/oder der Biomasse (> 0 bis 100 %) isoliert oder sammelt.

37. Verfahren nach Anspruch 31, **dadurch gekennzeichnet, dass** man
a) aus der in Schritt b) von Anspruch 34 erhaltenen Fermentationsbrühe die gebildete Biomasse in einer Menge von 0 bis 100 % entfernt, und
b) aus der in Schritt a) erhaltenen Brühe ein im wesentlichen trockenes und geformtes Produkt durch eine Methode ausgewählt aus der Gruppe Granulation, Kompaktierung, Sprühtrocknung und Extrusion herstellt.

38. Verfahren nach Anspruch 37, **dadurch gekennzeichnet, dass** man einer L-Lysin-haltigen Fermentationsbrühe vor oder nach Schritt a) eine Säure ausgewählt aus der Gruppe Schwefelsäure, Salzsäure und Phosphorsäure hinzufügt.

39. Verfahren nach Anspruch 37, **dadurch gekennzeichnet, dass** man aus der vor oder nach in Schritt a) erhaltenen Brühe Wasser entfernt.

40. Verfahren gemäß Anspruch 37, **dadurch gekennzeichnet, dass** man das in oder während Schritt b) erhaltene geformte Produkt mit einem Öl besprüht.

## Claims

1. Isolated mutants of coryneform bacteria, which comprise a gene coding for a polypeptide having glucose 6-phosphate dehydrogenase activity, **characterized in that** said polypeptide comprises an amino acid sequence according to SEQ ID NO:2 in which L-serine is present in position 321.

2. Mutants of coryneform bacteria according to Claim 1, **characterized in that** the coryneform bacteria comprise a bacterium selected from the group consisting of Corynebacterium efficiens, Corynebacterium glutamicum, Corynebacterium thermoaminogenes and Corynebacterium aminogenes.

3. Mutants of coryneform bacteria according to Claim 2, **characterized in that** they are Corynebacterium glutamicum.

4. Mutants of coryneform bacteria according to Claim 1, 2, or 3, **characterized in that** they are L-amino acid-secreting bacteria.

5. Mutants of coryneform bacteria according to Claim 4, **characterized in that** they are L-lysine- or L-tryptophan-secreting bacteria.

6. Mutants of coryneform bacteria according to Claims 1-5, **characterized in that** the amino acid L-serine in position 8 has been replaced with a different proteinogenic amino acid.

7. Mutants of coryneform bacteria according to Claims 1 to 6, **characterized in that** the encoded protein comprises an amino acid sequence selected from the group consisting of
a) an amino acid sequence according to SEQ ID NO:6, SEQ ID NO:8 or SEQ ID NO:10, and
b) an amino acid sequence according to SEQ ID NO:6, SEQ ID NO:8 or SEQ ID NO:10, including one or at most 5 amino acid insertions or deletions, with the enzymic activity differing by no more than 5% from the activity of the polypeptide having the amino acid sequence of SEQ ID NO:6, 8 or 10, and with L-serine being present in the position corresponding to position 321.

8. Mutants of coryneform bacteria according to Claim 7, **characterized in that** the gene comprises the nucleotide sequence of SEQ ID NO:5, SEQ ID NO:7 or SEQ ID NO:9.

9. Mutants of coryneform bacteria, **characterized in that** they are obtainable by the following steps:
a) treating a coryneform bacterium capable of secreting amino acids with a mutagenic agent,
b) isolating and propagating the mutant generated in a),
c) providing nucleic acid of the mutant obtained in b),
d) preparing a nucleic acid molecule, using the polymerase chain reaction, of the nucleic acid from c) and of a primer pair consisting of a first primer comprising at least 15 contiguous nucleotides selected from the nucleotide sequence between positions 1 and 1267 of SEQ ID NO:3 or SEQ ID NO:11 and a second primer comprising at least 15 contiguous nucleotides selected from the complementary nucleotide sequence between positions 2100 and 1271 of SEQ ID NO:3 or 11,
e) determining the nucleotide sequence of the nucleic acid molecule obtained in d) and determining the encoded amino acid sequence,
f) comparing, where appropriate, the amino acid sequence determined in e) with SEQ ID NO:6, 8 or 10, and
g) identifying a mutant comprising a polynucleotide which encodes a polypeptide having glucose 6-phosphate dehydrogenase activity and comprising L-serine in the position corresponding to position 321 of SEQ ID NO:2.

10. An isolated polynucleotide encoding a polypeptide according to SEQ ID NO:2 having glucose 6-phosphate dehydrogenase enzyme activity, which polypeptide comprises L-serine in position 321 of the amino acid sequence SEQ ID NO:2.

11. Isolated polynucleotide according to Claim 10, **characterized in that** the encoded protein comprises an amino acid sequence selected from the group consisting of
a) an amino acid sequence according to SEQ ID NO:6, SEQ ID NO:8 or SEQ ID NO:10,
b) an amino acid sequence according to SEQ ID NO:6, SEQ ID NO:8 or SEQ ID NO:10, including one or at most 5 amino acid insertions with the enzymic activity differing by no more than 5% from the activity of the polypeptide having the amino acid sequence of SEQ ID NO:6, 8 or 10, and with L-serine being present in the position corresponding to position 321.

12. Isolated polynucleotide according to one or both of Claims 10 and 11, **characterized in that** the encoded polypeptide comprises the amino acid sequence of SEQ ID NO:6, SEQ ID NO:8 or SEQ ID NO:10.

13. Isolated polynucleotide according to Claim 12, **characterized in that** the nucleotide sequence comprises SEQ ID NO:5, SEQ ID NO:7 or SEQ ID NO:9.

14. Isolated polynucleotide according to Claim 13, **characterized in that** the nucleotide sequence comprises SEQ ID NO:11.

15. An isolated polynucleotide fragment comprising a nucleic acid molecule which encodes at least one reading frame having an amino acid sequence corresponding to positions 307 to 335 of SEQ ID NO:2, with L-serine being present in position 321 of SEQ ID NO:2.

16. Isolated polynucleotide according to Claim 15, **characterized in that** it encodes at least one reading frame having an amino acid sequence corresponding to positions 307 to 335 of SEQ ID NO:6, SEQ ID NO:8 or SEQ ID NO:10.

17. Isolated polynucleotide according to Claim 15, **characterized in that** the amino acid sequence comprises one or more conservative amino acid substitutions which do not affect position 321.

18. Isolated polynucleotide according to Claim 15, **characterized in that** it comprises one or more silent mutations.

19. Isolated polynucleotide according to Claim 16, **characterized in that** it comprises at least the nucleotide sequence corresponding to positions 919 to 1005 of SEQ ID NO:5, SEQ ID NO:7 or SEQ ID NO:9.

20. Process for preparing a recombinant coryneform bacterium, **characterized in that**
a) an isolated polynucleotide according to Claims 10 to 14 or according to Claims 14 to 19 is transferred to a coryneform bacterium,
b) the glucose 6-phosphate dehydrogenase gene which is present in the chromosome of said coryneform bacterium and which encodes an amino acid sequence glycine in position 321 and, where appropriate, L-serine in position 8 or a comparable position of said amino acid sequence SEQ ID NO:2 is replaced with the polynucleotide of a), and
c) the coryneforme bacterium obtained by steps a) and b) is propagated.

21. Process for preparing a recombinant microorganism, **characterized in that**
a) an isolated polynucleotide according to Claims 10 to 14 is transferred to a microorganism,
b) said isolated polynucleotide is replicated in said microorganism, and
c) the microorganism obtained by steps a) and b) is propagated.

22. A recombinant microorganism comprising the isolated polynucleotide according to Claims 10 to 14 or 15 to 19.

23. Recombinant microorganism according to Claim 22, **characterized in that** it is a coryneform bacterium or a bacterium of the genus Escherichia.

24. Recombinant microorganism according to Claim 23, **characterized in that** the coryneform bacterium is the genus Corynebacterium.

25. Recombinant microorganism according to Claim 24, **characterized in that** the bacterium of the genus Corynebacterium is the species Corynebacterium glutamicum.

26. A vector comprising the isolated polynucleotide according to Claims 10 to 14 or 15 to 19.

27. A recombinant microorganism comprising the vector according to Claim 26.

28. Recombinant microorganism according to Claim 27, **characterized in that** it is a coryneform bacterium or a bacterium of the genus Escherichia.

29. Recombinant microorganism according to Claim 28, **characterized in that** the coryneform bacterium is the genus Corynebacterium.

30. Recombinant microorganism according to Claim 29, **characterized in that** the bacterium of the genus Corynebacterium is the species Corynebacterium glutamicum.

31. Process for preparing an L-amino acid, **characterized in that**
a) an isolated coryneform bacterium is fermented in a suitable medium, said bacterium harboring at least one copy of a gene encoding a polypeptide having glucose 6-phosphate dehydrogenase enzyme activity, with the amino acid sequences of said polypeptide being defined in Claims 1, 6 and 7, and
b) the L-amino acid is concentrated in the fermentation broth or in the cells of said bacterium.

32. Process according to Claim 31, **characterized in that** the isolated coryneform bacterium is a mutant according to Claims 1 to 9.

33. Process according to Claim 31, **characterized in that** the isolated coryneform bacterium is a recombinant coryneform bacterium which harbors an isolated polynucleotide according to Claims 10 to 21 or which has been prepared using the latter.

34. Process according to Claim 31, **characterized in that** the L-amino acid is isolated or collected.

35. Process according to Claim 34, **characterized in that** the L-amino acid is purified.

36. Process according to Claim 31, **characterized in that** the L-amino acid is isolated or collected together with components of the fermentation broth and/or the biomass (> 0 to 100%).

37. Process according to Claim 31, **characterized in that**
a) an amount of from 0 to 100% of the biomass produced is removed from the fermentation broth obtained in step b) of Claim 34, and
b) from the broth obtained in step a) an essentially dry and shaped product is prepared by a method selected from the group consisting of granulation, compacting, spray drying and extrusion.

38. Process according to Claim 37, **characterized in that** an acid selected from the group consisting of sulfuric acid, hydrochloric acid and phosphoric acid is added to an L-lysine-containing fermentation broth prior to or after step a).

39. Process according to Claim 37, **characterized in that** water is removed from the broth obtained prior to or after in step a).

40. Process according to Claim 37, **characterized in that** the shaped product obtained in or during step b) is sprayed with an oil.

## Revendications

1. Mutants isolés de bactéries corynéformes, qui contiennent un gène qui code pour un polypeptide doté d'une activité glucose-6-phosphate-déshydrogénase, **caractérisés en ce que** le polypeptide comprend une séquence d'acides aminés selon la SEQ ID NO:2, contenant, en position 321, une L-sérine.

2. Mutants de bactéries corynéformes selon la revendication 1, **caractérisés en ce qu'**il s'agit, pour les bactéries corynéformes, d'une bactérie choisie dans le groupe formé par Corynebacterium efficiens, Corynebacterium glutamicum, Corynebacterium thermoaminogenes et Corynebacterium aminogenes.

3. Mutants de bactéries corynéformes selon la revendication 2, **caractérisés en ce qu'**il s'agit de Corynebacterium glutamicum.

4. Mutants de bactéries corynéformes selon la revendication 1, 2 ou 3, **caractérisés en ce qu'**il s'agit de bactéries excrétant des acides aminés L.

5. Mutants de bactéries corynéformes selon la revendication 4, **caractérisés en ce qu'**il s'agit de bactéries excrétant de la L-lysine ou du L-tryptophane.

6. Mutants de bactéries corynéformes selon les revendications 1-5, **caractérisés en ce que** l'acide aminé L-sérine en position 8 est remplacé par un autre acide aminé protéinogène.

7. Mutants de bactéries corynéformes selon les revendications 1 à 6, **caractérisés en ce que** la protéine codante comprend une séquence d'acides aminés choisie dans le groupe formé par
a) la séquence d'acides aminés selon les séquences SEQ ID NO:6, SEQ ID NO:8 ou SEQ ID NO:10, et
b) la séquence d'acides aminés selon les séquences SEQ ID NO:6, SEQ ID NO:8 ou SEQ ID NO:10, y compris une ou au maximum 5 insertions ou délétions d'acides aminés,
l'activité enzymatique se distinguant d'au maximum 5% de l'activité du polypeptide présentant la séquence d'acides aminés des séquences SEQ ID NO:6, 8 ou 10 et une L-sérine étant contenue dans la position correspondant à la position 321.

8. Mutants de bactéries corynéformes selon la revendication 7, **caractérisés en ce que** le gène comprend la séquence nucléotidique des séquences SEQ ID NO:5, SEQ ID NO:7 ou SEQ ID NO:9.

9. Mutants de bactéries corynéformes, **caractérisés en ce qu'**ils peuvent être obtenus par les étapes suivantes :
a) traitement d'une bactérie corynéforme, qui présente l'aptitude à excréter des acides aminés, par un agent mutagène,
b) isolement et multiplication des mutants générés dans l'étape a),
c) préparation d'acides nucléiques des mutants obtenus dans l'étape b),
d) préparation d'une molécule d'acide nucléique en utilisant la réaction en chaîne par polymérase, l'acide nucléique de l'étape c), et une paire d'amorces, constituée d'une première amorce comprenant au moins 15 nucléotides successifs choisis dans la séquence nucléotidique entre la position 1 et 1267 des séquences SEQ ID NO:3 ou SEQ ID NO:11 et d'une deuxième amorce comprenant au moins 15 nucléotides consécutifs choisis dans la séquence nucléotidique complémentaire entre la position 2100 et 1271 des séquences SEQ ID NO:3 ou 11,
e) détermination de la séquence nucléotidique de la molécule d'acide nucléique obtenue dans l'étape d) et détermination de la séquence d'acides aminés codante,
f) le cas échéant comparaison de la séquence d'acides aminés déterminée dans l'étape e) avec les séquences SEQ ID NO:6, 8 ou 10, et
g) identification d'un mutant, qui contient un polynucléotide, qui code pour un polypeptide doté d'une activité glucose-6-phosphate déshydrogénase, qui contient une L-sérine dans la position correspondant à la position 321 de la séquence SEQ ID NO:2.

10. Polynucléotide isolé, qui code pour un polypeptide selon la séquence SEQ ID NO:2 doté d'une activité glucose-6-phosphate déshydrogénase, qui contient une L-sérine en position 321 de la séquence d'acides aminés SEQ ID NO:2.

11. Polynucléotide isolé selon la revendication 10, **caractérisé en ce que** la protéine codante comprend une séquence d'acides aminés choisie dans le groupe formé par
a) la séquence d'acides aminés selon les séquences SEQ ID NO:6, SEQ ID NO:8 ou SEQ ID NO:10,
b) la séquence d'acides aminés selon les séquences SEQ ID NO:6, SEQ ID NO:8 ou SEQ ID NO:10, y compris une ou au maximum 5 insertions d'acides aminés,
l'activité enzymatique se distinguant d'au maximum 5% de l'activité du polypeptide présentant la séquence d'acides aminés des séquences SEQ ID NO:6, 8 ou 10 et une L-sérine étant contenue dans la position correspondant à la position 321.

12. Polynucléotide isolé selon une ou plusieurs des revendications 10 ou 11, **caractérisé en ce que** le polypeptide codé comprend la séquence d'acides aminés selon les séquences SEQ ID NO:6, SEQ ID NO:8 ou SEQ ID NO:10.

13. Polynucléotide isolé selon la revendication 12, **caractérisé en ce que** la séquence nucléotidique comprend les séquences SEQ ID NO:5, SEQ ID NO:7 ou SEQ ID NO:9.

14. Polynucléotide isolé selon la revendication 13, **caractérisé en ce que** la séquence nucléotidique comprend la séquence SEQ ID NO:11.

15. Fragment de polynucléotide isolé, qui comprend une molécule d'acide nucléique, qui code au moins pour un cadre de lecture présentant une séquence d'acides aminés correspondant aux positions 307 à 335 de la séquence SEQ ID NO:2, une L-sérine étant contenue en position 321 de la SEQ ID NO:2.

16. Polynucléotide isolé selon la revendication 15, **caractérisé en ce qu'**il code au moins pour un cadre de lecture présentant une séquence d'acides aminés correspondant aux positions 307 à 335 des séquences SEQ ID NO:6, SEQ ID NO:8 ou SEQ ID NO:10.

17. Polynucléotide isolé selon la revendication 15, **caractérisé en ce que** la séquence d'acides aminés contient un ou plusieurs remplacements conservateurs d'acides aminés, les remplacements conservateurs d'acides aminés ne concernant pas la position 321.

18. Polynucléotide isolé selon la revendication 15, **caractérisé en ce qu'**il contient une ou plusieurs mutations silencieuses.

19. Polynucléotide isolé selon la revendication 16, **caractérisé en ce qu'**il comprend au moins la séquence nucléotidique correspondant aux positions 919 à 1005 des séquences SEQ ID NO:5, SEQ ID NO:7 ou SEQ ID NO:9.

20. Procédé pour la préparation d'une bactérie corynéforme recombinante, **caractérisé en ce qu'**on
a) transfère un polynucléotide isolé selon les revendications 10 à 14 ou selon les revendications 15 à 19 dans une bactérie corynéforme,
b) remplace le gène présent dans le chromosome de la bactérie corynéforme de la glucose-6-phosphate-déshydrogénase, qui code pour une séquence d'acides aminés avec de la glycine en position 321 et le cas échéant de la L-sérine en position 8 ou en une position comparable de la séquence d'acides aminés SEQ ID NO:2, par le polynucléotide de l'étape a), et
c) on multiplie la bactérie corynéforme obtenue selon les étapes a) et b).

21. Procédé pour la préparation d'un microorganisme recombinant, **caractérisé en ce qu'**on
a) transfère un polynucléotide isolé selon la revendication 10 à 14 dans un microorganisme,
b) réplique le polynucléotide isolé dans le microorganisme, et
c) multiplie le microorganisme obtenu selon les étapes a) et b).

22. Microorganisme recombinant qui contient le polynucléotide isolé selon les revendications 10 à 14 ou 15 à 19.

23. Microorganisme recombinant selon la revendication 22, **caractérisé en ce qu'**il s'agit d'une bactérie corynéforme ou d'une bactérie du genre Escherichia.

24. Microorganisme recombinant selon la revendication 23, **caractérisé en ce qu'**il s'agit, pour la bactérie corynéforme, du genre Corynebacterium.

25. Microorganisme recombinant selon la revendication 24, **caractérisé en ce qu'**il s'agit, pour la bactérie du genre Corynebacterium de l'espèce Corynebacterium glutamicum.

26. Vecteur, qui contient le polynucléotide isolé selon les revendications 10 à 14 ou 15 à 19.

27. Microorganisme recombinant, qui contient le vecteur selon la revendication 26.

28. Microorganisme recombinant selon la revendication 27, **caractérisé en ce qu'**il s'agit d'une bactérie corynéforme ou d'une bactérie du genre Escherichia.

29. Microorganisme recombinant selon la revendication 28, **caractérisé en ce qu'**il s'agit, pour la bactérie corynéforme, du genre Corynebacterium.

30. Microorganisme recombinant selon la revendication 29, **caractérisé en ce qu'**il s'agit, pour la bactérie du genre Corynebacterium de l'espèce Corynebacterium glutamicum.

31. Procédé pour la préparation d'un acide aminé L, **caractérisé en ce qu'**on
a) fermente une bactérie corynéforme isolée dans un milieu approprié, la bactérie contenant au moins une copie d'un gène codant pour un polypeptide doté d'une activité enzymatique glucose-6-phosphate-déshydrogénase, les séquences d'acides aminés du polypeptide étant définies dans les revendications 1, 6 et 7,
b) on enrichit l'acide aminé L dans le bouillon de fermentation ou dans les cellules de la bactérie.

32. Procédé selon la revendication 31, **caractérisé en ce qu'**il s'agit, pour la bactérie corynéforme isolée, d'un mutant selon la revendication 1 à 9.

33. Procédé selon la revendication 31, **caractérisé en ce qu'**il s'agit, pour la bactérie corynéforme isolée, d'une bactérie corynéforme recombinante qui contient un polynucléotide isolé selon la revendication 10 à 21 ou préparée en utilisant celui-ci.

34. Procédé selon la revendication 31, **caractérisé en ce qu'**on isole ou récupère l'acide aminé L.

35. Procédé selon la revendication 34, **caractérisé en ce qu'**on purifie l'acide aminé L.

36. Procédé selon la revendication 31, **caractérisé en ce qu'**on isole ou récupère l'acide amine L ensemble avec les constituants du bouillon de fermentation et/ou de la biomasse (> 0 à 100%).

37. Procédé selon la revendication 31, **caractérisé en ce qu'**on
a) élimine, du bouillon de fermentation obtenu dans l'étape b) de la revendication 34, la biomasse formée en une quantité de 0 à 100%, et
b) prépare, à partir du bouillon obtenu dans l'étape a), un produit pratiquement séché et façonné par un procédé choisi dans le groupe formé par la granulation, le compactage, le séchage par pulvérisation et l'extrusion.

38. Procédé selon la revendication 37, **caractérisé en ce qu'**on ajoute à un bouillon de fermentation contenant de la L-lysine, avant ou après l'étape a), un acide choisi dans le groupe formé par l'acide sulfurique, l'acide chlorhydrique et l'acide phosphorique.

39. Procédé selon la revendication 37, **caractérisé en ce qu'**on élimine l'eau du bouillon obtenu avant ou après l'étape a).

40. Procédé selon la revendication 37, **caractérisé en ce qu'**on asperge le produit façonné obtenu dans ou pendant l'étape b) d'une huile.


### SEQUENCE LISTING

<110> Degussa AG
<120> Allele des zwf-Gens aus coryneformen Bakterien
<130> 050009BT
<160> 24
<170> PatentIn version 3.3
<210> 1
   <211> 1545
   <212> DNA
   <213> Corynebacterium glutamicum
<220>
   <221> CDS
   <222> (1)..(1542)
   <223> zwf Wildtyp-Gen
<400> 1
   gtg agc aca aac acg acc ccc tcc agc tgg aca aac cca ctg cgc gac        48
   Val Ser Thr Asn Thr Thr Pro Ser Ser Trp Thr Asn Pro Leu Arg Asp
   1               5                   10                  15
   ccg cag gat aaa cga ctc ccc cgc atc gct ggc cct tcc ggc atg gtg        96
   Pro Gln Asp Lys Arg Leu Pro Arg Ile Ala Gly Pro Ser Gly Met Val
               20                  25                  30
   atc ttc ggt gtc act ggc gac ttg gct cga aag aag ctg ctc ccc gcc       144
   Ile Phe Gly Val Thr Gly Asp Leu Ala Arg Lys Lys Leu Leu Pro Ala
           35                  40                  45
   att tat gat cta gca aac cgc gga ttg ctg ccc cca gga ttc tcg ttg       192
   Ile Tyr Asp Leu Ala Asn Arg Gly Leu Leu Pro Pro Gly Phe Ser Leu
       50                  55                  60
   gta ggt tac ggc cgc cgc gaa tgg tcc aaa gaa gac ttt gaa aaa tac       240
   Val Gly Tyr Gly Arg Arg Glu Trp Ser Lys Glu Asp Phe Glu Lys Tyr
   65                  70                  75                  80
   gta cgc gat gcc gca agt gct ggt gct cgt acg gaa ttc cgt gaa aat       288
   Val Arg Asp Ala Ala Ser Ala Gly Ala Arg Thr Glu Phe Arg Glu Asn
                   85                  90                  95
   gtt tgg gag cgc ctc gcc gag ggt atg gaa ttt gtt cgc ggc aac ttt       336
   Val Trp Glu Arg Leu Ala Glu Gly Met Glu Phe Val Arg Gly Asn Phe
               100                 105                 110
   gat gat gat gca gct ttc gac aac ctc gct gca aca ctc aag cgc atc       384
   Asp Asp Asp Ala Ala Phe Asp Asn Leu Ala Ala Thr Leu Lys Arg Ile
           115                 120                 125
   gac aaa acc cgc ggc acc gcc ggc aac tgg gct tac tac ctg tcc att       432
   Asp Lys Thr Arg Gly Thr Ala Gly Asn Trp Ala Tyr Tyr Leu Ser Ile
       130                 135                 140
   cca cca gat tcc ttc aca gcg gtc tgc cac cag ctg gag cgt tcc ggc      480
   Pro Pro Asp Ser Phe Thr Ala Val Cys His Gln Leu Glu Arg Ser Gly
   145                 150                 155                 160
   atg gct gaa tcc acc gaa gaa gca tgg cgc cgc gtg atc atc gag aag      528
   Met Ala Glu Ser Thr Glu Glu Ala Trp Arg Arg Val Ile Ile Glu Lys
                   165                 170                 175
   cct ttc ggc cac aac ctc gaa tcc gca cac gag ctc aac cag ctg gtc      576
   Pro Phe Gly His Asn Leu Glu Ser Ala His Glu Leu Asn Gln Leu Val
               180                 185                 190
   aac gca gtc ttc cca gaa tct tct gtg ttc cgc atc gac cac tat ttg      624
   Asn Ala Val Phe Pro Glu Ser Ser Val Phe Arg Ile Asp His Tyr Leu
           195                 200                 205
   ggc aag gaa aca gtt caa aac atc ctg gct ctg cgt ttt gct aac cag      672
   Gly Lys Glu Thr Val Gln Asn Ile Leu Ala Leu Arg Phe Ala Asn Gln
       210                 215                 220
   ctg ttt gag cca ctg tgg aac tcc aac tac gtt gac cac gtc cag atc      720
   Leu Phe Glu Pro Leu Trp Asn Ser Asn Tyr Val Asp His Val Gln Ile
   225                 230                 235                 240
   acc atg gct gaa gat att ggc ttg ggt gga cgt gct ggt tac tac gac      768
   Thr Met Ala Glu Asp Ile Gly Leu Gly Gly Arg Ala Gly Tyr Tyr Asp
                   245                 250                 255
   ggc atc ggc gca gcc cgc gac gtc atc cag aac cac ctg atc cag ctc      816
   Gly Ile Gly Ala Ala Arg Asp Val Ile Gln Asn His Leu Ile Gln Leu
               260                 265                 270
   ttg gct ctg gtt gcc atg gaa gaa cca att tct ttc gtg cca gcg cag      864
   Leu Ala Leu Val Ala Met Glu Glu Pro Ile Ser Phe Val Pro Ala Gln
           275                 280                 285
   ctg cag gca gaa aag atc aag gtg ctc tct gcg aca aag ccg tgc tac      912
   Leu Gln Ala Glu Lys Ile Lys Val Leu Ser Ala Thr Lys Pro Cys Tyr
       290                 295                 300
   cca ttg gat aaa acc tcc gct cgt ggt cag tac gct gcc ggt tgg cag      960
   Pro Leu Asp Lys Thr Ser Ala Arg Gly Gln Tyr Ala Ala Gly Trp Gln
   305                 310                 315                 320
   ggc tct gag tta gtc aag gga ctt cgc gaa gaa gat ggc ttc aac cct     1008
   Gly Ser Glu Leu Val Lys Gly Leu Arg Glu Glu Asp Gly Phe Asn Pro
                   325                 330                 335
   gag tcc acc act gag act ttt gcg gct tgt acc tta gag atc acg tct     1056
   Glu Ser Thr Thr Glu Thr Phe Ala Ala Cys Thr Leu Glu Ile Thr Ser
               340                 345                 350
   cgt cgc tgg gct ggt gtg ccg ttc tac ctg cgc acc ggt aag cgt ctt     1104
   Arg Arg Trp Ala Gly Val Pro Phe Tyr Leu Arg Thr Gly Lys Arg Leu
           355                 360                 365
   ggt cgc cgt gtt act gag att gcc gtg gtg ttt aaa gac gca cca cac     1152
   Gly Arg Arg Val Thr Glu Ile Ala Val Val Phe Lys Asp Ala Pro His
       370                 375                 380
   cag cct ttc gac ggc gac atg act gta tcc ctt ggc caa aac gcc atc      1200
   Gln Pro Phe Asp Gly Asp Met Thr Val Ser Leu Gly Gln Asn Ala Ile
   385                 390                 395                 400
   gtg att cgc gtg cag cct gat gaa ggt gtg ctc atc cgc ttc ggt tcc      1248
   Val Ile Arg Val Gln Pro Asp Glu Gly Val Leu Ile Arg Phe Gly Ser
                   405                 410                 415
   aag gtt cca ggt tct gcc atg gaa gtc cgt gac gtc aac atg gac ttc      1296
   Lys Val Pro Gly Ser Ala Met Glu Val Arg Asp Val Asn Met Asp Phe
               420                 425                 430
   tcc tac tca gaa tcc ttc act gaa gaa tca cct gaa gca tac gag cgc      1344
   Ser Tyr Ser Glu Ser Phe Thr Glu Glu Ser Pro Glu Ala Tyr Glu Arg
           435                 440                 445
   ctc att ttg gat gcg ctg tta gat gaa tcc agc ctc ttc cct acc aac      1392
   Leu Ile Leu Asp Ala Leu Leu Asp Glu Ser Ser Leu Phe Pro Thr Asn
       450                 455                 460
   gag gaa gtg gaa ctg agc tgg aag att ctg gat cca att ctt gaa gca      1440
   Glu Glu Val Glu Leu Ser Trp Lys Ile Leu Asp Pro Ile Leu Glu Ala
   465                 470                 475                 480
   tgg gat gcc gat gga gaa cca gag gat tac cca gcg ggt acg tgg ggt      1488
   Trp Asp Ala Asp Gly Glu Pro Glu Asp Tyr Pro Ala Gly Thr Trp Gly
                   485                 490                 495
   cca aag agc gct gat gaa atg ctt tcc cgc aac ggt cac acc tgg cgc      1536
   Pro Lys Ser Ala Asp Glu Met Leu Ser Arg Asn Gly His Thr Trp Arg
               500                 505                 510
   agg cca taa                                                          1545
   Arg Pro
<210> 2
   <211> 514
   <212> PRT
   <213> Corynebacterium glutamicum
<400> 2
   Val Ser Thr Asn Thr Thr Pro Ser Ser Trp Thr Asn Pro Leu Arg Asp
   1               5                   10                  15
   Pro Gln Asp Lys Arg Leu Pro Arg Ile Ala Gly Pro Ser Gly Met Val
               20                  25                  30
   Ile Phe Gly Val Thr Gly Asp Leu Ala Arg Lys Lys Leu Leu Pro Ala
           35                  40                  45
   Ile Tyr Asp Leu Ala Asn Arg Gly Leu Leu Pro Pro Gly Phe Ser Leu
       50                  55                  60
   Val Gly Tyr Gly Arg Arg Glu Trp Ser Lys Glu Asp Phe Glu Lys Tyr
   65                  70                  75                  80
   Val Arg Asp Ala Ala Ser Ala Gly Ala Arg Thr Glu Phe Arg Glu Asn
   85 90 95
   Val Trp Glu Arg Leu Ala Glu Gly Met Glu Phe Val Arg Gly Asn Phe
   100 105 110
   Asp Asp Asp Ala Ala Phe Asp Asn Leu Ala Ala Thr Leu Lys Arg Ile
   115 120 125
   Asp Lys Thr Arg Gly Thr Ala Gly Asn Trp Ala Tyr Tyr Leu Ser Ile
   130 135 140
   Pro Pro Asp Ser Phe Thr Ala Val Cys His Gln Leu Glu Arg Ser Gly
   145 150 155 160
   Met Ala Glu Ser Thr Glu Glu Ala Trp Arg Arg Val Ile Ile Glu Lys
   165 170 175
   Pro Phe Gly His Asn Leu Glu Ser Ala His Glu Leu Asn Gln Leu Val
   180 185 190
   Asn Ala Val Phe Pro Glu Ser Ser Val Phe Arg Ile Asp His Tyr Leu
   195 200 205
   Gly Lys Glu Thr Val Gln Asn Ile Leu Ala Leu Arg Phe Ala Asn Gln
   210 215 220
   Leu Phe Glu Pro Leu Trp Asn Ser Asn Tyr Val Asp His Val Gln Ile
   225 230 235 240
   Thr Met Ala Glu Asp Ile Gly Leu Gly Gly Arg Ala Gly Tyr Tyr Asp
   245 250 255
   Gly Ile Gly Ala Ala Arg Asp Val Ile Gln Asn His Leu Ile Gln Leu
   260 265 270
   Leu Ala Leu Val Ala Met Glu Glu Pro Ile Ser Phe Val Pro Ala Gln
   275 280 285
   Leu Gln Ala Glu Lys Ile Lys Val Leu Ser Ala Thr Lys Pro Cys Tyr
   290 295 300
   Pro Leu Asp Lys Thr Ser Ala Arg Gly Gln Tyr Ala Ala Gly Trp Gln
   305 310 315 320
   Gly Ser Glu Leu Val Lys Gly Leu Arg Glu Glu Asp Gly Phe Asn Pro
                   325                 330                 335
   Glu Ser Thr Thr Glu Thr Phe Ala Ala Cys Thr Leu Glu Ile Thr Ser
               340                 345                 350
   Arg Arg Trp Ala Gly Val Pro Phe Tyr Leu Arg Thr Gly Lys Arg Leu
           355                 360                 365
   Gly Arg Arg Val Thr Glu Ile Ala Val Val Phe Lys Asp Ala Pro His
       370                 375                 380
   Gln Pro Phe Asp Gly Asp Met Thr Val Ser Leu Gly Gln Asn Ala Ile
   385                 390                 395                 400
   Val Ile Arg Val Gln Pro Asp Glu Gly Val Leu Ile Arg Phe Gly Ser
                   405                 410                 415
   Lys Val Pro Gly Ser Ala Met Glu Val Arg Asp Val Asn Met Asp Phe
               420                 425                 430
   Ser Tyr Ser Glu Ser Phe Thr Glu Glu Ser Pro Glu Ala Tyr Glu Arg
           435                 440                 445
   Leu Ile Leu Asp Ala Leu Leu Asp Glu Ser Ser Leu Phe Pro Thr Asn
       450                 455                 460
   Glu Glu Val Glu Leu Ser Trp Lys Ile Leu Asp Pro Ile Leu Glu Ala
   465                 470                 475                 480
   Trp Asp Ala Asp Gly Glu Pro Glu Asp Tyr Pro Ala Gly Thr Trp Gly
                   485                 490                 495
   Pro Lys Ser Ala Asp Glu Met Leu Ser Arg Asn Gly His Thr Trp Arg
               500                 505                 510
   Arg Pro
<210> 3
   <211> 2100
   <212> DNA
   <213> Corynebacterium glutamicum
<220>
   <221> CDS
   <222> (308)..(1849)
   <223> zwf Wildtyp-Gen
<400> 3
   tcgacgcggt tctggagcag ggcaacctgc acggtgacac cctgtccaac tccgcggcag      60
   aagctgacgc tgtgttctcc cagcttgagg ctctgggcgt tgacttggca gatgtcttcc     120
   aggtcctgga gaccgagggt gtggacaagt tcgttgcttc ttggagcgaa ctgcttgagt     180
   ccatggaagc tcgcctgaag tagaatcagc acgctgcatc agtaacggcg acatgaaatc     240
   gaattagttc gatcttatgt ggccgttaca catctttcat taaagaaagg atcgtgacac     300
   taccatc gtg agc aca aac acg acc ccc tcc agc tgg aca aac cca ctg      349
           Val Ser Thr Asn Thr Thr Pro Ser Ser Trp Thr Asn Pro Leu
           1               5                   10
   cgc gac ccg cag gat aaa cga ctc ccc cgc atc gct ggc cct tcc ggc      397
   Arg Asp Pro Gln Asp Lys Arg Leu Pro Arg Ile Ala Gly Pro Ser Gly
   15                  20                  25                  30
   atg gtg atc ttc ggt gtc act ggc gac ttg gct cga aag aag ctg ctc      445
   Met Val Ile Phe Gly Val Thr Gly Asp Leu Ala Arg Lys Lys Leu Leu
                   35                  40                  45
   ccc gcc att tat gat cta gca aac cgc gga ttg ctg ccc cca gga ttc      493
   Pro Ala Ile Tyr Asp Leu Ala Asn Arg Gly Leu Leu Pro Pro Gly Phe
               50                  55                  60
   tcg ttg gta ggt tac ggc cgc cgc gaa tgg tcc aaa gaa gac ttt gaa      541
   Ser Leu Val Gly Tyr Gly Arg Arg Glu Trp Ser Lys Glu Asp Phe Glu
           65                  70                  75
   aaa tac gta cgc gat gcc gca agt gct ggt gct cgt acg gaa ttc cgt      589
   Lys Tyr Val Arg Asp Ala Ala Ser Ala Gly Ala Arg Thr Glu Phe Arg
       80                  85                  90
   gaa aat gtt tgg gag cgc ctc gcc gag ggt atg gaa ttt gtt cgc ggc      637
   Glu Asn Val Trp Glu Arg Leu Ala Glu Gly Met Glu Phe Val Arg Gly
   95                  100                 105                 110
   aac ttt gat gat gat gca gct ttc gac aac ctc gct gca aca ctc aag      685
   Asn Phe Asp Asp Asp Ala Ala Phe Asp Asn Leu Ala Ala Thr Leu Lys
                   115                 120                 125
   cgc atc gac aaa acc cgc ggc acc gcc ggc aac tgg gct tac tac ctg      733
   Arg Ile Asp Lys Thr Arg Gly Thr Ala Gly Asn Trp Ala Tyr Tyr Leu
               130                 135                 140
   tcc att cca cca gat tcc ttc aca gcg gtc tgc cac cag ctg gag cgt      781
   Ser Ile Pro Pro Asp Ser Phe Thr Ala Val Cys His Gln Leu Glu Arg
           145                 150                 155
   tcc ggc atg gct gaa tcc acc gaa gaa gca tgg cgc cgc gtg atc atc      829
   Ser Gly Met Ala Glu Ser Thr Glu Glu Ala Trp Arg Arg Val Ile Ile
       160                 165                 170
   gag aag cct ttc ggc cac aac ctc gaa tcc gca cac gag ctc aac cag      877
   Glu Lys Pro Phe Gly His Asn Leu Glu Ser Ala His Glu Leu Asn Gln
   175                 180                 185                 190
   ctg gtc aac gca gtc ttc cca gaa tct tct gtg ttc cgc atc gac cac     925
   Leu Val Asn Ala Val Phe Pro Glu Ser Ser Val Phe Arg Ile Asp His
                   195                 200                 205
   tat ttg ggc aag gaa aca gtt caa aac atc ctg gct ctg cgt ttt gct     973
   Tyr Leu Gly Lys Glu Thr Val Gln Asn Ile Leu Ala Leu Arg Phe Ala
               210                 215                 220
   aac cag ctg ttt gag cca ctg tgg aac tcc aac tac gtt gac cac gtc    1021
   Asn Gln Leu Phe Glu Pro Leu Trp Asn Ser Asn Tyr Val Asp His Val
           225                 230                 235
   cag atc acc atg gct gaa gat att ggc ttg ggt gga cgt gct ggt tac    1069
   Gln Ile Thr Met Ala Glu Asp Ile Gly Leu Gly Gly Arg Ala Gly Tyr
       240                 245                 250
   tac gac ggc atc ggc gca gcc cgc gac gtc atc cag aac cac ctg atc    1117
   Tyr Asp Gly Ile Gly Ala Ala Arg Asp Val Ile Gln Asn His Leu Ile
   255                 260                 265                 270
   cag ctc ttg gct ctg gtt gcc atg gaa gaa cca att tct ttc gtg cca    1165
   Gln Leu Leu Ala Leu Val Ala Met Glu Glu Pro Ile Ser Phe Val Pro
                   275                 280                 285
   gcg cag ctg cag gca gaa aag atc aag gtg ctc tct gcg aca aag ccg    1213
   Ala Gln Leu Gln Ala Glu Lys Ile Lys Val Leu Ser Ala Thr Lys Pro
               290                 295                 300
   tgc tac cca ttg gat aaa acc tcc gct cgt ggt cag tac gct gcc ggt    1261
   Cys Tyr Pro Leu Asp Lys Thr Ser Ala Arg Gly Gln Tyr Ala Ala Gly
           305                 310                 315
   tgg cag ggc tct gag tta gtc aag gga ctt cgc gaa gaa gat ggc ttc    1309
   Trp Gln Gly Ser Glu Leu Val Lys Gly Leu Arg Glu Glu Asp Gly Phe
       320                 325                 330
   aac cct gag tcc acc act gag act ttt gcg gct tgt acc tta gag atc    1357
   Asn Pro Glu Ser Thr Thr Glu Thr Phe Ala Ala Cys Thr Leu Glu Ile
   335                 340                 345                 350
   acg tct cgt cgc tgg gct ggt gtg ccg ttc tac ctg cgc acc ggt aag    1405
   Thr Ser Arg Arg Trp Ala Gly Val Pro Phe Tyr Leu Arg Thr Gly Lys
                   355                 360                 365
   cgt ctt ggt cgc cgt gtt act gag att gcc gtg gtg ttt aaa gac gca    1453
   Arg Leu Gly Arg Arg Val Thr Glu Ile Ala Val Val Phe Lys Asp Ala
               370                 375                 380
   cca cac cag cct ttc gac ggc gac atg act gta tcc ctt ggc caa aac    1501
   Pro His Gln Pro Phe Asp Gly Asp Met Thr Val Ser Leu Gly Gln Asn
           385                 390                 395
   gcc atc gtg att cgc gtg cag cct gat gaa ggt gtg ctc atc cgc ttc    1549
   Ala Ile Val Ile Arg Val Gln Pro Asp Glu Gly Val Leu Ile Arg Phe
       400                 405                 410
   ggt tcc aag gtt cca ggt tct gcc atg gaa gtc cgt gac gtc aac atg    1597
   Gly Ser Lys Val Pro Gly Ser Ala Met Glu Val Arg Asp Val Asn Met
   415                 420                 425                 430
   gac ttc tcc tac tca gaa tcc ttc act gaa gaa tca cct gaa gca tac      1645
   Asp Phe Ser Tyr Ser Glu Ser Phe Thr Glu Glu Ser Pro Glu Ala Tyr
                   435                 440                 445
   gag cgc ctc att ttg gat gcg ctg tta gat gaa tcc agc ctc ttc cct      1693
   Glu Arg Leu Ile Leu Asp Ala Leu Leu Asp Glu Ser Ser Leu Phe Pro
               450                 455                 460
   acc aac gag gaa gtg gaa ctg agc tgg aag att ctg gat cca att ctt      1741
   Thr Asn Glu Glu Val Glu Leu Ser Trp Lys Ile Leu Asp Pro Ile Leu
           465                 470                 475
   gaa gca tgg gat gcc gat gga gaa cca gag gat tac cca gcg ggt acg      1789
   Glu Ala Trp Asp Ala Asp Gly Glu Pro Glu Asp Tyr Pro Ala Gly Thr
       480                 485                 490
   tgg ggt cca aag agc gct gat gaa atg ctt tcc cgc aac ggt cac acc      1837
   Trp Gly Pro Lys Ser Ala Asp Glu Met Leu Ser Arg Asn Gly His Thr
   495                 500                 505                 510
   tgg cgc agg cca taatttaggg gcaaaaaatg atctttgaac ttccggatac          1889
   Trp Arg Arg Pro
   caccacccag caaatttcca agaccctaac tcgactgcgt gaatcgggca cccaggtcac   1949
   caccggccga gtgctcaccc tcatcgtggt cactgactcc gaaagcgatg tcgctgcagt   2009
   taccgagtcc accaatgaag cctcgcgcga gcacccatct cgcgtgatca ttttggtggt   2069
   tggcgataaa actgcagaaa acaaagttga c                                   2100
<210> 4
   <211> 514
   <212> PRT
   <213> Corynebacterium glutamicum
<400> 4
   Val Ser Thr Asn Thr Thr Pro Ser Ser Trp Thr Asn Pro Leu Arg Asp
   1               5                   10                  15
   Pro Gln Asp Lys Arg Leu Pro Arg Ile Ala Gly Pro Ser Gly Met Val
               20                  25                  30
   Ile Phe Gly Val Thr Gly Asp Leu Ala Arg Lys Lys Leu Leu Pro Ala
           35                  40                  45
   Ile Tyr Asp Leu Ala Asn Arg Gly Leu Leu Pro Pro Gly Phe Ser Leu
       50                  55                  60
   Val Gly Tyr Gly Arg Arg Glu Trp Ser Lys Glu Asp Phe Glu Lys Tyr
   65                  70                  75                  80
   Val Arg Asp Ala Ala Ser Ala Gly Ala Arg Thr Glu Phe Arg Glu Asn
                   85                  90                  95
   Val Trp Glu Arg Leu Ala Glu Gly Met Glu Phe Val Arg Gly Asn Phe
               100                 105                 110
   Asp Asp Asp Ala Ala Phe Asp Asn Leu Ala Ala Thr Leu Lys Arg Ile
           115                 120                 125
   Asp Lys Thr Arg Gly Thr Ala Gly Asn Trp Ala Tyr Tyr Leu Ser Ile
       130                 135                 140
   Pro Pro Asp Ser Phe Thr Ala Val Cys His Gln Leu Glu Arg Ser Gly
   145                 150                 155                 160
   Met Ala Glu Ser Thr Glu Glu Ala Trp Arg Arg Val Ile Ile Glu Lys
                   165                 170                 175
   Pro Phe Gly His Asn Leu Glu Ser Ala His Glu Leu Asn Gln Leu Val
               180                 185                 190
   Asn Ala Val Phe Pro Glu Ser Ser Val Phe Arg Ile Asp His Tyr Leu
           195                 200                 205
   Gly Lys Glu Thr Val Gln Asn Ile Leu Ala Leu Arg Phe Ala Asn Gln
       210                 215                 220
   Leu Phe Glu Pro Leu Trp Asn Ser Asn Tyr Val Asp His Val Gln Ile
   225                 230                 235                 240
   Thr Met Ala Glu Asp Ile Gly Leu Gly Gly Arg Ala Gly Tyr Tyr Asp
                   245                 250                 255
   Gly Ile Gly Ala Ala Arg Asp Val Ile Gln Asn His Leu Ile Gln Leu
               260                 265                 270
   Leu Ala Leu Val Ala Met Glu Glu Pro Ile Ser Phe Val Pro Ala Gln
           275                 280                 285
   Leu Gln Ala Glu Lys Ile Lys Val Leu Ser Ala Thr Lys Pro Cys Tyr
       290                 295                 300
   Pro Leu Asp Lys Thr Ser Ala Arg Gly Gln Tyr Ala Ala Gly Trp Gln
   305                 310                 315                 320
   Gly Ser Glu Leu Val Lys Gly Leu Arg Glu Glu Asp Gly Phe Asn Pro
                   325                 330                 335
   Glu Ser Thr Thr Glu Thr Phe Ala Ala Cys Thr Leu Glu Ile Thr Ser
   340 345 350
   Arg Arg Trp Ala Gly Val Pro Phe Tyr Leu Arg Thr Gly Lys Arg Leu
   355 360 365
   Gly Arg Arg Val Thr Glu Ile Ala Val Val Phe Lys Asp Ala Pro His
   370 375 380
   Gln Pro Phe Asp Gly Asp Met Thr Val Ser Leu Gly Gln Asn Ala Ile
   385 390 395 400
   Val Ile Arg Val Gln Pro Asp Glu Gly Val Leu Ile Arg Phe Gly Ser
   405 410 415
   Lys Val Pro Gly Ser Ala Met Glu Val Arg Asp Val Asn Met Asp Phe
   420 425 430
   Ser Tyr Ser Glu Ser Phe Thr Glu Glu Ser Pro Glu Ala Tyr Glu Arg
   435 440 445
   Leu Ile Leu Asp Ala Leu Leu Asp Glu Ser Ser Leu Phe Pro Thr Asn
   450 455 460
   Glu Glu Val Glu Leu Ser Trp Lys Ile Leu Asp Pro Ile Leu Glu Ala
   465 470 475 480
   Trp Asp Ala Asp Gly Glu Pro Glu Asp Tyr Pro Ala Gly Thr Trp Gly
   485 490 495
   Pro Lys Ser Ala Asp Glu Met Leu Ser Arg Asn Gly His Thr Trp Arg
   500 505 510
   Arg Pro
<210> 5
   <211> 1545
   <212> DNA
   <213> Corynebacterium glutamicum
<220>
   <221> CDS
   <222> (1)..(1542)
   <223> zwf-Allel
<220>
   <221> mutation
   <222> (961)..(961)
   <223> G -> A Transition
<400> 5
   gtg agc aca aac acg acc ccc tcc agc tgg aca aac cca ctg cgc gac      48
   Val Ser Thr Asn Thr Thr Pro Ser Ser Trp Thr Asn Pro Leu Arg Asp
   1               5                   10                  15
   ccg cag gat aaa cga ctc ccc cgc atc gct ggc cct tcc ggc atg gtg      96
   Pro Gln Asp Lys Arg Leu Pro Arg Ile Ala Gly Pro Ser Gly Met Val
               20                  25                  30
   atc ttc ggt gtc act ggc gac ttg gct cga aag aag ctg ctc ccc gcc     144
   Ile Phe Gly Val Thr Gly Asp Leu Ala Arg Lys Lys Leu Leu Pro Ala
           35                  40                  45
   att tat gat cta gca aac cgc gga ttg ctg ccc cca gga ttc tcg ttg     192
   Ile Tyr Asp Leu Ala Asn Arg Gly Leu Leu Pro Pro Gly Phe Ser Leu
       50                  55                  60
   gta ggt tac ggc cgc cgc gaa tgg tcc aaa gaa gac ttt gaa aaa tac     240
   Val Gly Tyr Gly Arg Arg Glu Trp Ser Lys Glu Asp Phe Glu Lys Tyr
   65                  70                  75                  80
   gta cgc gat gcc gca agt gct ggt gct cgt acg gaa ttc cgt gaa aat     288
   Val Arg Asp Ala Ala Ser Ala Gly Ala Arg Thr Glu Phe Arg Glu Asn
                   85                  90                  95
   gtt tgg gag cgc ctc gcc gag ggt atg gaa ttt gtt cgc ggc aac ttt     336
   Val Trp Glu Arg Leu Ala Glu Gly Met Glu Phe Val Arg Gly Asn Phe
               100                 105                 110
   gat gat gat gca gct ttc gac aac ctc gct gca aca ctc aag cgc atc     384
   Asp Asp Asp Ala Ala Phe Asp Asn Leu Ala Ala Thr Leu Lys Arg Ile
           115                 120                 125
   gac aaa acc cgc ggc acc gcc ggc aac tgg gct tac tac ctg tcc att     432
   Asp Lys Thr Arg Gly Thr Ala Gly Asn Trp Ala Tyr Tyr Leu Ser Ile
       130                 135                 140
   cca cca gat tcc ttc aca gcg gtc tgc cac cag ctg gag cgt tcc ggc     480
   Pro Pro Asp Ser Phe Thr Ala Val Cys His Gln Leu Glu Arg Ser Gly
   145                 150                 155                 160
   atg gct gaa tcc acc gaa gaa gca tgg cgc cgc gtg atc atc gag aag     528
   Met Ala Glu Ser Thr Glu Glu Ala Trp Arg Arg Val Ile Ile Glu Lys
                   165                 170                 175
   cct ttc ggc cac aac ctc gaa tcc gca cac gag ctc aac cag ctg gtc     576
   Pro Phe Gly His Asn Leu Glu Ser Ala His Glu Leu Asn Gln Leu Val
               180                 185                 190
   aac gca gtc ttc cca gaa tct tct gtg ttc cgc atc gac cac tat ttg     624
   Asn Ala Val Phe Pro Glu Ser Ser Val Phe Arg Ile Asp His Tyr Leu
           195                 200                 205
   ggc aag gaa aca gtt caa aac atc ctg gct ctg cgt ttt gct aac cag     672
   Gly Lys Glu Thr Val Gln Asn Ile Leu Ala Leu Arg Phe Ala Asn Gln
       210                 215                 220
   ctg ttt gag cca ctg tgg aac tcc aac tac gtt gac cac gtc cag atc     720
   Leu Phe Glu Pro Leu Trp Asn Ser Asn Tyr Val Asp His Val Gln Ile
   225                 230                 235                 240
   acc atg gct gaa gat att ggc ttg ggt gga cgt gct ggt tac tac gac      768
   Thr Met Ala Glu Asp Ile Gly Leu Gly Gly Arg Ala Gly Tyr Tyr Asp
                   245                 250                 255
   ggc atc ggc gca gcc cgc gac gtc atc cag aac cac ctg atc cag ctc      816
   Gly Ile Gly Ala Ala Arg Asp Val Ile Gln Asn His Leu Ile Gln Leu
               260                 265                 270
   ttg gct ctg gtt gcc atg gaa gaa cca att tct ttc gtg cca gcg cag      864
   Leu Ala Leu Val Ala Met Glu Glu Pro Ile Ser Phe Val Pro Ala Gln
           275                 280                 285
   ctg cag gca gaa aag atc aag gtg ctc tct gcg aca aag ccg tgc tac      912
   Leu Gln Ala Glu Lys Ile Lys Val Leu Ser Ala Thr Lys Pro Cys Tyr
       290                 295                 300
   cca ttg gat aaa acc tcc gct cgt ggt cag tac gct gcc ggt tgg cag      960
   Pro Leu Asp Lys Thr Ser Ala Arg Gly Gln Tyr Ala Ala Gly Trp Gln
   305                 310                 315                 320
   agc tct gag tta gtc aag gga ctt cgc gaa gaa gat ggc ttc aac cct     1008
   Ser Ser Glu Leu Val Lys Gly Leu Arg Glu Glu Asp Gly Phe Asn Pro
                   325                 330                 335
   gag tcc acc act gag act ttt gcg gct tgt acc tta gag atc acg tct     1056
   Glu Ser Thr Thr Glu Thr Phe Ala Ala Cys Thr Leu Glu Ile Thr Ser
               340                 345                 350
   cgt cgc tgg gct ggt gtg ccg ttc tac ctg cgc acc ggt aag cgt ctt     1104
   Arg Arg Trp Ala Gly Val Pro Phe Tyr Leu Arg Thr Gly Lys Arg Leu
           355                 360                 365
   ggt cgc cgt gtt act gag att gcc gtg gtg ttt aaa gac gca cca cac     1152
   Gly Arg Arg Val Thr Glu Ile Ala Val Val Phe Lys Asp Ala Pro His
       370                 375                 380
   cag cct ttc gac ggc gac atg act gta tcc ctt ggc caa aac gcc atc     1200
   Gln Pro Phe Asp Gly Asp Met Thr Val Ser Leu Gly Gln Asn Ala Ile
   385                 390                 395                 400
   gtg att cgc gtg cag cct gat gaa ggt gtg ctc atc cgc ttc ggt tcc     1248
   Val Ile Arg Val Gln Pro Asp Glu Gly Val Leu Ile Arg Phe Gly Ser
                   405                 410                 415
   aag gtt cca ggt tct gcc atg gaa gtc cgt gac gtc aac atg gac ttc     1296
   Lys Val Pro Gly Ser Ala Met Glu Val Arg Asp Val Asn Met Asp Phe
               420                 425                 430
   tcc tac tca gaa tcc ttc act gaa gaa tca cct gaa gca tac gag cgc     1344
   Ser Tyr Ser Glu Ser Phe Thr Glu Glu Ser Pro Glu Ala Tyr Glu Arg
           435                 440                 445
   ctc att ttg gat gcg ctg tta gat gaa tcc agc ctc ttc cct acc aac     1392
   Leu Ile Leu Asp Ala Leu Leu Asp Glu Ser Ser Leu Phe Pro Thr Asn
       450                 455                 460
   gag gaa gtg gaa ctg agc tgg aag att ctg gat cca att ctt gaa gca     1440
   Glu Glu Val Glu Leu Ser Trp Lys Ile Leu Asp Pro Ile Leu Glu Ala
   465                 470                 475                 480
   tgg gat gcc gat gga gaa cca gag gat tac cca gcg ggt acg tgg ggt      1488
   Trp Asp Ala Asp Gly Glu Pro Glu Asp Tyr Pro Ala Gly Thr Trp Gly
                   485                 490                 495
   cca aag agc gct gat gaa atg ctt tcc cgc aac ggt cac acc tgg cgc      1536
   Pro Lys Ser Ala Asp Glu Met Leu Ser Arg Asn Gly His Thr Trp Arg
               500                 505                 510
   agg cca taa                                                          1545
   Arg Pro
<210> 6
   <211> 514
   <212> PRT
   <213> Corynebacterium glutamicum
<400> 6
   Val Ser Thr Asn Thr Thr Pro Ser Ser Trp Thr Asn Pro Leu Arg Asp
   1               5                   10                  15
   Pro Gln Asp Lys Arg Leu Pro Arg Ile Ala Gly Pro Ser Gly Met Val
               20                  25                  30
   Ile Phe Gly Val Thr Gly Asp Leu Ala Arg Lys Lys Leu Leu Pro Ala
           35                  40                  45
   Ile Tyr Asp Leu Ala Asn Arg Gly Leu Leu Pro Pro Gly Phe Ser Leu
       50                  55                  60
   Val Gly Tyr Gly Arg Arg Glu Trp Ser Lys Glu Asp Phe Glu Lys Tyr
   65                  70                  75                  80
   Val Arg Asp Ala Ala Ser Ala Gly Ala Arg Thr Glu Phe Arg Glu Asn
                   85                  90                  95
   Val Trp Glu Arg Leu Ala Glu Gly Met Glu Phe Val Arg Gly Asn Phe
               100                 105                 110
   Asp Asp Asp Ala Ala Phe Asp Asn Leu Ala Ala Thr Leu Lys Arg Ile
           115                 120                 125
   Asp Lys Thr Arg Gly Thr Ala Gly Asn Trp Ala Tyr Tyr Leu Ser Ile
       130                 135                 140
   Pro Pro Asp Ser Phe Thr Ala Val Cys His Gln Leu Glu Arg Ser Gly
   145                 150                 155                 160
   Met Ala Glu Ser Thr Glu Glu Ala Trp Arg Arg Val Ile Ile Glu Lys
                   165                 170                 175
   Pro Phe Gly His Asn Leu Glu Ser Ala His Glu Leu Asn Gln Leu Val
               180                 185                     190
   Asn Ala Val Phe Pro Glu Ser Ser Val Phe Arg Ile Asp His Tyr Leu
           195                 200                 205
   Gly Lys Glu Thr Val Gln Asn Ile Leu Ala Leu Arg Phe Ala Asn Gln
       210                 215                 220
   Leu Phe Glu Pro Leu Trp Asn Ser Asn Tyr Val Asp His Val Gln Ile
   225                 230                 235                     240
   Thr Met Ala Glu Asp Ile Gly Leu Gly Gly Arg Ala Gly Tyr Tyr Asp
                   245                 250                     255
   Gly Ile Gly Ala Ala Arg Asp Val Ile Gln Asn His Leu Ile Gln Leu
               260                 265                     270
   Leu Ala Leu Val Ala Met Glu Glu Pro Ile Ser Phe Val Pro Ala Gln
           275                 280                     285
   Leu Gln Ala Glu Lys Ile Lys Val Leu Ser Ala Thr Lys Pro Cys Tyr
       290                 295                     300
   Pro Leu Asp Lys Thr Ser Ala Arg Gly Gln Tyr Ala Ala Gly Trp Gln
   305                 310                 315                     320
   Ser Ser Glu Leu Val Lys Gly Leu Arg Glu Glu Asp Gly Phe Asn Pro
                   325                 330                     335
   Glu Ser Thr Thr Glu Thr Phe Ala Ala Cys Thr Leu Glu Ile Thr Ser
               340                 345                     350
   Arg Arg Trp Ala Gly Val Pro Phe Tyr Leu Arg Thr Gly Lys Arg Leu
           355                 360                     365
   Gly Arg Arg Val Thr Glu Ile Ala Val Val Phe Lys Asp Ala Pro His
       370                 375                     380
   Gln Pro Phe Asp Gly Asp Met Thr Val Ser Leu Gly Gln Asn Ala Ile
   385                 390                 395                     400
   Val Ile Arg Val Gln Pro Asp Glu Gly Val Leu Ile Arg Phe Gly Ser
                   405                 410                     415
   Lys Val Pro Gly Ser Ala Met Glu Val Arg Asp Val Asn Met Asp Phe
   420 425 430
   Ser Tyr Ser Glu Ser Phe Thr Glu Glu Ser Pro Glu Ala Tyr Glu Arg
   435 440 445
   Leu Ile Leu Asp Ala Leu Leu Asp Glu Ser Ser Leu Phe Pro Thr Asn
   450 455 460
   Glu Glu Val Glu Leu Ser Trp Lys Ile Leu Asp Pro Ile Leu Glu Ala
   465 470 475 480
   Trp Asp Ala Asp Gly Glu Pro Glu Asp Tyr Pro Ala Gly Thr Trp Gly
   485 490 495
   Pro Lys Ser Ala Asp Glu Met Leu Ser Arg Asn Gly His Thr Trp Arg
   500 505 510
   Arg Pro
<210> 7
   <211> 1545
   <212> DNA
   <213> Corynebacterium glutamicum
<220>
   <221> CDS
   <222> (1)..(1542)
   <223> zwf-Allel
<220>
   <221> mutation
   <222> (22)..(22)
   <223> T -> A Transversion
<220>
   <221> mutation
   <222> (961)..(961)
   <223> G -> A Transition
<400> 7
   gtg agc aca aac acg acc ccc acc agc tgg aca aac cca ctg cgc gac 48
   Val Ser Thr Asn Thr Thr Pro Thr Ser Trp Thr Asn Pro Leu Arg Asp
   1 5 10 15
   ccg cag gat aaa cga ctc ccc cgc atc gct ggc cct tcc ggc atg gtg 96
   Pro Gln Asp Lys Arg Leu Pro Arg Ile Ala Gly Pro Ser Gly Met Val
   20 25 30
   atc ttc ggt gtc act ggc gac ttg gct cga aag aag ctg ctc ccc gcc 144
   Ile Phe Gly Val Thr Gly Asp Leu Ala Arg Lys Lys Leu Leu Pro Ala
   35 40 45
   att tat gat cta gca aac cgc gga ttg ctg ccc cca gga ttc tcg ttg      192
   Ile Tyr Asp Leu Ala Asn Arg Gly Leu Leu Pro Pro Gly Phe Ser Leu
       50                  55                  60
   gta ggt tac ggc cgc cgc gaa tgg tcc aaa gaa gac ttt gaa aaa tac      240
   Val Gly Tyr Gly Arg Arg Glu Trp Ser Lys Glu Asp Phe Glu Lys Tyr
   65                  70                  75                  80
   gta cgc gat gcc gca agt gct ggt gct cgt acg gaa ttc cgt gaa aat      288
   Val Arg Asp Ala Ala Ser Ala Gly Ala Arg Thr Glu Phe Arg Glu Asn
                   85                  90                  95
   gtt tgg gag cgc ctc gcc gag ggt atg gaa ttt gtt cgc ggc aac ttt      336
   Val Trp Glu Arg Leu Ala Glu Gly Met Glu Phe Val Arg Gly Asn Phe
               100                 105                 110
   gat gat gat gca gct ttc gac aac ctc gct gca aca ctc aag cgc atc      384
   Asp Asp Asp Ala Ala Phe Asp Asn Leu Ala Ala Thr Leu Lys Arg Ile
           115                 120                 125
   gac aaa acc cgc ggc acc gcc ggc aac tgg gct tac tac ctg tcc att      432
   Asp Lys Thr Arg Gly Thr Ala Gly Asn Trp Ala Tyr Tyr Leu Ser Ile
       130                 135                 140
   cca cca gat tcc ttc aca gcg gtc tgc cac cag ctg gag cgt tcc ggc      480
   Pro Pro Asp Ser Phe Thr Ala Val Cys His Gln Leu Glu Arg Ser Gly
   145                 150                 155                 160
   atg gct gaa tcc acc gaa gaa gca tgg cgc cgc gtg atc atc gag aag      528
   Met Ala Glu Ser Thr Glu Glu Ala Trp Arg Arg Val Ile Ile Glu Lys
                   165                 170                 175
   cct ttc ggc cac aac ctc gaa tcc gca cac gag ctc aac cag ctg gtc      576
   Pro Phe Gly His Asn Leu Glu Ser Ala His Glu Leu Asn Gln Leu Val
               180                 185                 190
   aac gca gtc ttc cca gaa tct tct gtg ttc cgc atc gac cac tat ttg      624
   Asn Ala Val Phe Pro Glu Ser Ser Val Phe Arg Ile Asp His Tyr Leu
           195                 200                 205
   ggc aag gaa aca gtt caa aac atc ctg gct ctg cgt ttt gct aac cag      672
   Gly Lys Glu Thr Val Gln Asn Ile Leu Ala Leu Arg Phe Ala Asn Gln
       210                 215                 220
   ctg ttt gag cca ctg tgg aac tcc aac tac gtt gac cac gtc cag atc      720
   Leu Phe Glu Pro Leu Trp Asn Ser Asn Tyr Val Asp His Val Gln Ile
   225                 230                 235                 240
   acc atg gct gaa gat att ggc ttg ggt gga cgt gct ggt tac tac gac      768
   Thr Met Ala Glu Asp Ile Gly Leu Gly Gly Arg Ala Gly Tyr Tyr Asp
                   245                 250                 255
   ggc atc ggc gca gcc cgc gac gtc atc cag aac cac ctg atc cag ctc      816
   Gly Ile Gly Ala Ala Arg Asp Val Ile Gln Asn His Leu Ile Gln Leu
               260                 265                 270
   ttg gct ctg gtt gcc atg gaa gaa cca att tct ttc gtg cca gcg cag      864
   Leu Ala Leu Val Ala Met Glu Glu Pro Ile Ser Phe Val Pro Ala Gln
           275                 280                 285
   ctg cag gca gaa aag atc aag gtg ctc tct gcg aca aag ccg tgc tac      912
   Leu Gln Ala Glu Lys Ile Lys Val Leu Ser Ala Thr Lys Pro Cys Tyr
       290                 295                 300
   cca ttg gat aaa acc tcc gct cgt ggt cag tac gct gcc ggt tgg cag      960
   Pro Leu Asp Lys Thr Ser Ala Arg Gly Gln Tyr Ala Ala Gly Trp Gln
   305                 310                 315                 320
   agc tct gag tta gtc aag gga ctt cgc gaa gaa gat ggc ttc aac cct     1008
   Ser Ser Glu Leu Val Lys Gly Leu Arg Glu Glu Asp Gly Phe Asn Pro
                   325                 330                 335
   gag tcc acc act gag act ttt gcg gct tgt acc tta gag atc acg tct     1056
   Glu Ser Thr Thr Glu Thr Phe Ala Ala Cys Thr Leu Glu Ile Thr Ser
               340                 345                 350
   cgt cgc tgg gct ggt gtg ccg ttc tac ctg cgc acc ggt aag cgt ctt     1104
   Arg Arg Trp Ala Gly Val Pro Phe Tyr Leu Arg Thr Gly Lys Arg Leu
           355                 360                 365
   ggt cgc cgt gtt act gag att gcc gtg gtg ttt aaa gac gca cca cac     1152
   Gly Arg Arg Val Thr Glu Ile Ala Val Val Phe Lys Asp Ala Pro His
       370                 375                 380
   cag cct ttc gac ggc gac atg act gta tcc ctt ggc caa aac gcc atc     1200
   Gln Pro Phe Asp Gly Asp Met Thr Val Ser Leu Gly Gln Asn Ala Ile
   385                 390                 395                 400
   gtg att cgc gtg cag cct gat gaa ggt gtg ctc atc cgc ttc ggt tcc     1248
   Val Ile Arg Val Gln Pro Asp Glu Gly Val Leu Ile Arg Phe Gly Ser
                   405                 410                 415
   aag gtt cca ggt tct gcc atg gaa gtc cgt gac gtc aac atg gac ttc     1296
   Lys Val Pro Gly Ser Ala Met Glu Val Arg Asp Val Asn Met Asp Phe
               420                 425                 430
   tcc tac tca gaa tcc ttc act gaa gaa tca cct gaa gca tac gag cgc     1344
   Ser Tyr Ser Glu Ser Phe Thr Glu Glu Ser Pro Glu Ala Tyr Glu Arg
           435                 440                 445
   ctc att ttg gat gcg ctg tta gat gaa tcc agc ctc ttc cct acc aac     1392
   Leu Ile Leu Asp Ala Leu Leu Asp Glu Ser Ser Leu Phe Pro Thr Asn
       450                 455                 460
   gag gaa gtg gaa ctg agc tgg aag att ctg gat cca att ctt gaa gca     1440
   Glu Glu Val Glu Leu Ser Trp Lys Ile Leu Asp Pro Ile Leu Glu Ala
   465                 470                 475                 480
   tgg gat gcc gat gga gaa cca gag gat tac cca gcg ggt acg tgg ggt     1488
   Trp Asp Ala Asp Gly Glu Pro Glu Asp Tyr Pro Ala Gly Thr Trp Gly
                   485                 490                 495
   cca aag agc gct gat gaa atg ctt tcc cgc aac ggt cac acc tgg cgc     1536
   Pro Lys Ser Ala Asp Glu Met Leu Ser Arg Asn Gly His Thr Trp Arg
               500                 505                 510
   agg cca taa                                                         1545
   Arg Pro
<210> 8
   <211> 514
   <212> PRT
   <213> Corynebacterium glutamicum
<400> 8
   Val Ser Thr Asn Thr Thr Pro Thr Ser Trp Thr Asn Pro Leu Arg Asp
   1               5                   10                  15
   Pro Gln Asp Lys Arg Leu Pro Arg Ile Ala Gly Pro Ser Gly Met Val
               20                  25                  30
   Ile Phe Gly Val Thr Gly Asp Leu Ala Arg Lys Lys Leu Leu Pro Ala
           35                  40                  45
   Ile Tyr Asp Leu Ala Asn Arg Gly Leu Leu Pro Pro Gly Phe Ser Leu
       50                  55                  60
   Val Gly Tyr Gly Arg Arg Glu Trp Ser Lys Glu Asp Phe Glu Lys Tyr
   65                  70                  75                  80
   Val Arg Asp Ala Ala Ser Ala Gly Ala Arg Thr Glu Phe Arg Glu Asn
                   85                  90                  95
   Val Trp Glu Arg Leu Ala Glu Gly Met Glu Phe Val Arg Gly Asn Phe
               100                 105                 110
   Asp Asp Asp Ala Ala Phe Asp Asn Leu Ala Ala Thr Leu Lys Arg Ile
           115                 120                 125
   Asp Lys Thr Arg Gly Thr Ala Gly Asn Trp Ala Tyr Tyr Leu Ser Ile
       130                 135                 140
   Pro Pro Asp Ser Phe Thr Ala Val Cys His Gln Leu Glu Arg Ser Gly
   145                 150                 155                 160
   Met Ala Glu Ser Thr Glu Glu Ala Trp Arg Arg Val Ile Ile Glu Lys
                   165                 170                 175
   Pro Phe Gly His Asn Leu Glu Ser Ala His Glu Leu Asn Gln Leu Val
               180                 185                 190
   Asn Ala Val Phe Pro Glu Ser Ser Val Phe Arg Ile Asp His Tyr Leu
           195                 200                 205
   Gly Lys Glu Thr Val Gln Asn Ile Leu Ala Leu Arg Phe Ala Asn Gln
       210                 215                 220
   Leu Phe Glu Pro Leu Trp Asn Ser Asn Tyr Val Asp His Val Gln Ile
   225 230 235 240
   Thr Met Ala Glu Asp Ile Gly Leu Gly Gly Arg Ala Gly Tyr Tyr Asp
   245 250 255
   Gly Ile Gly Ala Ala Arg Asp Val Ile Gln Asn His Leu Ile Gln Leu
   260 265 270
   Leu Ala Leu Val Ala Met Glu Glu Pro Ile Ser Phe Val Pro Ala Gln
   275 280 285
   Leu Gln Ala Glu Lys Ile Lys Val Leu Ser Ala Thr Lys Pro Cys Tyr
   290 295 300
   Pro Leu Asp Lys Thr Ser Ala Arg Gly Gln Tyr Ala Ala Gly Trp Gln
   305 310 315 320
   Ser Ser Glu Leu Val Lys Gly Leu Arg Glu Glu Asp Gly Phe Asn Pro
   325 330 335
   Glu Ser Thr Thr Glu Thr Phe Ala Ala Cys Thr Leu Glu Ile Thr Ser
   340 345 350
   Arg Arg Trp Ala Gly Val Pro Phe Tyr Leu Arg Thr Gly Lys Arg Leu
   355 360 365
   Gly Arg Arg Val Thr Glu Ile Ala Val Val Phe Lys Asp Ala Pro His
   370 375 380
   Gln Pro Phe Asp Gly Asp Met Thr Val Ser Leu Gly Gln Asn Ala Ile
   385 390 395 400
   Val Ile Arg Val Gln Pro Asp Glu Gly Val Leu Ile Arg Phe Gly Ser
   405 410 415
   Lys Val Pro Gly Ser Ala Met Glu Val Arg Asp Val Asn Met Asp Phe
   420 425 430
   Ser Tyr Ser Glu Ser Phe Thr Glu Glu Ser Pro Glu Ala Tyr Glu Arg
   435 440 445
   Leu Ile Leu Asp Ala Leu Leu Asp Glu Ser Ser Leu Phe Pro Thr Asn
   450 455 460
   Glu Glu Val Glu Leu Ser Trp Lys Ile Leu Asp Pro Ile Leu Glu Ala
   465 470 475 480
   Trp Asp Ala Asp Gly Glu Pro Glu Asp Tyr Pro Ala Gly Thr Trp Gly
   485 490 495
   Pro Lys Ser Ala Asp Glu Met Leu Ser Arg Asn Gly His Thr Trp Arg
   500 505 510
   Arg Pro
<210> 9
   <211> 1545
   <212> DNA
   <213> Corynebacterium glutamicum
<220>
   <221> CDS
   <222> (1)..(1542)
<220>
   <221> mutation
   <222> (22)..(22)
   <223> T -> A Transversion
<220>
   <221> mutation
   <222> (138)..(138)
   <223> C -> T Transition
<220>
   <221> mutation
   <222> (279) .. (279)
   <223> C -> T Transition
<220>
   <221> mutation
   <222> (738)..(738)
   <223> T -> C Transition
<220>
   <221> mutation
   <222> (777)..(777)
   <223> C -> T Transition
<220>
   <221> mutation
   <222> (906)..(906)
   <223> G -> A Transition
<220>
   <221> mutation
   <222> (961)..(961)
   <223> G -> A Transition
<400> 9
   gtg agc aca aac acg acc ccc acc agc tgg aca aac cca ctg cgc gac     48
   Val Ser Thr Asn Thr Thr Pro Thr Ser Trp Thr Asn Pro Leu Arg Asp
   1               5                   10                  15
   ccg cag gat aaa cga ctc ccc cgc atc gct ggc cct tcc ggc atg gtg     96
   Pro Gln Asp Lys Arg Leu Pro Arg Ile Ala Gly Pro Ser Gly Met Val
               20                  25                  30
   atc ttc ggt gtc act ggc gac ttg gct cga aag aag ctg ctt ccc gcc    144
   Ile Phe Gly Val Thr Gly Asp Leu Ala Arg Lys Lys Leu Leu Pro Ala
           35                  40                  45
   att tat gat cta gca aac cgc gga ttg ctg ccc cca gga ttc tcg ttg    192
   Ile Tyr Asp Leu Ala Asn Arg Gly Leu Leu Pro Pro Gly Phe Ser Leu
       50                  55                  60
   gta ggt tac ggc cgc cgc gaa tgg tcc aaa gaa gac ttt gaa aaa tac    240
   Val Gly Tyr Gly Arg Arg Glu Trp Ser Lys Glu Asp Phe Glu Lys Tyr
   65                  70                  75                  80
   gta cgc gat gcc gca agt gct ggt gct cgt acg gaa ttt cgt gaa aat    288
   Val Arg Asp Ala Ala Ser Ala Gly Ala Arg Thr Glu Phe Arg Glu Asn
                   85                  90                  95
   gtt tgg gag cgc ctc gcc gag ggt atg gaa ttt gtt cgc ggc aac ttt    336
   Val Trp Glu Arg Leu Ala Glu Gly Met Glu Phe Val Arg Gly Asn Phe
               100                 105                 110
   gat gat gat gca gct ttc gac aac ctc gct gca aca ctc aag cgc atc    384
   Asp Asp Asp Ala Ala Phe Asp Asn Leu Ala Ala Thr Leu Lys Arg Ile
           115                 120                 125
   gac aaa acc cgc ggc acc gcc ggc aac tgg gct tac tac ctg tcc att    432
   Asp Lys Thr Arg Gly Thr Ala Gly Asn Trp Ala Tyr Tyr Leu Ser Ile
       130                 135                 140
   cca cca gat tcc ttc aca gcg gtc tgc cac cag ctg gag cgt tcc ggc    480
   Pro Pro Asp Ser Phe Thr Ala Val Cys His Gln Leu Glu Arg Ser Gly
   145                 150                 155                 160
   atg gct gaa tcc acc gaa gaa gca tgg cgc cgc gtg atc atc gag aag    528
   Met Ala Glu Ser Thr Glu Glu Ala Trp Arg Arg Val Ile Ile Glu Lys
                   165                 170                 175
   cct ttc ggc cac aac ctc gaa tcc gca cac gag ctc aac cag ctg gtc    576
   Pro Phe Gly His Asn Leu Glu Ser Ala His Glu Leu Asn Gln Leu Val
               180                 185                 190
   aac gca gtc ttc cca gaa tct tct gtg ttc cgc atc gac cac tat ttg    624
   Asn Ala Val Phe Pro Glu Ser Ser Val Phe Arg Ile Asp His Tyr Leu
           195                 200                 205
   ggc aag gaa aca gtt caa aac atc ctg gct ctg cgt ttt gct aac cag    672
   Gly Lys Glu Thr Val Gln Asn Ile Leu Ala Leu Arg Phe Ala Asn Gln
       210                 215                 220
   ctg ttt gag cca ctg tgg aac tcc aac tac gtt gac cac gtc cag atc    720
   Leu Phe Glu Pro Leu Trp Asn Ser Asn Tyr Val Asp His Val Gln Ile
   225                 230                 235                 240
   acc atg gct gaa gat atc ggc ttg ggt gga cgt gct ggt tac tac gac     768
   Thr Met Ala Glu Asp Ile Gly Leu Gly Gly Arg Ala Gly Tyr Tyr Asp
                   245                 250                 255
   ggc atc ggt gca gcc cgc gac gtc atc cag aac cac ctg atc cag ctc     816
   Gly Ile Gly Ala Ala Arg Asp Val Ile Gln Asn His Leu Ile Gln Leu
               260                 265                 270
   ttg gct ctg gtt gcc atg gaa gaa cca att tct ttc gtg cca gcg cag     864
   Leu Ala Leu Val Ala Met Glu Glu Pro Ile Ser Phe Val Pro Ala Gln
           275                 280                 285
   ctg cag gca gaa aag atc aag gtg ctc tct gcg aca aag cca tgc tac     912
   Leu Gln Ala Glu Lys Ile Lys Val Leu Ser Ala Thr Lys Pro Cys Tyr
       290                 295                 300
   cca ttg gat aaa acc tcc gct cgt ggt cag tac gct gcc ggt tgg cag     960
   Pro Leu Asp Lys Thr Ser Ala Arg Gly Gln Tyr Ala Ala Gly Trp Gln
   305                 310                 315                 320
   agc tct gag tta gtc aag gga ctt cgc gaa gaa gat ggc ttc aac cct    1008
   Ser Ser Glu Leu Val Lys Gly Leu Arg Glu Glu Asp Gly Phe Asn Pro
                   325                 330                 335
   gag tcc acc act gag act ttt gcg gct tgt acc tta gag atc acg tct    1056
   Glu Ser Thr Thr Glu Thr Phe Ala Ala Cys Thr Leu Glu Ile Thr Ser
               340                 345                 350
   cgt cgc tgg gct ggt gtg ccg ttc tac ctg cgc acc ggt aag cgt ctt    1104
   Arg Arg Trp Ala Gly Val Pro Phe Tyr Leu Arg Thr Gly Lys Arg Leu
           355                 360                 365
   ggt cgc cgt gtt act gag att gcc gtg gtg ttt aaa gac gca cca cac    1152
   Gly Arg Arg Val Thr Glu Ile Ala Val Val Phe Lys Asp Ala Pro His
       370                 375                 380
   cag cct ttc gac ggc gac atg act gta tcc ctt ggc caa aac gcc atc    1200
   Gln Pro Phe Asp Gly Asp Met Thr Val Ser Leu Gly Gln Asn Ala Ile
   385                 390                 395                 400
   gtg att cgc gtg cag cct gat gaa ggt gtg ctc atc cgc ttc ggt tcc    1248
   Val Ile Arg Val Gln Pro Asp Glu Gly Val Leu Ile Arg Phe Gly Ser
                   405                 410                 415
   aag gtt cca ggt tct gcc atg gaa gtc cgt gac gtc aac atg gac ttc    1296
   Lys Val Pro Gly Ser Ala Met Glu Val Arg Asp Val Asn Met Asp Phe
               420                 425                 430
   tcc tac tca gaa tcc ttc act gaa gaa tca cct gaa gca tac gag cgc    1344
   Ser Tyr Ser Glu Ser Phe Thr Glu Glu Ser Pro Glu Ala Tyr Glu Arg
           435                 440                 445
   ctc att ttg gat gcg ctg tta gat gaa tcc agc ctc ttc cct acc aac    1392
   Leu Ile Leu Asp Ala Leu Leu Asp Glu Ser Ser Leu Phe Pro Thr Asn
       450                 455                 460
   gag gaa gtg gaa ctg agc tgg aag att ctg gat cca att ctt gaa gca    1440
   Glu Glu Val Glu Leu Ser Trp Lys Ile Leu Asp Pro Ile Leu Glu Ala
   465                 470                 475                 480
   tgg gat gcc gat gga gaa cca gag gat tac cca gcg ggt acg tgg ggt      1488
   Trp Asp Ala Asp Gly Glu Pro Glu Asp Tyr Pro Ala Gly Thr Trp Gly
                   485                 490                 495
   cca aag agc gct gat gaa atg ctt tcc cgc aac ggt cac acc tgg cgc      1536
   Pro Lys Ser Ala Asp Glu Met Leu Ser Arg Asn Gly His Thr Trp Arg
               500                 505                 510
   agg cca taa                                                          1545
   Arg Pro
<210> 10
   <211> 514
   <212> PRT
   <213> Corynebacterium glutamicum
<400> 10
   Val Ser Thr Asn Thr Thr Pro Thr Ser Trp Thr Asn Pro Leu Arg Asp
   1               5                   10                  15
   Pro Gln Asp Lys Arg Leu Pro Arg Ile Ala Gly Pro Ser Gly Met Val
               20                  25                  30
   Ile Phe Gly Val Thr Gly Asp Leu Ala Arg Lys Lys Leu Leu Pro Ala
           35                  40                  45
   Ile Tyr Asp Leu Ala Asn Arg Gly Leu Leu Pro Pro Gly Phe Ser Leu
       50                  55                  60
   Val Gly Tyr Gly Arg Arg Glu Trp Ser Lys Glu Asp Phe Glu Lys Tyr
   65                  70                  75                  80
   Val Arg Asp Ala Ala Ser Ala Gly Ala Arg Thr Glu Phe Arg Glu Asn
                   85                  90                  95
   Val Trp Glu Arg Leu Ala Glu Gly Met Glu Phe Val Arg Gly Asn Phe
               100                 105                 110
   Asp Asp Asp Ala Ala Phe Asp Asn Leu Ala Ala Thr Leu Lys Arg Ile
           115                 120                 125
   Asp Lys Thr Arg Gly Thr Ala Gly Asn Trp Ala Tyr Tyr Leu Ser Ile
       130                 135                 140
   Pro Pro Asp Ser Phe Thr Ala Val Cys His Gln Leu Glu Arg Ser Gly
   145                 150                 155                 160
   Met Ala Glu Ser Thr Glu Glu Ala Trp Arg Arg Val Ile Ile Glu Lys
                   165                 170                 175
   Pro Phe Gly His Asn Leu Glu Ser Ala His Glu Leu Asn Gln Leu Val
               180                 185                 190
   Asn Ala Val Phe Pro Glu Ser Ser Val Phe Arg Ile Asp His Tyr Leu
           195                 200                 205
   Gly Lys Glu Thr Val Gln Asn Ile Leu Ala Leu Arg Phe Ala Asn Gln
       210                 215                 220
   Leu Phe Glu Pro Leu Trp Asn Ser Asn Tyr Val Asp His Val Gln Ile
   225                 230                 235                 240
   Thr Met Ala Glu Asp Ile Gly Leu Gly Gly Arg Ala Gly Tyr Tyr Asp
                   245                 250                 255
   Gly Ile Gly Ala Ala Arg Asp Val Ile Gln Asn His Leu Ile Gln Leu
               260                 265                 270
   Leu Ala Leu Val Ala Met Glu Glu Pro Ile Ser Phe Val Pro Ala Gln
           275                 280                 285
   Leu Gln Ala Glu Lys Ile Lys Val Leu Ser Ala Thr Lys Pro Cys Tyr
       290                 295                 300
   Pro Leu Asp Lys Thr Ser Ala Arg Gly Gln Tyr Ala Ala Gly Trp Gln
   305                 310                 315                 320
   Ser Ser Glu Leu Val Lys Gly Leu Arg Glu Glu Asp Gly Phe Asn Pro
                   325                 330                 335
   Glu Ser Thr Thr Glu Thr Phe Ala Ala Cys Thr Leu Glu Ile Thr Ser
               340                 345                 350
   Arg Arg Trp Ala Gly Val Pro Phe Tyr Leu Arg Thr Gly Lys Arg Leu
           355                 360                 365
   Gly Arg Arg Val Thr Glu Ile Ala Val Val Phe Lys Asp Ala Pro His
       370                 375                 380
   Gln Pro Phe Asp Gly Asp Met Thr Val Ser Leu Gly Gln Asn Ala Ile
   385                 390                 395                 400
   Val Ile Arg Val Gln Pro Asp Glu Gly Val Leu Ile Arg Phe Gly Ser
                   405                 410                 415
   Lys Val Pro Gly Ser Ala Met Glu Val Arg Asp Val Asn Met Asp Phe
   420 425 430
   Ser Tyr Ser Glu Ser Phe Thr Glu Glu Ser Pro Glu Ala Tyr Glu Arg
   435 440 445
   Leu Ile Leu Asp Ala Leu Leu Asp Glu Ser Ser Leu Phe Pro Thr Asn
   450 455 460
   Glu Glu Val Glu Leu Ser Trp Lys Ile Leu Asp Pro Ile Leu Glu Ala
   465 470 475 480
   Trp Asp Ala Asp Gly Glu Pro Glu Asp Tyr Pro Ala Gly Thr Trp Gly
   485 490 495
   Pro Lys Ser Ala Asp Glu Met Leu Ser Arg Asn Gly His Thr Trp Arg
   500 505 510
   Arg Pro
<210> 11
   <211> 2100
   <212> DNA
   <213> Corynebacterium glutamicum
<220>
   <221> mutation
   <222> (208)..(208)
   <223> A -> G Transition
<220>
   <221> mutation
   <222> (235)..(235)
   <223> G -> A Transition
<220>
   <221> mutation
   <222> (245) .. (245)
   <223> T -> C Transition
<220>
   <221> mutation
   <222> (257)..(257)
   <223> A -> G Transition
<220>
   <221> mutation
   <222> (299)..(299)
   <223> A -> G Transition
<220>
   <221> CDS
   <222> (308) .. (1849)
<220>
   <221> mutation
   <222> (329)..(329)
   <223> T -> A Transversion
<220>
   <221> mutation
   <222> (445) .. (445)
   <223> C -> T Transition
<220>
   <221> mutation
   <222> (586) .. (586)
   <223> C -> T Transition
<220>
   <221> mutation
   <222> (1045)..(1045)
   <223> T -> C Transition
<220>
   <221> mutation
   <222> (1084)..(1084)
   <223> C -> T Transition
<220>
   <221> mutation
   <222> (1213) .. (1213)
   <223> G -> A Transition
<220>
   <221> mutation
   <222> (1268)..(1268)
   <223> G -> A Transition
<400> 11
   tcgacgcggt tctggagcag ggcaacctgc acggtgacac cctgtccaac tccgcggcag      60
   aagctgacgc tgtgttctcc cagcttgagg ctctgggcgt tgacttggca gatgtcttcc     120
   aggtcctgga gaccgagggt gtggacaagt tcgttgcttc ttggagcgaa ctgcttgagt     180
   ccatggaagc tcgcctgaag tagaatcggc acgctgcatc agtaacggcg acataaaatc     240
   gaatcagttc gatcttgtgt ggccgttaca catctttcat taaagaaagg atcgtgacgc     300
   taccatc gtg agc aca aac acg acc ccc acc agc tgg aca aac cca ctg       349
           Val Ser Thr Asn Thr Thr Pro Thr Ser Trp Thr Asn Pro Leu
           1               5                   10
   cgc gac ccg cag gat aaa cga ctc ccc cgc atc gct ggc cct tcc ggc       397
   Arg Asp Pro Gln Asp Lys Arg Leu Pro Arg Ile Ala Gly Pro Ser Gly
   15                  20                  25                  30
   atg gtg atc ttc ggt gtc act ggc gac ttg gct cga aag aag ctg ctt       445
   Met Val Ile Phe Gly Val Thr Gly Asp Leu Ala Arg Lys Lys Leu Leu
                   35                  40                  45
   ccc gcc att tat gat cta gca aac cgc gga ttg ctg ccc cca gga ttc       493
   Pro Ala Ile Tyr Asp Leu Ala Asn Arg Gly Leu Leu Pro Pro Gly Phe
               50                  55                  60
   tcg ttg gta ggt tac ggc cgc cgc gaa tgg tcc aaa gaa gac ttt gaa      541
   Ser Leu Val Gly Tyr Gly Arg Arg Glu Trp Ser Lys Glu Asp Phe Glu
           65                  70                  75
   aaa tac gta cgc gat gcc gca agt gct ggt gct cgt acg gaa ttt cgt      589
   Lys Tyr Val Arg Asp Ala Ala Ser Ala Gly Ala Arg Thr Glu Phe Arg
       80                  85                  90
   gaa aat gtt tgg gag cgc ctc gcc gag ggt atg gaa ttt gtt cgc ggc      637
   Glu Asn Val Trp Glu Arg Leu Ala Glu Gly Met Glu Phe Val Arg Gly
   95                  100                 105                 110
   aac ttt gat gat gat gca gct ttc gac aac ctc gct gca aca ctc aag      685
   Asn Phe Asp Asp Asp Ala Ala Phe Asp Asn Leu Ala Ala Thr Leu Lys
                   115                 120                 125
   cgc atc gac aaa acc cgc ggc acc gcc ggc aac tgg gct tac tac ctg      733
   Arg Ile Asp Lys Thr Arg Gly Thr Ala Gly Asn Trp Ala Tyr Tyr Leu
               130                 135                 140
   tcc att cca cca gat tcc ttc aca gcg gtc tgc cac cag ctg gag cgt      781
   Ser Ile Pro Pro Asp Ser Phe Thr Ala Val Cys His Gln Leu Glu Arg
           145                 150                 155
   tcc ggc atg gct gaa tcc acc gaa gaa gca tgg cgc cgc gtg atc atc      829
   Ser Gly Met Ala Glu Ser Thr Glu Glu Ala Trp Arg Arg Val Ile Ile
       160                 165                 170
   gag aag cct ttc ggc cac aac ctc gaa tcc gca cac gag ctc aac cag      877
   Glu Lys Pro Phe Gly His Asn Leu Glu Ser Ala His Glu Leu Asn Gln
   175                 180                 185                 190
   ctg gtc aac gca gtc ttc cca gaa tct tct gtg ttc cgc atc gac cac      925
   Leu Val Asn Ala Val Phe Pro Glu Ser Ser Val Phe Arg Ile Asp His
                   195                 200                 205
   tat ttg ggc aag gaa aca gtt caa aac atc ctg gct ctg cgt ttt gct      973
   Tyr Leu Gly Lys Glu Thr Val Gln Asn Ile Leu Ala Leu Arg Phe Ala
               210                 215                 220
   aac cag ctg ttt gag cca ctg tgg aac tcc aac tac gtt gac cac gtc     1021
   Asn Gln Leu Phe Glu Pro Leu Trp Asn Ser Asn Tyr Val Asp His Val
           225                 230                 235
   cag atc acc atg gct gaa gat atc ggc ttg ggt gga cgt gct ggt tac     1069
   Gln Ile Thr Met Ala Glu Asp Ile Gly Leu Gly Gly Arg Ala Gly Tyr
       240                 245                 250
   tac gac ggc atc ggt gca gcc cgc gac gtc atc cag aac cac ctg atc     1117
   Tyr Asp Gly Ile Gly Ala Ala Arg Asp Val Ile Gln Asn His Leu Ile
   255                 260                 265                 270
   cag ctc ttg gct ctg gtt gcc atg gaa gaa cca att tct ttc gtg cca     1165
   Gln Leu Leu Ala Leu Val Ala Met Glu Glu Pro Ile Ser Phe Val Pro
                   275                 280                 285
   gcg cag ctg cag gca gaa aag atc aag gtg ctc tct gcg aca aag cca     1213
   Ala Gln Leu Gln Ala Glu Lys Ile Lys Val Leu Ser Ala Thr Lys Pro
               290                 295                 300
   tgc tac cca ttg gat aaa acc tcc gct cgt ggt cag tac gct gcc ggt      1261
   Cys Tyr Pro Leu Asp Lys Thr Ser Ala Arg Gly Gln Tyr Ala Ala Gly
           305                 310                 315
   tgg cag agc tct gag tta gtc aag gga ctt cgc gaa gaa gat ggc ttc      1309
   Trp Gln Ser Ser Glu Leu Val Lys Gly Leu Arg Glu Glu Asp Gly Phe
       320                 325                 330
   aac cct gag tcc acc act gag act ttt gcg gct tgt acc tta gag atc      1357
   Asn Pro Glu Ser Thr Thr Glu Thr Phe Ala Ala Cys Thr Leu Glu Ile
   335                 340                 345                 350
   acg tct cgt cgc tgg gct ggt gtg ccg ttc tac ctg cgc acc ggt aag      1405
   Thr Ser Arg Arg Trp Ala Gly Val Pro Phe Tyr Leu Arg Thr Gly Lys
                   355                 360                 365
   cgt ctt ggt cgc cgt gtt act gag att gcc gtg gtg ttt aaa gac gca      1453
   Arg Leu Gly Arg Arg Val Thr Glu Ile Ala Val Val Phe Lys Asp Ala
               370                 375                 380
   cca cac cag cct ttc gac ggc gac atg act gta tcc ctt ggc caa aac      1501
   Pro His Gln Pro Phe Asp Gly Asp Met Thr Val Ser Leu Gly Gln Asn
           385                 390                 395
   gcc atc gtg att cgc gtg cag cct gat gaa ggt gtg ctc atc cgc ttc      1549
   Ala Ile Val Ile Arg Val Gln Pro Asp Glu Gly Val Leu Ile Arg Phe
       400                 405                 410
   ggt tcc aag gtt cca ggt tct gcc atg gaa gtc cgt gac gtc aac atg      1597
   Gly Ser Lys Val Pro Gly Ser Ala Met Glu Val Arg Asp Val Asn Met
   415                 420                 425                 430
   gac ttc tcc tac tca gaa tcc ttc act gaa gaa tca cct gaa gca tac      1645
   Asp Phe Ser Tyr Ser Glu Ser Phe Thr Glu Glu Ser Pro Glu Ala Tyr
                   435                 440                 445
   gag cgc ctc att ttg gat gcg ctg tta gat gaa tcc agc ctc ttc cct      1693
   Glu Arg Leu Ile Leu Asp Ala Leu Leu Asp Glu Ser Ser Leu Phe Pro
               450                 455                 460
   acc aac gag gaa gtg gaa ctg agc tgg aag att ctg gat cca att ctt      1741
   Thr Asn Glu Glu Val Glu Leu Ser Trp Lys Ile Leu Asp Pro Ile Leu
           465                 470                 475
   gaa gca tgg gat gcc gat gga gaa cca gag gat tac cca gcg ggt acg      1789
   Glu Ala Trp Asp Ala Asp Gly Glu Pro Glu Asp Tyr Pro Ala Gly Thr
       480                 485                 490
   tgg ggt cca aag agc gct gat gaa atg ctt tcc cgc aac ggt cac acc      1837
   Trp Gly Pro Lys Ser Ala Asp Glu Met Leu Ser Arg Asn Gly His Thr
   495                 500                 505                 510
   tgg cgc agg cca taatttaggg gcaaaaaatg atctttgaac ttccggatac          1889
   Trp Arg Arg Pro
   caccacccag caaatttcca agaccctaac tcgactgcgt gaatcgggca cccaggtcac    1949
   caccggccga gtgctcaccc tcatcgtggt cactgactcc gaaagcgatg tcgctgcagt    2009
   taccgagtcc accaatgaag cctcgcgcga gcacccatct cgcgtgatca ttttggtggt    2069
   tggcgataaa actgcagaaa acaaagttga c                                   2100
<210> 12
   <211> 514
   <212> PRT
   <213> Corynebacterium glutamicum
<400> 12
   Val Ser Thr Asn Thr Thr Pro Thr Ser Trp Thr Asn Pro Leu Arg Asp
   1               5                   10                  15
   Pro Gln Asp Lys Arg Leu Pro Arg Ile Ala Gly Pro Ser Gly Met Val
               20                  25                  30
   Ile Phe Gly Val Thr Gly Asp Leu Ala Arg Lys Lys Leu Leu Pro Ala
           35                  40                  45
   Ile Tyr Asp Leu Ala Asn Arg Gly Leu Leu Pro Pro Gly Phe Ser Leu
       50                  55                  60
   Val Gly Tyr Gly Arg Arg Glu Trp Ser Lys Glu Asp Phe Glu Lys Tyr
   65                  70                  75                  80
   Val Arg Asp Ala Ala Ser Ala Gly Ala Arg Thr Glu Phe Arg Glu Asn
                   85                  90                  95
   Val Trp Glu Arg Leu Ala Glu Gly Met Glu Phe Val Arg Gly Asn Phe
               100                 105                 110
   Asp Asp Asp Ala Ala Phe Asp Asn Leu Ala Ala Thr Leu Lys Arg Ile
           115                 120                 125
   Asp Lys Thr Arg Gly Thr Ala Gly Asn Trp Ala Tyr Tyr Leu Ser Ile
       130                 135                 140
   Pro Pro Asp Ser Phe Thr Ala Val Cys His Gln Leu Glu Arg Ser Gly
   145                 150                 155                 160
   Met Ala Glu Ser Thr Glu Glu Ala Trp Arg Arg Val Ile Ile Glu Lys
                   165                 170                 175
   Pro Phe Gly His Asn Leu Glu Ser Ala His Glu Leu Asn Gln Leu Val
               180                 185                 190
   Asn Ala Val Phe Pro Glu Ser Ser Val Phe Arg Ile Asp His Tyr Leu
           195                 200                 205
   Gly Lys Glu Thr Val Gln Asn Ile Leu Ala Leu Arg Phe Ala Asn Gln
   210 215 220
   Leu Phe Glu Pro Leu Trp Asn Ser Asn Tyr Val Asp His Val Gln Ile
   225 230 235 240
   Thr Met Ala Glu Asp Ile Gly Leu Gly Gly Arg Ala Gly Tyr Tyr Asp
   245 250 255
   Gly Ile Gly Ala Ala Arg Asp Val Ile Gln Asn His Leu Ile Gln Leu
   260 265 270
   Leu Ala Leu Val Ala Met Glu Glu Pro Ile Ser Phe Val Pro Ala Gln
   275 280 285
   Leu Gln Ala Glu Lys Ile Lys Val Leu Ser Ala Thr Lys Pro Cys Tyr
   290 295 300
   Pro Leu Asp Lys Thr Ser Ala Arg Gly Gln Tyr Ala Ala Gly Trp Gln
   305 310 315 320
   Ser Ser Glu Leu Val Lys Gly Leu Arg Glu Glu Asp Gly Phe Asn Pro
   325 330 335
   Glu Ser Thr Thr Glu Thr Phe Ala Ala Cys Thr Leu Glu Ile Thr Ser
   340 345 350
   Arg Arg Trp Ala Gly Val Pro Phe Tyr Leu Arg Thr Gly Lys Arg Leu
   355 360 365
   Gly Arg Arg Val Thr Glu Ile Ala Val Val Phe Lys Asp Ala Pro His
   370 375 380
   Gln Pro Phe Asp Gly Asp Met Thr Val Ser Leu Gly Gln Asn Ala Ile
   385 390 395 400
   Val Ile Arg Val Gln Pro Asp Glu Gly Val Leu Ile Arg Phe Gly Ser
   405 410 415
   Lys Val Pro Gly Ser Ala Met Glu Val Arg Asp Val Asn Met Asp Phe
   420 425 430
   Ser Tyr Ser Glu Ser Phe Thr Glu Glu Ser Pro Glu Ala Tyr Glu Arg
   435 440 445
   Leu Ile Leu Asp Ala Leu Leu Asp Glu Ser Ser Leu Phe Pro Thr Asn
   450 455 460
   Glu Glu Val Glu Leu Ser Trp Lys Ile Leu Asp Pro Ile Leu Glu Ala
   465                 470                 475                 480
   Trp Asp Ala Asp Gly Glu Pro Glu Asp Tyr Pro Ala Gly Thr Trp Gly
                   485                 490                 495
   Pro Lys Ser Ala Asp Glu Met Leu Ser Arg Asn Gly His Thr Trp Arg
               500                 505                 510
   Arg Pro
<210> 13
   <211> 87
   <212> DNA
   <213> Corynebacterium glutamicum
<220>
   <221> CDS
   <222> (1) .. (87)
<220>
   <221> misc_feature
   <222> (43) .. (45)
   <223> n is a, c, g, or t
<400> 13
   gat aaa acc tcc gct cgt ggt cag tac gct gcc ggt tgg cag nnn tct 48
   Asp Lys Thr Ser Ala Arg Gly Gln Tyr Ala Ala Gly Trp Gln Xaa Ser
   1               5                   10                  15
   gag tta gtc aag gga ctt cgc gaa gaa gat ggc ttc aac 87
   Glu Leu Val Lys Gly Leu Arg Glu Glu Asp Gly Phe Asn
               20                  25
<210> 14
   <211> 29
   <212> PRT
   <213> Corynebacterium glutamicum
<220>
   <221> misc_feature
   <222> (15)..(15)
   <223> The 'Xaa' at location 15 stands for Lys, Asn, Arg, Ser, Thr, Ile, Met, Glu, Asp, Gly, Ala, Val, Gln, His, Pro, Leu, Tyr, Trp, Cys, or Phe.
<400> 14
   Asp Lys Thr Ser Ala Arg Gly Gln Tyr Ala Ala Gly Trp Gln Xaa Ser
   1               5                   10                  15
   Glu Leu Val Lys Gly Leu Arg Glu Glu Asp Gly Phe Asn
               20                  25
<210> 15
   <211> 87
   <212> DNA
   <213> Corynebacterium glutamicum
<220>
   <221> CDS
   <222> (1) .. (87)
<400> 15
   gat aaa acc tcc gct cgt ggt cag tac gct gcc ggt tgg cag agc tct        48
   Asp Lys Thr Ser Ala Arg Gly Gln Tyr Ala Ala Gly Trp Gln Ser Ser
   1               5                   10                  15
   gag tta gtc aag gga ctt cgc gaa gaa gat ggc ttc aac                    87
   Glu Leu Val Lys Gly Leu Arg Glu Glu Asp Gly Phe Asn
               20                  25
<210> 16
   <211> 29
   <212> PRT
   <213> Corynebacterium glutamicum
<400> 16
   Asp Lys Thr Ser Ala Arg Gly Gln Tyr Ala Ala Gly Trp Gln Ser Ser
   1               5                   10                  15
   Glu Leu Val Lys Gly Leu Arg Glu Glu Asp Gly Phe Asn
               20                  25
<210> 17
   <211> 20
   <212> DNA
   <213> Corynebacterium glutamicum
<220>
   <221> misc_feature
   <222> (1)..(20)
   <223> Primer zwf-K1
<400> 17
   aaggatcgtg acactaccat 20
<210> 18
   <211> 20
   <212> DNA
   <213> Corynebacterium glutamicum
<220>
   <221> misc_feature
   <222> (1)..(20)
   <223> Primer zwf-K2
<400> 18
   ggtggtatcc ggaagttcaa 20
<210> 19
   <211> 20
   <212> DNA
   <213> Corynebacterium glutamicum
<220>
   <221> misc_feature
   <222> (1) .. (20)
   <223> Primer zwf-L1
<400> 19
   agaagctgac gctgtgttct 20
<210> 20
   <211> 20
   <212> DNA
   <213> Corynebacterium glutamicum
<220>
   <221> misc_feature
   <222> (1) .. (20)
   <223> Primer zwf-L2
<400> 20
   cattggtgga ctcggtaact 20
<210> 21
   <211> 1263
   <212> DNA
   <213> Corynebacterium glutamicum
<220>
   <221> CDS
   <222> (1) .. (1263)
   <223> lysC-Wildtypgen
<400> 21
   gtg gcc ctg gtc gta cag aaa tat ggc ggt tcc tcg ctt gag agt gcg      48
   Val Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct      96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat     144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt      192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc      240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gcc caa tct ttc acg      288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc      336
   Gly Ser Gln Ala Gly Val Leu Thr Thr Glu Arg His Gly Asn Ala Arg
               100                 105                 110
   att gtt gat gtc act cca ggt cgt gtg cgt gaa gca ctc gat gag ggc      384
   Ile Val Asp Val Thr Pro Gly Arg Val Arg Glu Ala Leu Asp Glu Gly
           115                 120                 125
   aag atc tgc att gtt gct ggt ttc cag ggt gtt aat aaa gaa acc cgc      432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg      480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt      528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag      576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc      624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat      672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg      720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc      768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att      816
   Gly Val Ala Thr Asp Lys Ser Glu Ala Lys Val Thr Val Leu Gly Ile
               260                 265                 270
   tcc gat aag cca ggc gag gct gcg aag gtt ttc cgt gcg ttg gct gat      864
   Ser Asp Lys Pro Gly Glu Ala Ala Lys Val Phe Arg Ala Leu Ala Asp
           275                 280                 285
   gca gaa atc aac att gac atg gtt ctg cag aac gtc tct tct gta gaa      912
   Ala Glu Ile Asn Ile Asp Met Val Leu Gln Asn Val Ser Ser Val Glu
       290                 295                 300
   gac ggc acc acc gac atc acc ttc acc tgc cct cgt tcc gac ggc cgc      960
   Asp Gly Thr Thr Asp Ile Thr Phe Thr Cys Pro Arg Ser Asp Gly Arg
   305                 310                 315                 320
   cgc gcg atg gag atc ttg aag aag ctt cag gtt cag ggc aac tgg acc     1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct     1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg     1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt     1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca     1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat     1248
   Leu His Glu Gln Phe Gln Leu Gly Gly Glu Asp Glu Ala Val Val Tyr
                   405                 410                 415
   gca ggc acc gga cgc                                                 1263
   Ala Gly Thr Gly Arg
               420
<210> 22
   <211> 421
   <212> PRT
   <213> Corynebacterium glutamicum
<400> 22
   Val Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
   85 90 95
   Gly Ser Gln Ala Gly Val Leu Thr Thr Glu Arg His Gly Asn Ala Arg
   100 105 110
   Ile Val Asp Val Thr Pro Gly Arg Val Arg Glu Ala Leu Asp Glu Gly
   115 120 125
   Lys Ile Cys Ile Val Ala Gly Phe Gln Gly Val Asn Lys Glu Thr Arg
   130 135 140
   Asp Val Thr Thr Leu Gly Arg Gly Gly Ser Asp Thr Thr Ala Val Ala
   145 150 155 160
   Leu Ala Ala Ala Leu Asn Ala Asp Val Cys Glu Ile Tyr Ser Asp Val
   165 170 175
   Asp Gly Val Tyr Thr Ala Asp Pro Arg Ile Val Pro Asn Ala Gln Lys
   180 185 190
   Leu Glu Lys Leu Ser Phe Glu Glu Met Leu Glu Leu Ala Ala Val Gly
   195 200 205
   Ser Lys Ile Leu Val Leu Arg Ser Val Glu Tyr Ala Arg Ala Phe Asn
   210 215 220
   Val Pro Leu Arg Val Arg Ser Ser Tyr Ser Asn Asp Pro Gly Thr Leu
   225 230 235 240
   Ile Ala Gly Ser Met Glu Asp Ile Pro Val Glu Glu Ala Val Leu Thr
   245 250 255
   Gly Val Ala Thr Asp Lys Ser Glu Ala Lys Val Thr Val Leu Gly Ile
   260 265 270
   Ser Asp Lys Pro Gly Glu Ala Ala Lys Val Phe Arg Ala Leu Ala Asp
   275 280 285
   Ala Glu Ile Asn Ile Asp Met Val Leu Gln Asn Val Ser Ser Val Glu
   290 295 300
   Asp Gly Thr Thr Asp Ile Thr Phe Thr Cys Pro Arg Ser Asp Gly Arg
   305 310 315 320
   Arg Ala Met Glu Ile Leu Lys Lys Leu Gln Val Gln Gly Asn Trp Thr
                   325                 330                 335
   Asn Val Leu Tyr Asp Asp Gln Val Gly Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Thr Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg
               420
<210> 23
   <211> 30
   <212> DNA
   <213> Artificial Sequence
<220>
   <223> Primer zwf-int1-bam
<400> 23
   ctagggatcc acgtacgcga tgccgcaagt 30
<210> 24
   <211> 30
   <212> DNA
   <213> Artificial Sequence
<220>
   <223> Primer zwf-int2-bam
<400> 24
   ctagggatcc tcaggctgca cgcgaatcac 30